# EUROPEAN PATENT SPECIFICATION

(11) **EP 1 947 199 B1**
(45) Date of publication and mention of the grant of the patent: **20.06.2012**
(21) Application number: 08005525.4
(22) Date of filing: 26.03.2004
(51) Int. Cl.: C12Q 1/68

(54) **Method for identifying nucleic acid molecules associated with angiogenesis**
Verfahren zur Identifizierung von Nukleinsäuremolekülen, die mit der Angiogenese assoziiert sind
Procédé d'identification de molécules d'acide nucléique associées à l'angionèse

(30) Priority: 28.03.2003 AU 2003901511
(43) Date of publication of application: 23.07.2008
(62) Divisional of application: 04723453.9
(73) Proprietor: Bionomics Limited, Thebarton, S.A. 5031 (AU)
(72) Inventor: Gonda, Thomas John, Chapel Hill Queensland 4069 (AU); Kremmidiotis, Gabriel, Flagstaff Hill South Australia 5159 (AU)
(74) Representative: Chapman, Paul Gilmour

(56) References cited:
- WO-A-00/58495
- WO-A-01/53312
- WO-A-02/086443
- GLIENKE JENS ET AL: "Differential gene expression by endothelial cells in distinct angiogenic states" EUROPEAN JOURNAL OF BIOCHEMISTRY, BERLIN, DE, vol. 267, no. 9, May 2000 (2000-05), pages 2820-2830, XP002213005 ISSN: 0014-2956
- KAHN JEANNE ET AL: "Gene expression profiling in an in vitro model of angiogenesis" AMERICAN JOURNAL OF PATHOLOGY, PHILADELPHIA, PA, US, vol. 156, no. 6, June 2000 (2000-06), pages 1887-1900, XP002178225 ISSN: 0002-9440

### SEQUENCE LISTING

<110> Bionomics Limited
<120> Method of identifying nucleic acid molecules associated with angiogenesis
<130> PM318469 EPA, Genbank reference AB 020684.1
<160> 1
<170> PatentIn version 3.3
<210> 59
   <211> 4436
   <212> DNA
   <213> Homo sapiens
<400> 59
   cccccttgtc attaatacat taaaaagatt caatctttac cctgaggtaa ttttggccag      60
   ttggtaccgg atttatacca aaataatgga cttgattggt attcaaacca agatatgttg     120
   gacggttacc agaggagaag gactcagtcc tattgaaagc tgtgaaggat tgggagatcc     180
   tgcttgcttt tatgttgctg taatttttat tttaaatgga ctaatgatgg cattattctt     240
   catatatggc acatatttaa gtggcagccg attaggaggc ctggttacag tgttgtgctt     300
   ctttttcaat catggagagt gtacccgtgt aatgtggaca ccacctctcc gtgaaagctt     360
   ctcatatcca tttcttgttc ttcagatgtt gctagtgact catattctca gggctacaaa     420
   actttataga ggaagcttga ttgcactctg catttccaat gtatttttca tgcttccttg     480
   gcagtttgct cagtttgtac ttcttactca gattgcatca ttatttgcag tatatgttgt     540
   cgggtacatt gatatatgta aattacggaa gatcatttat atacacatga tttctcttgc     600
   actttgtttt gttttgatgt ttgggaactc aatgttatta acttcttatt atgcttcttc     660
   tttggtaatt atttggggta ttctggcaat gaaaccacat ttcctgaaaa taaatgtatc     720
   tgaacttagt ttatgggtta ttcaaggatg tttttggtta tttggaactg tcatacttaa     780
   atacttgaca tctaaaattt ttggtattgc agatgacgct catattggca acttactaac     840
   atcaaaattc tttagttata aggattttga tactttattg tatacctgtg cagcggagtt     900
   tgactttatg gaaaaagaga ctccactgag atacacaaag acattattgc ttccagttgt     960
   tcttgtagtg tttgttgcta ttgttagaaa gattattagt gatatgtggg gtgtcttagc    1020
   taaacaacag acacatgtaa gaaaacacca gtttgatcat ggagagctgg tttaccatgc    1080
   attgcaattg ttagcatata cagcccttgg tattttaatt atgagactaa aactcttctt    1140
   gacaccacac atgtgtgtta tggcatcact gatctgctca agacagctat ttggatggct    1200
   cttttgcaaa gtacatcctg gtgctattgt gtttgctata ttagcagcaa tgtcaataca    1260
   aggttcagca aatctgcaaa cccagtggaa tattgtaggg gagttcagca atttgcccca    1320
   agaagaactt atagaatgga tcaaatatag tactaaacca gatgcagtgt ttgcgggtgc    1380
   catgcccacg atggcaagtg ttaagctctc tgcacttcgg cccattgtga atcatccaca 1440
   ttatgaagac gcaggcttga gagccagaac aaaaatagta tactcaatgt atagtcggaa 1500
   agcagccgaa gaagtgaagc gagaactgat aaagttaaaa gtgaactatt acattctaga 1560
   agagtcatgg tgtgtaagaa gatccaagcc tggttgcagt atgcctgaaa tttgggatgt 1620
   agaagatcct gccaatgctg ggaaaactcc cttatgtaac ctcttggtga aggattccaa 1680
   acctcacttc accactgtat tccagaacag tgtttacaaa gtcctagaag ttgtaaaaga 1740
   atgactgcta catgacctgc tgcctacgga gaactacatc tgtaatggtt ttaatgtttt 1800
   gctaagtcat gtgttgttca tatcccaaaa acttttatag gtaactgttt tcaaatagaa 1860
   aacgttttat ttggtcaatt tgaatgtcat tctaattata aaaatgactt acacctttat 1920
   caattggtta ctatttcaat gcacccttta aaatttgcta tgcaaatgag tatatgcttg 1980
   tacttgactt taatatttgt gctaaagtga gcaaagctac ctgtataaag aaaacacagt 2040
   gggttgtgac aaggatgaca tgaaaataca ggacaattct gacaatgtag ggctgatttt 2100
   tatagtgtaa gaactattaa tgccccttgc ttcttttttc tgcctcttgc tcttgtcttt 2160
   tggacatttc agtgattgta agttcttcgg tcatgtcagc ccctgtcatc aacttgagtt 2220
   acagtagatg gggcagacat ggagtgtttg ctatataaaa ctatctgttt gttttacttc 2280
   cttgtgcgct ttttgttctc tgttctcttg ttaatgaagc ttttcctgcc cattattaat 2340
   ccaaactctt ggaccttgtg gttaggaaat tcccttaact tccagccata tggcattatc 2400
   gtgtctcttt ctctctctct cttgctctct ctcttctcct cttccccata ttttctgtca 2460
   aataagtact gtttactcat ttagttgctt atcaagtact tattcttggt tttaaaaaaa 2520
   attaatggta actgtatttt tctcattttt agcattattc aaatgtttat attttaatac 2580
   ctttaaacca ctttaaagtt ttttcatgtt taattatagt tttaagaaaa actattttga 2640
   acaaccccaa atatagtgca tctagaaact aatgtatatt tgattagaca tcatttatag 2700
   tggaacagta gactgtagta catggtaatt tttcttttac tattaagata caataaaaca 2760
   tgactaattt tgctgtcaaa aatgtaaaga ataatgataa atggagtttt ttatatttta 2820
   cttttaagat tgcctgtctt taataagaca aagccttaag ccttatgtta taattttggt 2880
   tctaaaaacc atcatttcag tataaggaat aagtatattt cgtcctcctc tttagttttt 2940
   ttcttcctat ttatttttat tttgaaaaat ttctacacct tctttgaatt ccttgtatga 3000
   atttttgttt cttagaagtt aatttgtgtg aaatgagatt cttcaaaacg atgaaacctc 3060
   atagctctga gaaaaggttt tagggtttta aattctaagc aaagcgtgac tatggctgac 3120
   agactacaca tttaattata cagcttctct ttcttaacca caggcagatt aacctcattg 3180
   tggattgtcc ttcagacctt agtcctcagg catggtttct ggtgcccact cctggaagcc 3240
   gctgttccct ttctaccttc ttaccagagc ccaagggcag gcctggtccc ggggaagcag 3300
   cagcttgctg acataagtca gctgcaaagg ctgaggagtg tgccctcaga gaagcaccgc   3360
   cccccagtct tgtgccagcg cctagagccg cagctcccag ggatgctcct tccctggagg   3420
   cagcccagga gagggactct ggcagcgttc ttcagatttg tggccactgt ttctcatttg   3480
   ctggttgact gtttttattt cttaggcttt tgctagtttt agaaaatagg gaagcagccc   3540
   ttgatttgtg gattaaaagc aacatttgag cgatgatgca caacagtcca ggaaaatggg   3600
   cggtggacac ttgaggctga ggatgggagt tgacatgagc agggagaggg aggtgcgcgc   3660
   tgcttatctg tgattgttgc tcacctgagt gtggctgatt gtgtacatcc agcagttaca   3720
   atttttaaaa attatacttt tacatttatt ttatattttt ctcaccccca gtaatttcct   3780
   tccaaagaag ttcacatgta ataagtagaa attctgtata ggaaaaaagc attaaaaata   3840
   ctattataac tgcttcattt gctgggaacc attaaaagta atataaatta gctttttcca   3900
   gaaggatcct tttgtagcag tgtttatgaa tgtaaccccc agcaaaatat ggctatatat   3960
   taggggagcc agtttggagc agaggcctga aggtccctgc tatgcagccg tggccacagc   4020
   tcgcagccca agcactgtgg agcatccaca cctttgatgg caatgcagat tggtagcagg   4080
   ttccataggc gtacaaaaca gtattaaagc tcagtgtttt gcatattgtt agcatttaca   4140
   aatatttttg ctttagtatg aggaaagtaa ggatgggcaa agaagcgatc aaaatagcta   4200
   ttgctacaac attttcgaaa acaaagttgg ggctgtattt ctttaaaaag ataagcctct   4260
   aaaaatgctt ggcaaaaaaa atatagtgtt aaaataggcc agtgatatta atgagaaaat   4320
   gaaagtatgt atcaggaata aagtgatatt gcataggagt attgtatttt tatgaatttt   4380
   atgccagttg tttacatgta ctatatatgt taaattaaaa aaaatcatga gaaatg       4436

### SEQUENCE LISTING

<110> Bionomics Limited
<120> P14
<130> Angiogenesis Microarray Genes
<160> 58
<170> PatentIn version 3.2
<210> 1
   <211> 369
   <212> DNA
   <213> Homo sapiens
<400> 1
   tttccttttt aaaaattaaa ctaagtggtt tattactgga aatgagataa ttttctttac     60
   tatgcaaaaa gaattttaaa agccctttat ttcaaaatgt tttccatgct ttggccccca    120
   atatcttcaa tattaaaata aaaatgatat atttcatcac aaaaagaact gtgaaaaata    180
   aaaatgtctt aaaatctgaa ataccttttg aaatattact gcctagtcac tcaaaaattt     240
   atgtaactta caattctggt ttaatttctt aatatattat acatctacca agacaatttt    300
   cccaaatact taaattttaa gagggcaatc ttcttgacat gcaatagtgt aaaagcatgt    360
   tttattgta                                                            369
<210> 2
   <211> 834
   <212> DNA
   <213> Homo sapiens
<400> 2
   agtaccaatt atttgcacac tttggaaatg tgaatgtttc agcaatgttt catagcttga     60
   aaaacaccgt ataatttggt catctaacac ctagttattg attcaagaga aatgttattc    120
   tgttggcact gagaaggctg ttgaatgagg aaggattaga tacatatttt atgaaggtca    180
   tctttagaac aatacatagg ctgagttaag atactggatt tgaattattg aacattactg    240
   ggtatcacaa aataaaagta atgcttctgg gggactttac taaaaataca aattctcatt    300
   tatatttgta tttgtattgt agttcaaggt tggaactcag aagtgtatat tttaacattg    360
   gtggcctttg ataacattgt aaaggaaaga caagttaaag tgaaccaaca aataaagaga    420
   ctgtggaaag tatccctatg gaatgtaaaa aggatcttaa gcgggactaa ttgtggaaat    480
   gtaaggatcg agctttgaaa ttattgcaaa ggcataagta gaataaaatg ctgactaatc    540
   cacaaaatag gcgtttacga ggataattcc caggtttgga ctctgcactg ttgaatacta    600
   ctcgaactac tcaaatggaa aattctgata ggacgcaatt actcagcgtt aagcgcggcc    660
   aaccaaaccc tggagttcac cctggccttt cctgagattt aagggcgttt tcgctgaaac    720
   acctacgtag caactgggct cgggagatgg aacgaaaagg gccttttcaa atcttgtcgc    780
   cccaaaagaa acttgaaact actcgcagaa cggaggccct cccccatcat aaaa          834
<210> 3
   <211> 162
   <212> DNA
   <213> Homo sapiens
<400> 3
   ggcaggtacc ctcattgaaa tagaggatag agattataaa ttattttttt ctgtcttctt 60
   tttacctaga taagaattta ccacatttag tctggtaaat aaccatctgt atggtcacag 120
   agagccacac agaaatgaaa aaaaaaaaaa aaaaaaaaaa aa 162
<210> 4
   <211> 501
   <212> DNA
   <213> Homo sapiens
<400> 4
   ggcaggtact tgaagtatat tctctttttg gattgctgtc taattttaat cacaaaatac 60
   agaatgtaaa ctatcaacac cattttgcct actatgcatt ttgcacttta atagctgcta 120
   cttcaaaaaa cagaactaaa ggcccattct ttggaaccat ttcacatttt acaactaaga 180
   agcaatacca agaataaaca aattcatata actcatcaat atgtattgac tatttatctt 240
   aatttttttc tttcttaaat atcacgatta aaaaaaacat tggcagcatt tataagaact 300
   aagtagttat aagtagttca ctaagaatga cctcatcaca ggttttagct gcaacaatat 360
   aaaactaaag aggtcaaatt tattctccag gctgcgacta ctttttcttt tctttttcct 420
   tgaagcatag cttactttga ttttaaccct tcctcaccac ccctcttcct ctaagtgtaa 480
   tcaagcaagg aaaattccac t 501
<210> 5
   <211> 460
   <212> DNA
   <213> Homo sapiens
<400> 5
   caggtaccat ctctccagca tgttccagcc tttatcagtc agtccaggct tctataacaa 60
   aatatcatgg actgaattgc ttacacaaca gacatttctc acaattgtag aagctggaaa 120
   gtgcaagatt aaggtgctag caaatttgtt tcctggtgag ggtcctcttc cgggcttgct 180
   gtatcctcac atagcagata gagagagaaa gagagagaga gagagcactc tatagactct 240
   cttcctgttc ttataaggac actaattccg ttgatgaggg ccctatgctc atgactttgt 300
   ctaaacctaa ttaattctca aaggcctcac ttcccaatac tgtcacattg tggggtaggg 360
   ctttaacata tgaaatttga gggaaaagga atgttcagtc tagaacaaag ccccatacat 420
   ttcaaaaagc tgtgggacag gagggaatgg ggatggtatt 460
<210> 6
   <211> 282
   <212> DNA
   <213> Homo sapiens
<400> 6
   tacttttttt tttttttttt ttttttcctt taataagcat cgtgtttata tataaatggc 60
   ttacattttt ccatgtccat atatgagtca cacatgatga aatgcttgat gacttactcc 120
   tttttaaact aggtgcactg tgggacacct tttatctcag tgcctaaatt accattgcca 180
   tataataaca gcactcaaat taagaaccgt ttccactaaa attctatttt taagaagcaa 240
   tattcatttg ttgctctact atgcttcttt ttccatgcag ta 282
<210> 7
   <211> 740
   <212> DNA
   <213> Homo sapiens
<400> 7
   ttttaaattt ttgaggttat agagtttcca taaaaagtca tcagtgataa taaattttat 60
   ttgactttct acttattccg gattctgcac gagctgacat ataaacacgt tacttatgga 120
   tcactttgag agaggaaaca ctattcagaa gatttaaatc ccagaattta atccattgct 180
   ttattgcaag tttacagaag acttgtcttc agttttatct gcagtagttg atctattgac 240
   tttaaatgtt gaaaacttat actaatgttg cttactgtcc aaaattattc ctgggagata 300
   aacattcaga ttttctacaa taaccactga ctggcccctt agcagccatt ttaaatgtaa 360
   atttgatgat attgcccaaa catccccagt ccagttcaac tttcagatcc tcaagcctac 420
   catagtcctt tgtgtttgat atttctttct tatccttaaa tgtggtagct ccatgagcac 480
   agggacctgt gttttgattc actgtctgca tacttcactt tgcagaacat ttttaaggtc 540
   catgcatgtt gtagcatgta tcagaactag actcctttta tggctaaata ttccattgcc 600
   tgcgtatagc atatttatcc attcagtttt caatggacac ttggggygtt tccacctttt 660
   ggctcttacg aataatgctg caataaatat tgtcatrtaa gtatctgttc aagttcaaaa 720
   aaaaaaaaaa aaaaaaaaaa 740
<210> 8
   <211> 1811
   <212> DNA
   <213> Homo sapiens
<400> 8
   gtgagggaga cagtagatgg cctgggatga cttgagtcca tcatactatt gcttggcagg 60
   tgtcctcccc catgtttgat tcaaattcca tgagtgacct acctttcccc aggaatggga 120
   ctgagagggt agtctccagc aactcagtct gcacagggct ccccgttcag gctgcctttg 180
   gtggttgtgc ttttgtaagt ttctttctct gcacttcgac ttacctctga atcagaaagc 240
   aagcccagca ggtgaatgag ggatgtctgc ttggcattgc ccaatctaac cagggaggct 300
   ggctggccac ccactgtccg ctagagggga gagctagcag gtgttggtat gaactcagga 360
   atagaaacac gaggcctttt taaatacgag ggagaagaat ccatgatgca tacctgtaac 420
   cccctagaac ccaagtgcca gaattcctag atgctgcttc tgtttgaaca aaatgtcact 480
   gcttttacac ttgaaaaaaa cacactcgaa aaatgttcaa ctccatgaaa aatatttttt 540
   ggctttaaga aattgtttgg tgtttaactg tttcctttga ttgccattcc accagtaaat 600
   tgttggttga tttgcactgc actctggggt tggggttggg aggggagggt ccttatacag 660
   agccgaacct ggggttgctc aggaagtggg ccagggaatg tggaagtcgt tgacattgcc 720
   tgggccaaaa gagtgggaga tagtttttct cccctcagcc cactcctggt agcacctgtc 780
   gccagcctgg tacaaagcca ggcctttttc ttctgtgagc atctcatcac tgtccagcag 840
   caggtggaaa caagggggac aacaaccaga cctatttttt tctcccccat tttttccaaa 900
   ttttgctgtg ccaaatgttt aaaattttat aaatatgaat ctattgaaat ttccttaatc 960
   aagagcttct tcgtgtaaag tttgcttttt tagctataga acaaagaaaa cagtaaatat 1020
   ctcttaatgg catccagcct tgctgagctc accttttttc ctgaagaatg ggtaggagtg 1080
   aaatatttaa tgtaaacatt tcaccaagtc cctttaccct aattttgaag ctgcattaaa 1140
   cccaactcac taacacaggg aatgattgca ccctagtcct ctgtgggcca agaaactttc 1200
   agaagcatta aaaaaactag ttgaagtata tcacttctca ccaagtgggt agagtcagtt 1260
   ggctgtttgt cccttgtttt ttatttattc cataattatg tttgtgcttt ttgttttgta 1320
   aacagtaatg gaacgtacat ttttattttg tttagaagac aactttgatt caatctttca 1380
   agaactgttc cattcttggt ttcttcttag ggggataaaa agttaccagt taatttgttt 1440
   tgagatattt aagcattctt tgagttatag aattgtgatg cagggatttg tgaatgagac 1500
   atttacatgt gaaaggtgac ttcactagtt acctgcttga gcagagtaaa gtgtgtatat 1560
   gtacataaaa tgtaagtaat cttaactcca ttgtgcagtg ccttttagat gttccgcttt 1620
   ctataaagtc ttcaaatttt tgcatatata ttatatatat atatgtaatg ttatagaaat 1680
   atatgtataa tatacatatt ttttccaggg gtatctgata gctctgtatt ttgttatgga 1740
   agttgaaaga aaaaagtatt ttacctcaga aattaaataa aaaaatactt ttaagtaaaa 1800
   aaaaaaaaaa a 1811
<210> 9
   <211> 608
   <212> DNA
   <213> Homo sapiens
<400> 9
   tacagagtct ctctcatcac tttcatagca ggaccctact taccgataat tcatagcata   60
   cctcccctta ttttaaaact ctatgatagc tgatttccta gctgtagcaa tcaggattct  120
   tagaaagaat cgaaactgaa tttagctaac taaggaagcg gatttcatta aaaatattgg  180
   attagtttac agaatcagta gtggagaacc aggattgcat aaaaggtaag aaccaaggga  240
   gactggtcaa aagaaataat ttgcccagaa agtcaccacc aacaattcta ataatgggca  300
   cacgatgctg acaatattgc tagattccaa agtctgtgga gtttaatcag gcaagagcta  360
   atggctggcc aagccttagt cattagtcac atgctccctc agtagcatct acgatctttg  420
   gattcagtag cagaaaatac ataccgcgtt ttaagttcca tacctttctt ttgtccccca  480
   aagaaagcag gtttatacgc tgagtcacca aaaacaaaac aaaaaacacc cagcaatatt  540
   caaatattca ataatgtcat caaaaatttc atgataccca aaggttttgt gtgtgcatta  600
   gcaatgta                                                           608
<210> 10
   <211> 383
   <212> DNA
   <213> Homo sapiens
<400> 10
   ggagctcccc gcggtggcgg ccgcccgggc aggtacgcgg gggccctaca taaaaaagat   60
   tattaaggct aggtgtagtg gctcaggtct gtaattttag cacttgggga gccaaggcga  120
   gaagatcacc tgagctcagg agttccagac atacttgggc aacatagtga gaccttgtct  180
   ctacaaaaaa atttaaaaaa aaaaaaatct gggcatggtg gcatatgcct gtaagtccta  240
   gctactcagg aggctgaggt gggaggatca cttgagctca gggggttgag gctgcagtga  300
   gccatggttt gtgccactgc actccagcct gggtgacaat gaggttctgt ctcaaaaaaa  360
   aaaaaaaaaa aaaaaaagta cct                                          383
<210> 11
   <211> 652
   <212> DNA
   <213> Homo sapiens
<400> 11
   gcaggtactc tgtttctgag ggtgggtttt ttgttgttcc tgttctttta ctcttttata 60
   tacatacaga ttggtgaacc taattttctc actttttcag tttatttctt tgggacagtt 120
   ttatggcctt agaaatatac tagtcattat atttttttca ttaaattcct ttatccagga 180
   attatgaata aatctggcat tatcaaaaag tttagtcact tcaactccat gaataggaaa 240
   caaatggtaa cttgacatca gaaaaaaaaa ttgaaccctg ttcattttct ctatctgaat 300
   tttattatat tttatgctac ttattttaaa aatattactt attttattgt atttggtgaa 360
   ttttatttta ttctacttct ctgaaaaaat ggtcatctat tcatagttcg tgttattcct 420
   ccagttaact aactgatgca gcagttgatg ggaacttctc tgtaacacta gatacatgag 480
   atattataat atttttcttt tttctttagt tgttgtatag tcgtctatct cagctagtag 540
   aatttaaagt gaaaggcata aaaatctgag gtgcagatat gaaacacagc agttgtttta 600
   tatataaata tctggtcact tctgtaagca attttttttt tacttactca ga 652
<210> 12
   <211> 539
   <212> DNA
   <213> Homo sapiens
<400> 12
   gatgaccata atcataactt ttggaaactg ggggatcttt gcccaccttc ccaataaatc 60
   tgggagtata cgccatttta taatctaact atgtcttatt gtccaaaata acattaccaa 120
   accctgacat ttagtttcaa aagtaaattc agcaataaaa ttggatccca gactttaagg 180
   agactatgta tgtcagtgtt acatctattg catctaatat aggaccctag ctgtgaaggc 240
   ctttaagaat agtataattt tatggaagca tgcctttttt ggcttgtaaa gactaccctc 300
   ttgactttca gaaactaagt catcaccttc acgtataatt aaatcaaaga tgcaaattct 360
   cacctaaaat ttactcatac atattttaga aacccataga actttaattt ctttttttct 420
   atacggcaac ccaaatcctt atgaatgcga taaggtaata agaaagatgt cttaaagaaa 480
   aaacagaata actgttactt atgggggcca gacaatcagc tgttttttac ttaagggta 539
<210> 13
   <211> 1524
   <212> DNA
   <213> Homo sapiens
<400> 13
   cccacgcgtc ctgtaccctt tctattacat gtattcgatg ttttcataag cctaggaaac 60
   atcgattcct ttttaataat gtcaatctga ttatttaaag aggtaacaat tatctgttaa 120
   tgcttggaaa aacaagtagg gttgccttgg aggccaggct tcttagttca ttcaaaaata 180
   ttccttggat ttatgccatg tattaagcat ttttagcccc cagtattaca actgtgaacc 240
   aaacggataa ggccctaacc attttcagca ttctctttgg atggggtggg attggggact 300
   taattaaaat agagatatag aaaaataggc atctaaataa gataataagt gtggggttga 360
   aatgaagcat ctaacaatag ttgaagttag aagtaatatt ttacagtatt gtaacctcta 420
   tttaagtttg ggtattagtt acagatagca taaaaaagcc ttaatttttc actttccttg 480
   ctggcaaagg tacatttatt tagactgtcc atttaaagta atgtttaaca taaacattac 540
   tgtgaaaaac attccattac atattcccaa gcaaatgagc tgcatcttct ttactgtatt 600
   ttacaattta gtacaacagt tttaggcctc aatcttaaca tcactggtat tttaaatttg 660
   gcaatgaata tgaaattact tttgacttac agattgatta tattattact ttgaaaatgc 720
   attaatttct tagaaaagtt tggagcctct atcttttttt gagttaatac ttaaattctc 780
   attacttata ttaatagcct gtactaagtg aaaatattat ttatgcaagt aaacaagtca 840
   ctataggctt ttaagacttt tctttaattt tgagattttg tcatcaaagt ttaaattttt 900
   tacctactgt ccacttaaat ataatttaac agtttgtaaa gtgaaatagt tttaagtatg 960
   atgtatgatg cacctgcata taaatgaaaa tggggtgcac aaagacactt tactatggga 1020
   actgtactgg aagatttatg aaagcatgtg aaattgcacc taaaattgtg ttattagtga 1080
   ctataagcag caatgctaaa tttattgtac ttgatgaatg aatgtattta gtcacagtta 1140
   ctttggttta aatgtataaa tgtctttagg gttttttttt aaatgtgttt gtaatttgta 1200
   ctattgtggg ggtatacttg gactgcaggg gttattgtca atgtgtgatt tgtgttttta 1260
   ttttatagaa tcatctaatg tgatatacca atttttataa gtgatattta cataattcta 1320
   ataactgtat atttgacaac ctattaaaat gttttgcatt ggaacttttt tcattaattg 1380
   taacatgcta acgtggttta aaaataactc attcattcaa cagatattga atgttgtgta 1440
   tcagaaacta taggaatcac tggggaatca aagatgacaa agtgcatcag tggaaagatc 1500
   catgctctca cgacgaattg ggga 1524
<210> 14
   <211> 1886
   <212> DNA
   <213> Homo sapiens
<400> 14
   tttaaaatgg acctggttga aaagagattt cattgattct ttttaatgat attggaaaat     60
   atttatgcaa ttttattttt taaccttttc tccatttcat tagttatact atttactttg    120
   ataatcacaa aactatgtgg aagaaatcat tctgattata ttttatgaaa gagaggggta    180
   gagtaaattt tttaattaga caatgttgat atgcagaaat ctctcaaaag aaattgcaaa    240
   ggaaaaatta cgttgagatg aaatgttgag aaataaggag tatcttaaaa acctaatttc    300
   ttacactttt tagagcaatc aacttatttc caaattatta tttcatgctg ttgtttccat    360
   gaattgtgtt tttagcatag tatatacttt tctaccacgt tagcatttac actctaatta    420
   ctgaaaataa aggaagaagt tggtgaataa aagccaggga aactgggttt attctcattt    480
   tccaatagca ctaatgtata atccctttaa ccttccatat ggtgctttgt gtgatgagta    540
   tataacattc atatacgtta ttagtgcctt atttttttca cttgacttta tttcttatct    600
   aaagagccat atattttttg tgagtgttct ttggtccctc tatttaaagt gctaaggaga    660
   aagcatatct ttagggatgt atataggctg cgtttgctac cttaatctac tgtattatct    720
   ctaaaacagg ttagccaatg tatttttctt aaattcattg ttcattttta agctaaccta    780
   tacaaatgga tattaaacac cagaaattaa agccattaaa attgaattgt actttaaaac    840
   cataaaaaat gctaattaag tttagaacag gagttgaaat gactctcagt tttatctata    900
   gctccaatta ggacaaaatt actttacaaa aagcattaaa aataaaagta actgtatggt    960
   acttcaggaa tttgaccagt tccttacaac ttattcttgg caatattttg tcagtaatat   1020
   actttcatac agatgctaga gaaccatggt gtctgctatt tacatgttat ttgttcccag   1080
   tgagctttat caacatcaac cctgtttata taatatgtta ttttgctctt cttgatctgt   1140
   gccttttgtt ttagctttat acaaaatgct aggtttgtta tattctttat gtatcataat   1200
   atataattca acttttcctg atgaatgtat gctgaataat taaactaaac catgtgctta   1260
   gaaaagacaa agacaggttg tatatgcagt tatgtgtaaa tatttgacaa agaaaaaaaa   1320
   aggaaaagaa tcttcctgcc aacaggtagg tcctgctgga caggacctga aaattaagag   1380
   attttttttt ttaagcagtg aaatttaaaa atctattacc tcaagaattt tcagtcgata   1440
   tgtcaatgta ctgttccttc tcagtaagta cagtgtcaca tgctgctggc ggtaaaagca   1500
   acccaagtgt cagatcacac aaattttagg gacaagtctt tcaaaagacg gacaccttgt   1560
   gatattttgg agtctaattt ccgacttttc gtaatgtccc aatgaccttt tgttgtggtt   1620
   gggcttttat aaaaaattat tttcggttgg ttgttgtaga atggaagagt aatcttaatt   1680
   atgaaatcag attgcatgga agaatataaa gacactcact cggatgaaaa ctataacggt   1740
   tttcaaacta ttttgatggt cattgtacga gctattgtat ggattactgt ggagtgctgt   1800
   ttaccacatg atgtgtgcaa tacatcatgc aatgttattt agcaatgcgt aataaaagtt   1860
   tttaaaagag aaaaaaaaaa aaaaaa                                        1886
<210> 15
   <211> 674
   <212> DNA
   <213> Homo sapiens
<400> 15
   ccgggcgggc ggcagcaggc acgtgcgcag ggtacacatt gagatgtata tgtacagtta     60
   caccgaatga agaaacaaaa taatgggcgc atcgcaacac cggatcctaa cattcatgat    120
   gtaagtcctc tatcccagca tatgaccctc aataaataga tgagtgggaa cagcggatca    180
   tgaatatgac caactctctc actcgtgcgt atacaaattg tagtccttcc tatgaataaa    240
   gcagtggagc ataggacttc ttggacaact tcatacccag aaacttgggg agaagccaaa    300
   ccaggaggga cttcatgcaa tgggaaggct ttgcttctct acaggcttgg aattaacccc    360
   agggaaccat tatctccacc tgttgctttg agcacgtaaa gtccctgaat gaaatgctta    420
   attttactta atattaaaat tggcagataa attgcaggac actcaaatgt gaatttcata    480
   taaataatga atgaattttg atcacatccc aaatactgca gaggacatat ttatattaaa    540
   atttgttttc attgttcatc tgaaattcaa atttatccgg gcatcctgta tttgtatctg    600
   ttaggtctaa agagacaagc catatacaac tgttttttgt aactctcatc ccaaaaaaaa    660
   aaaaaaaaaa aaaa                                                      674
<210> 16
   <211> 1011
   <212> DNA
   <213> Homo sapiens
<400> 16
   taaagttcct ccatagaaat gtgtggcatg ggaacagagc ctaatacttt ctgaataaga     60
   tattaacagg cttgtagtgg atgctgggaa tttagaaagc tctgtttttt ttttttttct    120
   aaagcctccc agtagaatgt cagactaata aatggactgc atatttccac agtgttttta    180
   tgacatgttt cacacactgt cttggtgcag atatgagaaa gcccttgtcc acagcccagg    240
   ttctgactca ctcattaatt ttaccttgta ttcaagtttt cttggggtgg atctagacta    300
   gaatagaata gaataagccg acctttaact acagtcctta aatgaggaaa tctccaccct    360
   gggccagctg tgccaggaat cccgacgaga tggaactccg ctgtggttta taggcagggc    420
   tgattttcag gggggagtgc acccatctgg actccctgct gttcccatca atctattcca    480
   gccctggtac ccctggacca ccctactgtc cagcaactaa aggattaaca ggagccacct    540
   ccaggagcct ccgggaccct tcagaaggtg tctgatttga tggatcagag gctaggccac    600
   tgccagctct ctgcacatat tttacatatc acccatttgc aggtgttgaa gaccggtaag    660
   tgcccaggag ctggcaggtg ttgcaaacat gtcagtgtac ctggtggggg cgctcctgcc    720
   cagctgatag gagacagctt ctacccagcc cacccctttg gtggctacac cagcttggca    780
   cagtctctca ttgctgaaga gcatcccccc gccaactccg cccaatcccc acctccagtc 840
   ttgactactt ttcattggtt tgtttgttta aattgacatc tcctgatttt caaaaccttg 900
   ctgattaaca aagcaaaaag agcacacaca aaaaaaaaaa aaaaaaaaaa aggacgcggc 960
   cgcaagctta ttccctttag tgagggttaa ttttagcttg gcactggccg t 1011
<210> 17
   <211> 1279
   <212> DNA
   <213> Homo sapiens
<400> 17
   tcttgccttc attaatagat ttactaactg tatttatagt aataaaaatc ctttatgtct 60
   ttatatgtac ctgtgccaac atacacaaat taatgtaata tcctatatta agggaataaa 120
   atataaaaat tatatgatta tctcaataga tgcagaaaaa tacttaacaa agttcaacat 180
   gttttcatga tacaaactct caacaaaata catatagaag aaaatttcct caacataact 240
   aaagccattt ataaaaagcc aactgccaac ataattttct ttgaagtttt aaagaccatg 300
   gatcgatttt tctctttatg tagcctaatc tgatttttat attcatatct gttaaggcat 360
   acacaataga aattatatgt tgacactacc tttagagtcc aggctcctta acctatacat 420
   gcatttgtaa ctgtatctta cctgttattt gatgctaaaa gatggaaagg ttttcctcta 480
   agatacagta caaggcaagg tacaaagatg cccaccctct gcacttctat tcaacagttc 540
   tattaaaatt gctggaaata ctagcaagga caatcagata agaaaaataa atagaagcca 600
   tccaaatgca aaaggaagca aaattatctt tgtttgcaga tgatatgatc ctctgtgtag 660
   aaaatcctta aaatgtcaca aaaattgtta gaactaatga tcgaattcag taaagtagca 720
   ggacacaaaa tcaacatcaa aaatcagttg catttattta catcaataat gatctatctg 780
   taaaagaaat caagatggtc agacagtttt tataagataa tctgtggaaa atatgtaaaa 840
   cttcttctga tattatacca ttcttgtatt tgaatgatat gacctatttg tatattattt 900
   tttaaatgca ttgctagatt ttgttggtta cattttaagt atattgaatc tatactcata 960
   tataacattg ttagatgtat tcacagtgaa ctgcaacttt tatcattatg ttcttactat 1020
   ttgttatatt tttcttttta ttgaactatc aaagaagttt tctacattat tttcatttat 1080
   gtataaaatc atttatatat aacatttata cgtaaaatca tttttatata aaaatataca 1140
   taatttatga ataaatgatc ttacattatt tctacaaaaa aaaaaaaaaa aaaaattcct 1200
   gcggccgcaa gcttattccc tttagtgagg gttaatttta gcttggcact ggccgtcttt 1260
   tacaacacgc catcacaac 1279
<210> 18
   <211> 2290
   <212> DNA
   <213> Homo sapiens
<400> 18
   acaaacttag aaaacttcat gtactctgga gttcacaggt gttttccttt tgcatgttgc 60
   cttctccttt cctcctatca caaagaatgc ttctattatc actcccagtc acttaaatgc 120
   cattctgctt ctttagcagg aaaaaatacc tgaatgaatt ctcttacaga aagacagttg 180
   gactgaattt gctttttaca atcagtgtgg aaaatatcat agtcaattga ggcaatcagt 240
   agaaatctca ctaaaagttt gtaccccctg agtatgggta acagcttaaa cggcaagaat 300
   atataaaagt agcaacagtt tagtgggagc caaaaagcac taggttaaat atcatactac 360
   gtttttaaag acttgataaa tgtgtacact tgaacaacca tccagaggga tagttacttg 420
   agcattacag agtagtggaa ctcaaaatct aagattcatc tgggatactt ctaaaagaaa 480
   tgtcagagtt tggaatctca ctcttaaaaa tgtgtattcc caggctaggc atggtggctc 540
   acgcctgtaa tcctagcact ttgggaggcc aaggcgggtg aatcacttga agtcaggaga 600
   tcaagaccag cttggtcgac atggtgaaac cccgtctcta ctaaaagtac aaaaaaatta 660
   gctgggcatg atggcgtgtg cctataatcc cacctactcg ggaggctgag gcaggagaat 720
   cgcttgaacc tgggaggcgg aagttgcagt gagccgagat ggcgccactg tagtccagtc 780
   tgggcgacag agtgagattc tgcctcaaaa taaataaata aataaaaatt tgtattccat 840
   tgatttgggt agacaccagg aatgtgcatt tctaacargc tttccaggcg atcctatagt 900
   aagtcatctg tggactactt taagaaactc ttctatagag aatggagttg gattaataat 960
   aggtgatttt ttacactgga ctgattcaca agaacctaaa cagtagtcca tgaagctgct 1020
   catctgtggt aactatttgg ccccgtctca ctctgaaagc agcaggagat gttgtttact 1080
   ttgtttctat cccctttgtc tggagattaa ttttggaatg aaagtttttc tctctatgcc 1140
   attcctggtt cttttccaaa gcctcataca agaggattag gtcacaatgc atgcattacc 1200
   ttttaaaaga atgcgatatt gataccgatg cttacttttt tttttttact acttgtttta 1260
[…]
   ttctgaagtt aattaaactc attgcactaa atgtgtctcc cttggtatag tggaggattt 1740
   gaggattgga atatagagta gagtgcttgc ttaagcctgg gagcccatct ttatagctat 1800
   ttgatgtaag aaaagagaca tggccatttc taaactatat aaggtgagtg tgtctattcc 1860
   cagcagatat aaaggaaaaa ggaaactttt ttgattccca ccttcccagc ctcacctagc 1920
   catcttccag cctcaaatat agagatgtta gtgcaaggtc ctgggctcta ggtgatcatt 1980
   tcataagtcc tttacagata aagaaaaagt agtgtttgta tgtttgtttt taagtaaccc 2040
   caaaacaaat ttatattgta ttcagcaaaa ttggaattca ggtgtttaat tttagaacat 2100
   gaagtgcctg ctgttttaag cattgacttg tataaaaaga attgcatgtc tccagtaagc 2160
   ttatgggttt tctcattttt aggtatatgg cttttaatca tgtaaagtga aacattagtt 2220
   ttcttgcatt ttattacagg ttctttgttg caataaagat gctgctgaaa tcaaaaaaaa 2280
   aaaaaaaaaa 2290
<210> 19
   <211> 1055
   <212> DNA
   <213> Homo sapiens
<400> 19
   gtttctagaa tggggctagt tgtatctagg caaatgggta actcttaatt attgtatgac 60
   atttttttct gtatttaatg gttaagattt agaggaaggg gcaatatgtg aaaaaaacac 120
   gttatgagaa tgtttcacat ttagaccttg agaaattggt atttatttat gccagttacc 180
   tatgtctctg aggtgccaag aggatgaatt aatattttat ttcacttgtg gagatattca 240
   gaaacattaa agtccttggt cctcatgaga aaagaatttt cttgagactt gaaagcatta 300
   ctgcagacat tttgctaaca cattttttgt gagctgtgtt aattgcacct acctttatca 360
   gtcagaagtt ttgctgattt aaatcagatc ccagctaacc caagcggggc tgcactgaac 420
   gccacatctc tattgcccag atttcccagg ggactctgga aattaaccag aagccctcct 480
   gctcgaagac caactcttga ggcgtatatg atttgcatag aggttccaat gtgccaagtt 540
   cttagaagtt tctgggagcc atacagatga ttactattcc aaatgttctc atcttgggtt 600
   ccaccctttt tattcaatga gatttgactt ttctactttt tcactcccat cttcaccaca 660
   ccaaaattca tatccaacag tcattgtgta tattatgaaa gttggtctca atttagtcaa 720
   acctgggttt caggggatat agctcacata aaaaggaatg ggaggtatca ttatatggaa 780
   atacctgctg tgtgccagag catcctttaa ggattttgtt ttctgttgat atttttcaaa 840
   ccctccactt tacagaagag gaaagtcagg cccagagagg tcaagaaaat cactttcagg 900
   caaacaaggt cacatggaaa atagggattc ctgtgaactc accctgtgtt cttctactcc 960
   ttcatgctca aaaactctcc cggaaatcaa atgaagcccc tgctgaaggg gggcctggtt 1020
   aaaggggatt cacaaaaagc caaaccctat ttgtc 1055
<210> 20
   <211> 398
   <212> DNA
   <213> Homo sapiens
<400> 20
   atatttaaaa agtaaaacaa actatttgga aaaaaatgac agtaaaaaca ataggaaagc 60
   ttgtaaactt cgggcttact atcaaaatca ctaccttaaa gtaatttact acttttaaaa 120
   taaagaataa aattaaataa ttcggcattt aaataataag gttagaaagc tgcaactatt 180
   ctatatggaa aaggatagaa aagaaaaaga aaaacaatga atacaattag taaatccaaa 240
   aggaggatga tgataactgt tgcaacctgg ggagtgggaa aaatgtgact ctttataaca 300
   aaaactctac ttttgtgcct gatgaacact tcataaacaa atttaaaata tatagataaa 360
   tccaatgatc agtcccttaa aagggcaaat gtaaagta 398
<210> 21
   <211> 443
   <212> DNA
   <213> Homo sapiens
<400> 21
   cgcggtggcg gccgaggtac tttttttttt tttttttttt tttttggtgt ctgacagaat 60
   tactagctat gccattaaag cccagatttt ctttattgga agttttaaac tactaattca 120
   atttatttac tctatatagg tctatttaga ttttctactt cttttggagt cagttttggt 180
   aattagtatc tttcaaagaa tttgttcatt tcatctaaat tgccaaattg gttgttgtaa 240
   acttgttcat aatattcctt tatcttttta gtatttatag tatctgtagt aatacccatt 300
   ctttcattct cagtatttct aatttacatc ccttcttgtt cattttagct aaagatttac 360
   taaactcttt aaagaaccag cttttgattt ccttgatttc tgttctgatc tttattactc 420
   cctttcttct acttactttg gta 443
<210> 22
   <211> 385
   <212> DNA
   <213> Homo sapiens
<400> 22
   ggcggccccc ggggcggtac cacgctcccg actgttcgag gtacgctctg ctctctcagt 60
   agccaaacag ataacagcca gtacgttgtt atccgtattt tcgttcgttg ttttaacctc 120
   agtagcacat ttggaccttg ggtgttatta tatctttgtc tttataataa actcatgcac 180
   ttgcttctaa gattgatact tggagcttgc aacttattca ctcataaatg taacttacat 240
   atgctttcta gattttgcac ttcttcccct gcaccttggt agacctacat tgaattaatt 300
   aatttaatta gttcaaacat ttattgaaga agaactatgc agtaggctcc caggatcaag 360
   cgatgactta gtctaccttc aaaag 385
<210> 23
   <211> 1014
   <212> DNA
   <213> Homo sapiens
<400> 23
   ggtacttttt tttttttttt tttttttcta taaaatcatc atcttcacca ccttatcagg 60
   gggcaacttt tatttaataa cacttgaagt gtttttactt ggtcaattat ggataaataa 120
   tccggtttgc ctttctacgc ttacttaaag gaggctgtga attgttggaa aaagtccaaa 180
   actgcaatta tttgatccac tattaactcc ttagtctctc ttgttgactt ccatatcaac 240
   atgtaacata tggggtattc taagcccttt gcacgcccaa ttcaggaaat ccaactgctg 300
   ataggactcc ccctcctccc tatcccattt ccccctactc ttagcaaaac tgcctctttc 360
   ccaaactcat ctcctatcca cctcaaaaat gttgtccttt attttccttt cctcctatct 420
   caaaaaagtg tttctccgaa ttttcaagat tagttcctat atctgtgcta ctgatagccc 480
   ctcttgcttt atcagttatt ctgtttttcc tcttcagtct ctcattctac ccttcttctt 540
   tcacaagtgc tcaaaagtaa acagatgaac agacaaatcg gtcgctaagt tttacttact 600
   tgcctgccac aaactctcat gcacttattc ttctgcattt cagactctac caatttaact 660
   taggctctca ttatccctcc ctcaaaaagt aaccaaacat ttctgtatag ccaatctaat 720
   cctccaacaa acatcttaat cataatactc atgtattcaa aattcatcca caagcccatc 780
   atagaattca aacacttttt tttctggctt tcaaattttt caacaatccg gctctgatca 840
   acctatctca taaaatgata ctgcattgct atctattatc ttttatctac ctacaaaatt 900
   caataaaata taatctacaa atttcagtaa aatacaatcc aaaaaatccc tcccttttcc 960
   ctactacctt aatttctccg tcctttattg aaaaaagtgc cctttaaact gaaa 1014
<210> 24
   <211> 207
   <212> DNA
   <213> Homo sapiens
<400> 24
   acatagaagg gaaaaatggc ttgcttattt caaatagttt tgaggatgaa gcactatgat 60
   ggagatccac tgaggtaaaa aggatcctca gcttttttga ttcagaaatg aaatattgat 120
   gatttcaatc aagattgatc aaaacctgat gaaaatatcc tgataatcaa tgggaagtag 180
   aattacagct tattgtgaga gtcaggg 207
<210> 25
   <211> 248
   <212> DNA
   <213> Homo sapiens
<400> 25
   tttttttttt ttttttttta aagttggagc tactctgccc atgaggtagc caccctttta 60
   ttccttttct taaaacaagt tggaaaacca ttgctataga taaacattcg atttcattct 120
   tttagaaata atacatacct atatattaat atattcaata tatgtatcag tcatatatat 180
   attcattaaa atgttaacac ccaagtattt agattaaatg ctgttatttg gcttataaat 240
   taacagta 248
<210> 26
   <211> 1132
   <212> DNA
   <213> Homo sapiens
<400> 26
   cgcccgggca ggtacagata tgtaaatatt ccaaaatcca gaaaaaaaat ccaaaatctg 60
   atatatttct ggtcccgggc atttcagata agggataccc aacatgtgtt tgtaactttc 120
   aatactaatg aaattagtaa attttgtttt ttacattagg tgcctaaaac tcttgatttt 180
   acaccaaaaa gtaatagaac aaaaataaaa agctattata tggaatggca tcagagtcac 240
   tctggagcaa caggaagcta actctgtata tcaaccataa tagccttatt actcccagaa 300
   ggacatagtt agaagcattt ctggttactc ttcatattaa aatctttggt gttttggctt 360
   caatacactc ccttaatggg tgttattatt ccattgtaat gaaataatat tccatggtag 420
   cagaaggaat gcttaaattc tgccttactg ttaaattcta tatagatact tgggttggtc 480
   aaattaattt ggtgcttcac ccaaagcccc aatgtatttg aactttaatc tttcatagga 540
   atctagaaaa gcacacaatt taattaccac tactattcat tatcaaagat tgcatgaatg 600
   aggtttaaaa caatagaaag aaataaaact ttagccccct attgctagat gttctggccc 660
   atgagagagg cagggcaact taagagcttt ggaggcagac agggcaagac tgtaaatctt 720
   ggagcaacaa tttattggct atgtagcctt ggaaaggtat cattattttt ctctacctca 780
   gttcactagg tggaaaaatg gaataacagc atctaactca acaaatgttg attattacaa 840
   aaactaacga ttcaaaaagc taatataaat aaagcatcat gttatgcatc tttctcataa 900
   aaataaatct taatgaaaag catcaacttt tagctttatc cataaaaaat accagacaaa 960
   acaaaaaaaa aattctaaaa atcaaaaaaa cttcctaatc ctgaaaaagt aaaattaatt 1020
   atgttctttt gttttcaaaa aaaaaaatta ttaattttgg aaaaaacatt ttaaaaacca 1080
   aaattttcct ttttaacttt ttcccaaaaa tttttttttt ttcccccaaa ac 1132
<210> 27
   <211> 646
   <212> DNA
   <213> Homo sapiens
<400> 27
   tcacttaaat ataataatga tgttattaat aaacgttagc ttgagatcaa agtgtcaaca      60
   aggcattctt ttctcttttg gtggtggtag atttcaccca catattttat gcatctataa     120
   caagtcccac atagctggct gattactgag ctttgagcag gtagcactgg tcactatgca     180
   cagattagta tgattactga gctttgagca gcttgaacag atcactatga tataggctct     240
   ggaaaggttg tgcagatatg cctgatgcca aggcactggc aaaattgttg gtaggctggc     300
   aagttagttc cttctcaagg aaaaaaaagt gtgaatgatc ctataggtta ctagacagac     360
   atgcagtttc cctttggaga aggttgactt tggactttga gaaatacatt tttttctttt     420
   taatctaaag agatttgaaa ataatagatg tccaattttt gtttacctat tctgaactat     480
   aaggcacatt gctttgttct tctgtgcctc agtttacccc ttttagtaaa ggaggtctat     540
   tttaatcagt gagagcttca ggtagctgga ggaatgcttc aggaaacaca aaacacaatg     600
   aaaaaattgt tgaatcttaa gctgcatgtt gtggaaaaga actgta                    646
<210> 28
   <211> 417
   <212> DNA
   <213> Homo sapiens
<400> 28
   aggtactttt tttttttttt ttttttcttt tttttttttt ttttttttgt aactataata      60
   tttatcattt taattgatgt gtatagtttt acaaaatcaa atagtaatag aaacaaaata     120
   gcatcccttg tgcctcccct ccctattgct ccctggtctt atacagtcat aaaaagtcac     180
   aagaaaccac gtacaaattg ttaagccatt tcttctgcca tttaccttcc aattcctaag     240
   gagtatgcat gtgctgttat ttattttaaa cattatcttt ttttctccct gtcttgacag     300
   atgttatcta actcaagtgc actttgctct ccctccccca ttcccctggt gtaagcatac     360
   cacacagtct ttgagttcac attattattt gtgtcatata gatactgttc acatgag       417
<210> 29
   <211> 628
   <212> DNA
   <213> Homo sapiens
<400> 29
   cacttgaagt caaaatgcaa tcacagttct tttgatcttt ttaatcagtt tctccattgg 60
   acatttttct tgcattgcct cagtaacaga atagaggaga tatattttga tgttactttt 120
   ctcttgtcac ttttgaaggg tggtgttgaa gagatgccac catcagttgg atatataaaa 180
   gaaatagcca aataaagaga taatctgtct ctcccatcag cccctgaaca attgatagtg 240
   gaaaataatg taatacaatt tataatgttc aagataaaag cttattgata attttttatc 300
   aataaccatc aaggatgatg taattacaaa actcaagatg atttgacaga atttttcaaa 360
   ttatttaaat ctaaatattc agactgaata aacttcaaat tagttaagtg gattcatttg 420
   atagatgctt tattttactc attagccaat aaaactctta agagttttct taaggtctta 480
   ggacagacag atttatgctt cagtgacaaa aatcaagaat ttaatcagtt acacaaggag 540
   aaagtatgtg tgtatgtgtg tgtatacaca taaccagata ttctcctaag tttttcaaaa 600
   taataaaaac agatattttg ggattcat 628
<210> 30
   <211> 291
   <212> DNA
   <213> Homo sapiens
<400> 30
   gtactttttg taacatacct gtgatgctgc tccaacatcc ctgctaagag ctttgctgat 60
   tttaacaagt atgtgattcc actgctgtaa gccaagggta aacttgattc caccaaattt 120
   gcttaagtgt tacttctctg tattaaagct tcaagttcac tcactgtcta accaatgata 180
   cacagctacc tgaagacatt aaaatctttc ataaatttat tctcaatttt tttcttctta 240
   taaattacta gcatatcagg ataaagaata aagtgtgtga agaggcaaaa t 291
<210> 31
   <211> 1154
   <212> DNA
   <213> Homo sapiens
<400> 31
   tttggagggt aacaaaattt cagggacata taaaagaagt tgcttattta ttagacaaac 60
   aaaagtcacc cagcaaatat gccatggcta ttaaaaatag attgtccagg gaggaagaat     120
   aaaccactaa ttaaacactt ttgtttctct tttttttaag ccattaatac tgatacctag     180
   agagaaaaat aaaaaccaga aatactttca aaagccaagc atgatagact agcatttatc     240
   agttaccttt ttattttcaa tcctcaagaa atgcaatcgc ccaaataaat tttaagtata     300
   ccaaaacact aagaaattac aaaactcaat agcaaaataa ctcacgtgtt acaaccataa     360
   ttacttctat aactagaaaa ttattatcct cataggaaat cctggcaaat tgctgaaaac     420
   atttctcttc tgagttaatc taggagggaa aaaaaataaa acttcagagt ccagtcactt     480
   tgaagtcctt atgcccaaaa agacattatc tccatcaatt gtctacatgc gaataatttc     540
   aaatgtcttt gtctgtgcaa cagccacatt tccttctctt atgatgtttt tcttctctcc     600
   ctatcccatt ttcattttca cagctgtttc tggagaataa aaatgaagga acactttctt     660
   gctctgcctc tccatgctcc actttttaca agtctttgtc tgatttacta cagagaacta     720
   taaccaaacc caaaaaaaga atggcaagcg aaagtaggag agaagagtgt gcttttgcgc     780
   tcagaagcca ccacttctca ggtggaaaat aagaatgtca tgccatgatg acattctgcc     840
   ttgtgtcaca atgattaata gtcataattt tttcccatta atgggagtaa atgcaaccaa     900
   gtcgatggaa tctgaccaat tttgcacagc atgggtcagt ggagtgctat tattttggat     960
   ggagaaatga gaaccaaaaa ggaattattt ttccatttag cacctaaagt ggaaaaatta    1020
   attttttcat cccccttttt tttccaacct aaaaaaaaaa aaaagaaaat ttttcaaaaa   1080
   aaaaaaaatt ttttttccaa aattttaccc ccttttaaac caagtgtttt cttttttaaa   1140
   ttgggaaccc cggc                                                     1154
<210> 32
   <211> 875
   <212> DNA
   <213> Homo sapiens
<400> 32
   tactgtctta gtctgtttgt gctgctatca caaaatacca gaaactgggt aatttataaa 60
   taacataaat ttatttctca tggttctgga ggctggaaag tccaaaataa agatgctggc 120
   aggttgagtc tggtgagggc ccagactttg cttccaaaat ggcattgtgt tgctacatcc 180
   ttcagcaggg actaatgcta tgtcctcaca tggcagaagg gccagaaagg gagaagggcc 240
   tagccttagt tcccttgaat ccttttatag gggcactaat ccattcacaa gggttccacc 300
   ctcatgacct aatcacctcc caaaggccct acatcttaat atcatcacat tggcaaataa 360
   cttttaatat atgaattttg tggggatgca ttcagaccat agaagatgct ctttgtttcc 420
   cttgaacatt actgtttttt aaggatttgg cctcaaattc cttctcttga tagatctcct 480
   tttggaatta cctattctga aaatgctcat gtttcactaa ttttcctatg acttttacat 540
   ctgtatctct catcttgaat tccagttcta cattttcaaa tgactcatac gtggacaatg 600
   cactgtcagc agaaactcaa catttgtaag gcaggactct tcaatttttc ctcattttga 660
   tctcctccat accctttaat tgtcaaggac atacttttta ggtgtccaga ttcaaaattt 720
   tagaagcatc ctaaataccc tcctttccct aatccccact tatcccctca gtgacttaat 780
   cttggaaatc attatgatca ttttcataaa tgtcctttta tttttaaaat gattttgaag 840
   attttttttc cataaaaacc tttttttttt ctggc 875
<210> 33
   <211> 1270
   <212> DNA
   <213> Homo sapiens
<400> 33
   cggtggcatt gcactcgttc gttgttagat atggaaaacc aattgactgt tgtatattaa 60
   acttgtatcg tgcaaccttg ctataaatta gttccaggat tttttttttt ttttttggtt 120
   ggttcctcgt ggatttagct atgtagtcac atgtatcgtc acttgtgaaa gaaaaacaca 180
   atgttattac ttccatcgct catatatcca atcagtaaac ttaccatagt gaaaagaata 240
   tagctaccac agctagtctc tcacatgaca gataagatga atggatagcg catcaatggt 300
   tgacaacttc tctaaagtaa atacgcgctg gctgtctctt ttcaagatta acacaagata 360
   tgtgtcaaac ctacaaacaa gaggacgctt attccctcgc ctctacagtt attgaatacc 420
   tggagctgca cgacttctat atcaaactta cagaccccgt ctacctctag ggaggagcac 480
   cggactcgct caagacatcg tatgaaagag tctctataac ccgctgctct attcgtcgtc 540
   ccagagtgac cgtcccagcc ctagctacat ctgtggaaat acccgagtta atacccctta 600
   gcaggttatc ccccgttcga tcaacaagtt gtgatagccc aaaaaagcgc ccgacacaaa 660
   atcaacctta gcctaactat tagaaacaaa cgaccaaccg cccaggtgca cggtaaccat 720
   gaaccccacc gctatcaccc tcccgctttg acgcggtgca caaccgcccc ccgcgctcca 780
   ccaactaccc cttattctgg gaaccacctc tcgccccgct cctctttcca ttacccccat 840
   tacaattgtc ccggttccct ccacgcccta cttatccacc taccaaaagc ccctaaactt 900
   ccgaaacgcc tctcttccca ccagttccac aaatatattt caatttatca ccgggacaac 960
   caccccccca caaaaatctt tattcaccgg cctctgggat ttacctgata ttgcgcttca 1020
   accctctcac caccagacac atttttatca tcctatcgcg caggattgct atgccccccc 1080
   tacctacttg aaagaaatcg aatctaaggt ttgtctacaa ccttacccac cactccccac 1140
   tccaagcagc cacatatctt gtccacattt atttttctta atcctcagta atggaaaccg 1200
   cttccgctca tatcacccaa cctacactgt ctcggtttga gcgctgtatt ggttgttgct 1260
   tagacaatac 1270
<210> 34
   <211> 501
   <212> DNA
   <213> Homo sapiens
<400> 34
   ggggagctcc ccgcggtggc ggccgcccgt ggcatgtaca ctgtaaagtg acaaaacatg 60
   actgaaagaa atttttaaga agctaataaa aaagggcgga cagcccatct tcatggattg 120
   ggaagactta atactgttaa gatggcaata cttctcacct tagtctatag attcaatgca 180
   acccacatcc cactttcctt tactgcagaa attgacaagc tgatccaaaa aattgtaagg 240
   aaatggcaag ggatccataa tagacaaaac aattttgaaa aagaataaaa tcaaagggct 300
   ttgtaacact aaggttataa aactcttata agaaaataaa ggaggaaatc tttgtgacct 360
   tatctcaggc aaatagtttc tttgatatga catcaaaagc atacgtaata aaagaaaaaa 420
   atagagatat tgaacttcat caaaattaaa aacttttgca tttcaactat caaaaaaaaa 480
   aaaaaaaaaa aaaaagtacc t 501
<210> 35
   <211> 373
   <212> DNA
   <213> Homo sapiens
<400> 35
   ccatagatag aaatattaat acccatgaaa gagaggacaa tgaaaggttt gtatcatttg 60
   tatgtcacaa gtcaactttt ttcaatcact cattattagt ttaactgtaa aaaattattt 120
   acatttagcg tgaaactttc ctgtattctc aacatatttc cttcgcgtag aaaagcaaac 180
   ctccagttct ctgttctttg cttggatact tgccagtttg taactcagct atcaaacagt 240
   aaagctcaca aaacacttat taaaatgact aaaatccaaa acaccaagag cacagcatgc 300
   tggtgagatg tggagcaaca agaactttca ttcattcact aatgctggca atacaaaatg 360
   gtacctgccc ggg 373
<210> 36
   <211> 563
   <212> DNA
   <213> Homo sapiens
<400> 36
   gggaaagaaa gggggagcgc cggccggggg gcggccgccc gggggcaagt actctagaag 60
   tgagcattag cgtgtgtgtt attgttcgtc gaattcacat cgcaagttac aacatgtgct 120
   ctatcaacac gatttaaaga aatatggggt tggaaattaa aaatgtgcta tttcaaaaaa 180
   caaatctcaa cgcccattcc ttgggaccat ttctcttgtt agaacgaata agcacgcacc 240
   agaaagaaac gaattcataa aactgatcaa tatgtattga ctaagtatct taattttttt 300
   atttcgtaat atcacgatta aaaaaaacat tgagagcatt tataagaact aagatgttat 360
   aagtaattca cccggaatga ccgtcctcac aggtcgcagc tgcaacactc tataactaag 420
   gaggacaaat ttattcgcca ggctgcaact actcgtgcgt ttctttatcc ttgaagcata 480
   gctaactttg attataaccc ttcctcacaa cgcctcttcc tgtaagtgta cacaaacaag 540
   gaaaattcca ctttgaagta tct 563
<210> 37
   <211> 1280
   <212> DNA
   <213> Homo sapiens
<400> 37
   gcgcgcggcg gggaggcaca gcctgtttaa ctcgttaatg ctgcatcagt gatagatatt 60
   tcgcgaagcg ggaatacaca atgtgtccag ggtggtgctc ttttgtgttg tttgtatggt 120
   tcagagggga gggtggctat atccttgcga gctaaggaga tgctcaggct tacacactac 180
   ctggtgtcca gcgaatgact catcttacag catcacgaat atgttggcgt acaccaatac 240
   cttatccacc cgttctgact gccttaaatg ggtattacag gagaagactt tgatccatcg 300
   catcctgaac gtcatcattg gtgagaggac aaccgtcctt gtactatgac catcttctaa 360
   acagacatgc atcggaccag aggaagatcg gctgacatcg tgtatctgcg tgcctatgcg 420
   tttccgctgt agctccttag ccctgtggac acagtatttg gactgcctgt taagttacgt 480
   aggcactgct tgacgggttc tcccacacga agatcctcac gttgacacag atttcctgtt 540
   catcttatgt gtctggtcaa cttgttgccc cggcccaaca tgacctatcc cttctacggg 600
   ttcacaatag taccgttccc taacagaatt cctcacgaac tgttaccagt ctacaggaaa 660
   agccattacc ctgactctct gactttgcca cactcaagat cccctgctct acgacaaggg 720
   aagcagacgt cagcacctat agtttacacg tttgattctt tcttgttact ttgacggtca 780
   tacagtgtta tgcggaaagt atcacaaact aaccgaacgt gccccagcag acatcctccg 840
   caaatcgaaa ccgctcccca ttcgagttga catgtacacc aacctctctt ccctgtctat 900
   gcctatatta tgtcagcaga attctttaaa aaaattagtc agtttgcctc cgctttcggt 960
   tggactctcg cacccaaagc gtaccgaacc cttaaccctc cagatgcccc cgcgtgtctc 1020
   cacttgtctc caatcctgag ggctccgccc cctacccttt cctctatcgc aaaccccctt 1080
   atcctcattg acggcgcttt taatccacta tgtggccccc cccgtccctc gcttgaaatc 1140
   accggctttc atccccctat cccattcccc cacacccttc atggtgctgg gtccccccga 1200
   cccccttttc tccactcata ggccagacac ctcatctatc tacgaataac cccgccgccc 1260
   cctcctcatt tttataataa 1280
<210> 38
   <211> 953
   <212> DNA
   <213> Homo sapiens
<400> 38
   tggtagtaat gtagtattta agataaaggg gtttagatag taaagataga aaaagaataa    60
   aaaaaatatt gattggataa ttaaaaagag attttggtga tggggatgaa gatctatata   120
   gagtgtgatt ggtagggttg tggagtatga gttgatgatt atgggaatga taaggatgta   180
   gttggtggag tattattgaa tttggtatta aaagtaatgg ataaaggtgg gtgaaggaat   240
   tttattgtac aaaaatggtt ggggcgtctc acgggggtga gtcaacttac cctattagac   300
   gtttatactc tctatcacat ctgggcgtca tgacaacaaa tagccgccgc tattcccaag   360
   tccctcaact gggtggcgtg atacgcgccc gggacccccc ctttaacagt cgtttgcccg   420
   cgggagtgtt gtatgcttcc cccttgacgc cagtgcaaaa acctagatcc taggccccta   480
   acgcaattat tatgacacta tctcacacca tgggcatgcg ggcattcacg tcgataccat   540
   taaccttgtt tatttcccct gtgttgcgac caatattgtt ttttaggcca gagcctttac   600
   tcaaggggtt tagccatttc cgcgcccgta gcatacgcca tcccctctcc taatagtagc   660
   attaactgca acgaagacat cctacacgtc cctgttatac atcattccac acaaattttc   720
   gtcccccaac tacctatgat ttcccctaac attacctcaa actatcgtct ctacaactga   780
   ggagtaatac cacccgtaca acgtcacaag aatggctaat ttctaaaaca tgcgatagcc   840
   tgcgatagac tagaatacac aattcatcta caaaaaaaat ctgacccaat gaaattaata   900
   aacacaataa tgacaatacc acattgccct acacaccagc tacaaaacca ttc          953
<210> 39
   <211> 660
   <212> DNA
   <213> Homo sapiens
<400> 39
   cggccgaggt acattcactt aaatataata atgatgttat taataaacgt tagcttgaga    60
   tcaaagtgtc aacaaggcat tcttttctct tttggtggag gtagatttca cccacatatt   120
   ttatgcatct ataacaagtc ccacatagct ggctgattac tgagctttga gcaggtagca   180
   ctggtcacta tgcacagatt agtatgatta ctgagctttg agcagcttga acagatcact   240
   atgatatagg ctctggaaag gttgtgcaga tatgcctgat gccaaggcac tggcaaaatt   300
   gttggtaggc tggcaagtta gttccttctc aaggaaaaaa aagtgtgaat gatcctatag   360
   gttactagac agacatgcag tttccctttg gagaaggttg actttggact ttgagaaata   420
   catttttttt cttttaatct aaagagattt gaaaataata gatgtccaat ttttgtttac   480
   ctattctgaa ctataaggca cattgctttg ttcttctgtg cctcagttta ccccttttag   540
   taaaggaggt ctattttaat cagtgagagc ttcaggtagc tggaggaatg cttcaggaaa   600
   cacaaaacac aatgaaaaaa ttgttgaatc ttaagctgca tgttgtggaa aagaactgta   660
<210> 40
   <211> 652
   <212> DNA
   <213> Homo sapiens
<400> 40
   tgatgtctga gtaagtaaaa aaaaaaattg cttacagaag tgaccagata tttatatata      60
   aaacaactgc tgtgtttcat atctgcacct cagattttta tacctttcac tttaaattct     120
   actagctgag atagacgact atacaacaac taaagaaaaa gaaaaaatat tataatatct     180
   catgtatcta gtgttacaga gaagttccca tcaactgctg catcagttag ttaactggag     240
   gaataacacg aactatgaat agatgaccat tttttcagag aagtagaata aaataaaatt     300
   caccaaatac aataaaataa gtaatatttt taaaataagt agcataaaat ataataaaat     360
   tcagatagag aaaatgaaca gggttcaatt ttttttctga tgtcaagtta ccatttgttt     420
   cctattcatg gagttgaagt gactaaactt tttgataatg ccagatttat tcataattcc     480
   tggataaagg aatttaatga aaaaaatata atgactagta tatttctaag gccataaaac     540
   tgtcccaaag aaataaactg aaaaagtgag aaaattaggt tcaccaatct gtatgtatat     600
   aaaagagtaa aagaacagga acaacaaaaa acccaccctc agaaacagag ta             652
<210> 41
   <211> 878
   <212> DNA
   <213> Homo sapiens
<400> 41
   ggaatgcatt ggtatgtgtt ctcggctcag aaagctatat gagatccgcg gtcagggagg    60
   atagcaccac catgggatct acaagaactc cgccttctct catgtgggta tctaactcat   120
   caactactct gtcttgagct caaatgggac aggcatactc ttcagcatag ccatcgtaca   180
   attgctcttc tcgagctact atcacccaca ttaaggtctt tcatgtcctg atttagacgc   240
   gagctctgtg cctagcgagg ttattcctac ccctgttgta cgtgcccggg cggacggtca   300
   tgatcatggg catgagacgg gctgatggaa ggctggatct agcaaaatcc tggcgtcaaa   360
   tgctaggttt tccactaaac tatcttaaga caatagagtg tacatctata cgaggagaaa   420
   gttggctatt atactgtcct tgagatcaca tataacagac ctatctcaag gctgtgaaaa   480
   aagtccttta acacgatcaa attatctgtg tagcagacat atatatcaag cagtatagaa   540
   actacttaca catgcaattt acagatatct ctagggaaaa aacaagcact cacgaatagc   600
   agtataactt gaaagagttg agagaacttg cagacgatcc acagactagc tctatagctg   660
   attatacatt aggtagagac tcttgatatg tctgttaact agatgatttt ccaaatatat   720
   ttgtttttcc atgtttccct ttggatccag atatagtcat tacttccttg aagctcacaa   780
   aaccctctga ggatatgaat gactgaaagg aaacatccct catatataaa acatatttcg   840
   ttacacatgt taaactccct tcttaaaaaa tggtggtt                           878
<210> 42
   <211> 572
   <212> DNA
   <213> Homo sapiens
<400> 42
   aggcatttct tattaataat gctagcattt atcctttaca gatcttcact ttatccacaa    60
   gacaatatgt aaattaacaa taaattgatt gtttcattga tcattggttt ctttggcaaa   120
   catttgtcct atgggaagct gaattaaggt aggaaaatac tttttgctac attgctattt   180
   ggggaaacgc tctgatagaa ggtctccata gttggtggaa ttccagaatc caattagcac   240
   ccgaatgaaa tgtaaataat aggtctcaca attttatgtc agatgtgctg tgtatgagcc   300
   cagacatatt ttcacaaatt agttgtccta atgctggttt tattttggtg tattataatt   360
   cacaaaacgc tactgatacc aaacaagata atcaatgtga ttcctggtat gtactaggac   420
   cacagtgatg tgtattgaat aaatgaatac atgaatgaat aaacctcttg atcactgccc   480
   cctagctgtt gaatttcatg atattcacag gtgttcttta ctgccttcat ttctctcctc   540
   ctttaaccaa aaaaaaaaaa ataaaaaaaa aa                                 572
<210> 43
   <211> 330
   <212> DNA
   <213> Homo sapiens
<400> 43
   gctgatcaca ccatgctgtt ttcgtgtttt atggagaagt gttcaggtgt ggctgctgtg 60
   tttgatatta aatctgagat gaacttcagg agatttcctg ctttttatta caaataattt 120
   tggctagggg cagtgttatc ctgttcataa atcaggtgtt ctcagagatt taataattta 180
   gaattcacat gaggatttgg tggaataaac atttcctttc tctgttaagc tgcctcttaa 240
   ctttctatct tcccctttct cttcactgct tccatttttt taagccttct ttaagtcttt 300
   gccaaaaaaa aaaaaagaaa aaaaaaggta 330
<210> 44
   <211> 478
   <212> DNA
   <213> Homo sapiens
<400> 44
   cgggtttatg cctttttgct ttttaaaatt cttttttata tcatgttcaa aaaggtttca 60
   ggagggaata agataagctc atgtgttcaa catactgtgt ttaactgaaa acctctttat 120
   agtcttctaa gattatagct ttaaaatgac taatttaggg tattagatat gccaagttct 180
   atgaagagtt cccttgtaat taaactccac aaaaagtaaa ttcaattgta tttaaaatgc 240
   cttccattta tcccttttag taaactaatt ctgacattcc cagaagttgt ctaaaaattt 300
   gcttctcagt ctatgtaagc ggtaaacata tttttctgga acaaaggtga ttgaagctag 360
   tctgaactaa tcattagcaa gtttgctaaa ataaggttaa gagggaaaac tctctattcc 420
   caggaatcta agacatgatt taaagaaatc ctcagagaac aaaaggctgt caaatcaa 478
<210> 45
   <211> 19
   <212> DNA
   <213> Homo sapiens
<400> 45
   agacagagga catccacct 19
<210> 46
   <211> 19
   <212> DNA
   <213> Homo sapiens
<400> 46
   atcgtgagcc ttcgtttgc 19
<210> 47
   <211> 19
   <212> DNA
   <213> Homo sapiens
<400> 47
   ttatgggaga gagttccct 19
<210> 48
   <211> 19
   <212> DNA
   <213> Homo sapiens
<400> 48
   atatcatagg tgatgggcc 19
<210> 49
   <211> 19
   <212> DNA
   <213> Homo sapiens
<400> 49
   atgttacttg ggttatggt 19
<210> 50
   <211> 19
   <212> DNA
   <213> Homo sapiens
<400> 50
   ctgtgatgct ctttcacac 19
<210> 51
   <211> 64
   <212> PRT
   <213> Homo sapiens
<400> 51
<210> 52
   <211> 63
   <212> PRT
   <213> Homo sapiens
<400> 52
<210> 53
   <211> 42
   <212> PRT
   <213> Homo sapiens
<400> 53
<210> 54
   <211> 98
   <212> PRT
   <213> Homo sapiens
<400> 54
   Met Asp Gln Arg Leu Gly His Cys Gln Leu Ser Ala His Ile Leu His
   1               5                   10                  15
   Ile Thr His Leu Gln Val Leu Lys Thr Gly Lys Cys Pro Gly Ala Gly
               20                  25                  30
   Arg Cys Cys Lys His Val Ser Val Pro Gly Gly Gly Ala Pro Ala Gln
           35                  40                  45
   Leu Ile Gly Asp Ser Phe Tyr Pro Ala His Pro Phe Gly Gly Tyr Thr
       50                  55                  60
   Ser Leu Ala Gln Ser Leu Ile Ala Glu Glu His Pro Pro Ala Asn Ser
   65                  70                  75                  80
   Ala Gln Ser Pro Pro Pro Val Leu Thr Thr Phe His Trp Phe Val Cys
                   85                  90                  95
   Leu Asn
<210> 55
   <211> 62
   <212> PRT
   <213> Homo sapiens
<400> 55
   Ala Ala Pro Gly Ala Val Pro Arg Ser Arg Leu Phe Glu Val Arg Ser
   1               5                   10                  15
   Ala Leu Ser Val Ala Lys Gln Ile Thr Ala Ser Thr Leu Leu Ser Val
               20                  25                  30
   Phe Ser Phe Val Val Leu Thr Ser Val Ala His Leu Asp Leu Gly Cys
           35                  40                  45
   Tyr Tyr Ile Phe Val Phe Ile Ile Asn Ser Cys Thr Cys Phe
       50                  55                  60
<210> 56
   <211> 52
   <212> PRT
   <213> Homo sapiens
<400> 56
<210> 57
   <211> 33
   <212> PRT
   <213> Homo sapiens
<400> 57
<210> 58
   <211> 103
   <212> PRT
   <213> Homo sapiens
<400> 58
   Met Ser Ala Glu Phe Phe Lys Lys Ile Ser Gln Phe Ala Ser Ala Phe
   1               5                   10                  15
   Gly Trp Thr Leu Ala Pro Lys Ala Tyr Arg Thr Leu Asn Pro Pro Asp
               20                  25                  30
   Ala Pro Ala Cys Leu His Leu Ser Pro Ile Leu Arg Ala Pro Pro Pro
           35                  40                  45
   Thr Leu Ser Ser Ile Ala Asn Pro Leu Ile Leu Ile Asp Gly Ala Phe
       50                  55                  60
   Asn Pro Leu Cys Gly Pro Pro Arg Pro Ser Leu Glu Ile Thr Gly Phe
   65                  70                  75                  80
   His Pro Pro Ile Pro Phe Pro His Thr Leu His Gly Ala Gly Ser Pro
                   85                  90                  95
   Arg Pro Pro Phe Leu His Ser
               100

## Description

### Technical Field

The present invention relates to novel nucleic acid sequences ("angiogenic genes") involved in the process of angiogenesis. Each of the angiogenic genes encode a polypeptide that has a role in angiogenesis. In view of the realisation that these genes play a role in angiogenesis, the invention is also concerned with the therapy of pathologies associated with angiogenesis, the screening of drugs for pro- or anti-angiogenic activity, the diagnosis and prognosis of pathologies associated with angiogenesis, and in some cases the use of the nucleic acid sequences to identify and obtain full-length angiogenesis-related genes.

### Background Art

The formation of new blood vessels from pre-existing vessels, a process termed angiogenesis, is essential for normal growth. Important angiogenic processes include those taking place in embryogenesis, renewal of the endometrium, formation and growth of the corpus luteum of pregnancy, wound healing and in the restoration of tissue structure and function after injury.

The formation of new capillaries requires a coordinated series of events mediated through the expression of multiple genes which may have either pro- or anti-angiogenic activities. The process begins with an angiogenic stimulus to existing vasculature, usually mediated by growth factors such as vascular endothelial growth factor or basic fibroblast growth factor. This is followed by degradation of the extracellular matrix, cell adhesion changes (and disruption), an increase in cell permeability, proliferation of endothelial cells (ECs) and migration of ECs towards the site of blood vessel formation. Subsequent processes include capillary tube or lumen formation, stabilisation and differentiation by the migrating ECs.

In the (normal) healthy adult, angiogenesis is virtually arrested and occurs only when needed. However, a number of pathological situations are characterised by enhanced, uncontrolled angiogenesis. These conditions include cancer, rheumatoid arthritis, diabetic retinopathy, psoriasis and cardiovascular diseases such as atherosclerosis. In other pathologies such as ischaemic limb disease or in coronary artery disease, growing new vessels through the promotion of an expanding vasculature would be of benefit.

A number of *in vitro* assays have been established which are thought to mimic angiogenesis and these have provided important tools to examine the mechanisms by which the angiogenic process takes place and the genes most likely to be involved.

Lumen formation is a key step in angiogenesis. The presence of vacuoles within ECs undergoing angiogenesis have been reported and their involvement in lumen formation has been postulated (Folkman and Haudenschild, 1980; Gamble et al., 1993). The general mechanism of lumen formation suggested by Folkman and Haudenschild (1980), has been that vacuoles form within the cytoplasm of a number of aligned ECs which are later converted to a tube. The union of adjacent tubes results in the formation of a continuous unicellular capillary lumen. However, little is known about the changes in cell morphology leading to lumen formation or the signals required for ECs to construct this feature.

An *in vitro* model of angiogenesis has been created from human umbilical vein ECs plated onto a 3 dimensional collagen matrix (Gamble et al., 1993). In the presence of phorbol myristate acetate (PMA) these cells form capillary tubes within 24 hours. With the addition of anti-integrin antibodies, the usually unicellular tubes (thought to reflect an immature, poorly differentiated phenotype) are converted to form a multicellular lumen through the inhibition of cell-matrix interactions and promotion of cell-cell interactions. This model has subsequently allowed the investigation of the morphological events which occur in lumen formation.

For the treatment of diseases associated with angiogenesis, understanding the molecular genetic mechanisms of the process is of paramount importance. The use of the *in vitro* model described above (Gamble et al., 1993), a model that reflects the critical events that occur during angiogenesis *in vivo* in a time dependant and broadly synchronous manner, has provided a tool for the identification of the key genes involved.
WO00/58495 discloses a cDNA of a gene (number 3) which is identical to BN0802 and GenBank accession AB020684.
WO02/086443 and WO01/53312 disclose the nucleic acid and polypeptide sequence of BNO802 and GenBank accession AB020684.
Glienke et al (2000) European Journal of Biochemistry vol 267, no, 9, p2820-2830 and Kahn et al (2000) American Journal of Pathology vol 156, no 6, p1887-1900 disclose information regarding genes that are differentially expressed during angiogenesis.

### Disclosure of the Invention

A first aspect of the present invention provides a modulator of expression of a polypeptide encoded by a nucleic acid molecule identified herein as SEQ ID NO: 59 (BNO802) wherein the modulator is an antisense nucleic acid molecule.

A second aspect of the present invention provides a modulator of expression of a polypeptide encoded by a nucleic acid molecule identified herein as SEQ ID NO: 59 (BNO802) wherein the modulator is a nucleic acid which is a complement of at least a portion of said the nucleic acid molecule and is capable of modulating expression or levels of the nucleic acid molecule. The nucleic acid is preferably an RNA molecule that hybridizes with the mRNA encoded by said nucleic acid molecule, or a short interfering oligonucleotide that hybridizes with the mRNA encoded by said nucleic acid molecule.

A third aspect of the present invention provides a modulator of expression of a polypeptide encoded by a nucleic acid molecule identified herein as SEQ ID NO: 59 (BNO802) wherein the modulator is an antibody capable of binding the polypeptide. The antibody is preferably a full human antibody, or may be selected from the group consisting of a monoclonal antibody, a humanised antibody, a chimaeric antibody or an antibody fragment including a Fab fragment, (Fab')₂ fragment, Fv fragment, single chain antibodies and single domain antibodies.

A fourth aspect of the present invention provides use of a nucleic acid molecule identified herein as SEQ ID NO: 59 (BNO802), or a fragment thereof which encodes a polypeptide that plays a role in angiogenesis process, for the screening of candidate pharmaceutical compounds useful in the treatment of an angiogenesis-related disorder.

A fifth aspect of the present invention provides use of a polypeptide identified herein as SEQ ID NO: 59 (BNO802), or a fragment thereof that plays a role in an angiogenesis process, for the screening of candidate pharmaceutical compounds useful in the treatment of an angiogenesis-related disorder.

A sixth aspect of the present invention provides use of cell comprising an expression vector comprising a nucleic acid molecule identified herein as SEQ ID NO: 59 (BNO802), or a fragment thereof which encodes a polypeptide that plays a role in an angiogenesis process, for the screening of candidate pharmaceutical compounds useful in the treatment of an angiogenesis-related disorder.

In the uses according to any one of the fourth, fifth and sixth aspects the angiogenesis-related disorder preferably involves uncontrolled or enhanced angiogenesis, or is preferably a disorder in which to decreased vasculature is of benefit. The disorder may be selected from the group consisting of cancer, rheumatoid arthritis, diabetic retinopathy, psoriasis and cardiovascular diseases such as atherosclerosis.

In the uses according to any one of the fourth, fifth and sixth aspects the angiogenesis related disorder preferably involves inappropriately arrested or decreased angiogenesis, or is preferably a disorder in which an expanding vasculature is of benefit. The disorder may be selected from the group consisting of ischaemic limb diseases or coronary artery disease.

A seventh aspect of the present invention provides a method of screening for a candidate pharmaceutical compound useful in the treatment of an angiogenesis-related disorder, comprising the steps of:
(1) providing a polypeptide identified herein as SEQ ID NO: 59 (BNO802), or a fragment thereof that plays a role in an angiogenesis process;
(2) adding a candidate pharmaceutical compound to said polypeptide, and
(3) determining the binding of said candidate compound to said polypeptide;
wherein a compound that binds to the polypeptide is a candidate pharmaceutical compound.

An eighth aspect of the present invention provides a method of screening for a candidate pharmaceutical compound useful in the treatment of an angiogenesis-related disorder, comprising the steps of:
(1) providing a cell comprising an expression vector comprising a nucleic acid molecule identified herein as SEQ ID NO: 59 (BNO802), or a fragment thereof which encodes a polypeptide that plays a role in angiogenesis process;
(2) adding a candidate pharmaceutical compound to said cell; and
(3) determining the effect of said candidate pharmaceutical compound on the functional properties of said cell;
wherein a compound that modulates angiogenesis in a system comprising said cell is a candidate pharmaceutical compound.

A ninth aspect of the present invention provides a method of screening for a candidate pharmaceutical compound useful in the treatment of an angiogenesis-related disorder, comprising the steps of:
(1) providing a cell comprising an expression vector comprising a nucleic acid molecule identified herein as SEQ ID NO: 59 (BN0802), or a fragment thereof which encodes a polypeptide that plays a role in angiogenesis process;
(2) adding a candidate pharmaceutical compound to said cell; and
(3) determining the effect of said candidate pharmaceutical compound on the expression of the nucleic acid molecule that is part of the expression vector in said cell;
wherein a compound that alters the expression of the nucleic acid molecule that is part of the expression vector in said cell is a candidate pharmaceutical compound.

A tenth aspect of the present invention provides a method of screening for a candidate pharmaceutical compound useful in the treatment of an angiogenesis-related disorder, comprising the steps of:
(1) providing a cell comprising an expression vector comprising a nucleic acid molecule identified herein as SEQ ID NO: 59 (BNO802), or a fragment thereof which encodes a polypeptide that plays a role in angiogenesis process;
(2) adding a candidate pharmaceutical compound to said cell; and
(3) determining the effect of said candidate pharmaceutical compound on the expression or activity of the polypeptide encoded by the nucleic acid molecule that is part of the expression vector in said cell;
wherein a compound that alters the expression or activity of the polypeptide encoded by the nucleic acid molecule that is part of the expression vector in said cell is a candidate pharmaceutical compound.

In the method according to any one of the seventh to tenth aspects of the present invention the angiogenesis-related disorder preferably involves uncontrolled or enhanced angiogenesis, or is preferably a disorder in which a decreased vasculature is of benefit.

In the method according to any one of the seventh to tenth aspects of the present invention the disorder is selected from the group consisting of cancer, rheumatoid arthritis, diabetic retinopathy, psoriasis and cardiovascular diseases such as atherolsclerosis.

In the method according to any one of the seventh to tenth aspects of the present invention the angiogenesis-related disorder involves inappropriately arrested or decreased angiogenesis, or is a disorder in which an expanding vasculature is of benefit.

In the method according to any one of the seventh to tenth aspects of the present invention the disorder is selected form the group consisting of ischaemic limb disease or coronary artery disease.

Total RNA from cells harvested at specific time points from a biological model, in this case the Gamble et al (1993) model for angiogenesis, were used to prepare cDNAs, which were subjected to a novel process incorporating suppression subtractive hybridization (SSH) to identify cDNAs derived from differentially expressed genes.

A method for the identification of a gene differentially expressed in an *in vitro* model of a biological system, comprises the steps of:
(1) harvesting cells from the model system at predetermined time points;
(2) obtaining total RNA from the cells harvested at each time point;
(3) preparing cDNA from the total RNA from each time point to provide a plurality of pools of cDNA;
(4) performing a suppression subtractive hybridization (SSH) on the cDNA pools from each time point sequentially so as to progressively amplify cDNAs derived from genes differentially expressed from one time period to the next.

Thus, up-regulation of a gene whose expression subsequently remains up-regulated at the same level will be detected (and the cDNA amplified) only in the first time period where the level cDNA is elevated, as the quantity of cDNA in pools from the subsequent time points will be the same. This reduction in redundancy reduces the possibility that other genes of lower representation in the cell mRNA expression pool will be masked. The model system may be an *in vitro* model for angiogenesis (Gamble *et al.,* 1993).

Those cDNAs identified to be differentially expressed in the SSH process were cloned and subjected to microarray analysis, which lead to the identification of a number of genes that are up-regulated in their expression during the angiogenesis process.

A method for the identification of a gene up-regulated in an *in vitro* model of a biological system, comprises the steps of:
(1) harvesting cells from the model system at predetermined time points;
(2) obtaining total RNA from the cells harvested at each time point;
(3) preparing cDNA from the total RNA from each time point to provide a plurality of pools of cDNA;
(4) performing a suppression subtractive hybridization (SSH) on the cDNA pools from each time point sequentially so as to progressively amplify cDNAs derived from genes differentially expressed from one time period to the next.
(5) cloning the amplified cDNAs;
(6) locating DNA from each clone on a microarray;
(7) generating antisense RNA by reverse transcription of total RNA from cells harvested from the *in vitro* model at said predetermined time intervals and labelling the antisense RNA; and
(8) probing the microarray with labelled antisense RNA from 0 hours and each of the other time points separately to identify clones containing cDNA derived from genes which are up-regulated at said time points in the *in vitro* model.

Functional analysis of a subset of these up-regulated angiogenic genes and their effect on endothelial cell function and capillary tube formation is described in detail below.

Described below are isolated nucleic acid molecules, which have been shown to be up-regulated in their expression during angiogenesis (see Tables 1 and 2). The isolation of these angiogenic genes has provided novel targets for the treatment of angiogenesis-related disorders.

Following the realisation that the isolated nucleic acid molecules of sequence ID Numbers: 1-44, and those listed in Tables 1 and 2, are up-regulated in their expression during angiogenesis, the isolated nucleic acid molecules as defined by SEQ ID Numbers: 1 to 44, and laid out in Tables 1 and 2, or fragments thereof, play a role in an angiogenic process. Such a process may include, but is not restricted to, embryogenesis, menstrual cycle, wound repair, tumour angiogenesis and exercise induced muscle hypertrophy.

In addition, this work suggested that isolated nucleic acid molecules as defined by SEQ ID Numbers: 1 to 44, and laid out in Tables 1 and 2 (hereinafter referred to as "angiogenic genes", "angiogenic nucleic acid molecules" or "angiogenic polypeptides" for the sake of convenience), or fragments thereof, play a role in diseases associated with the angiogenic process. Diseases may include, but are not restricted to, cancer, rheumatoid arthritis, diabetic retinopathy, psoriasis, and cardiovascular diseases such as atherosclerosis, ischaemic limb disease and coronary artery disease. Useful fragments may include those which are unique and which do not overlap any previously identified genes, unique fragments which do overlap with a known sequence, and fragments which span alternative splice junctions etc.

An isolated nucleic acid molecule that is at least 70% identical to any one of the angiogenic genes of the invention may play a role in the angiogenic process.

Such variants will have preferably at least about 85%, and most preferably at least about 95% sequence identity to the angiogenic genes. Any one of the polynucleotide variants described above can encode an amino acid sequence, which contains at least one functional or structural characteristic of the relevant angiogenic gene.

Sequence identity is typically calculated using the BLAST algorithm, described in Altschul *et al* (1997) with the BLOSUM62 default matrix.

An isolated nucleic acid molecule which hybridizes under stringent conditions with any one of the angiogenic genes may play a role in an angiogenic process.

Hybridization with PCR probes which are capable of detecting polynucleotide sequences, including genomic sequences, may be used to identify nucleic acid sequences which encode the relevant angiogenic gene. The specificity of the probe, whether it is made from a highly specific region, e.g., the 5' regulatory region, or from a less specific region, e.g., a conserved motif, and the stringency of the hybridization or amplification will determine whether the probe identifies only naturally occurring sequences encoding the angiogenic gene, allelic variants, or related sequences.

Probes may also be used for the detection of related sequences, and should preferably have at least 50% sequence identity to any of the angiogenic gene-encoding sequences. The hybridization probes may be DNA or RNA and may be derived from any one of the angiogenic gene sequences or from genomic sequences including promoters, enhancers, and introns of the angiogenic genes.

Means for producing specific hybridization probes for DNAs encoding any one of the angiogenic genes include the cloning of polynucleotide sequences encoding the relevant angiogenic gene or its derivatives into vectors for the production of mRNA probes. Such vectors are known in the art, and are commercially available. Hybridization probes may be labelled by radionuclides such as ³²P or ³⁵S, or by enzymatic labels, such as alkaline phosphatase coupled to the probe via avidin/biotin coupling systems, or other methods known in the art.

Under stringent conditions, hybridization with ³²P labelled probes will most preferably occur at 42°C in 750 mM NaCl, 75 mM trisodium citrate, 2% SDS, 50% formamide, 1X Denhart's, 10% (w/v) dextran sulphate and 100 µg/ml denatured salmon sperm DNA. Useful variations on these conditions will be readily apparent to those skilled in the art. The washing steps which follow hybridization most preferably occur at 65°C in 15 mM NaCl, 1.5 mM trisodium citrate, and 1% SDS. Additional variations on these conditions will be readily apparent to those skilled in the art.

The nucleic acid molecules, or fragments thereof, of the present invention have a nucleotide sequence obtainable from a natural source. They therefore include naturally occurring normal, naturally occurring mutant, naturally occurring polymorphic alleles, differentially spliced transcripts, splice variants etc. Natural sources include animal cells and tissues, body fluids, tissue culture cells etc.

The nucleic acid molecules of the present invention can also be engineered using methods accepted in the art so as to alter the angiogenic gene-encoding sequences for a variety of purposes. These include, but are not limited to, modification of the cloning, processing, and/or expression of the gene product. PCR reassembly of gene fragments and the use of synthetic oligonucleotides allow the engineering of angiogenic gene nucleotide sequences. For example, oligonucleotide-mediated site-directed mutagenesis can introduce mutations that create new restriction sites, alter glycosylation patterns and produce splice variants etc.

As a result of the degeneracy of the genetic code, a number of nucleic acid sequences encoding the angiogenic genes of the invention, some that may have minimal similarity to the nucleic acid sequences of any known and naturally occurring gene, may be produced. Thus, the invention includes each and every possible variation of polynucleotide sequence that could be made by selecting combinations based on possible codon choices. These combinations are made in accordance with the standard triplet genetic code as applied to the polynucleotide sequence of the naturally occurring angiogenic gene, and all such variations are to be considered as being specifically disclosed.

The nucleic acid molecules of this invention are typically DNA molecules, and include cDNA, genomic DNA, synthetic forms, and mixed polymers, both sense and antisense strands, and may be chemically or biochemically modified, or may contain non-natural or derivatised nucleotide bases as will be appreciated by those skilled in the art. Such modifications include labels, methylation, intercalators, alkylators and modified linkages. In some instances it may be advantageous to produce nucleotide sequences encoding an angiogenic gene or its derivatives possessing a substantially different codon usage than that of the naturally occurring gene. For example, codons may be selected to increase the rate of expression of the peptide in a particular prokaryotic or eukaryotic host corresponding with the frequency that the host utilizes particular codons. Other reasons to alter the nucleotide sequence encoding an angiogenic gene or its derivatives without altering the encoded amino acid sequence include the production of RNA transcripts having more desirable properties, such as a greater half-life, than transcripts produced from the naturally occurring sequence.

The invention also encompasses production of the nucleic acid molecules of the invention, entirely by synthetic chemistry. Synthetic sequences may be inserted into expression vectors and cell systems that contain the necessary elements for transcriptional and translational control of the inserted coding sequence in a suitable host. These elements may include regulatory sequences, promoters, 5' and 3' untranslated regions and specific initiation signals (such as an ATG initiation codon and Kozak consensus sequence) which allow more efficient translation of sequences encoding the angiogenic genes. In cases where the complete coding sequence including its initiation codon and upstream regulatory sequences are inserted into the appropriate expression vector, additional control signals may not be needed. However, in cases where only coding sequence, or a fragment thereof, is inserted, exogenous translational control signals as described above should be provided by the vector. Such signals may be of various origins, both natural and synthetic. The efficiency of expression may be enhanced by the inclusion of enhancers appropriate for the particular host cell system used (Scharf et al., 1994).

The invention also includes nucleic acid molecules that are the complements of the sequences described herein.

The present invention allows for the preparation of purified polypeptides or proteins. In order to do this, host cells may be transfected with a nucleic acid molecule as described above. Typically, said host cells are transfected with an expression vector comprising a nucleic acid molecule according to the invention. A variety of expression vector/host systems may be utilized to contain and express the sequences. These include, but are not limited to, microorganisms such as bacteria transformed with plasmid or cosmid DNA expression vectors; yeast transformed with yeast expression vectors; insect cell systems infected with viral expression vectors (e.g., baculovirus); or mouse or other animal or human tissue cell systems. Mammalian cells can also be used to express a protein that is encoded by a specific angiogenic gene of the invention using various expression vectors including plasmid, cosmid and viral systems such as a vaccinia virus expression system. The invention is not limited by the host cell or vector employed.

The nucleic acid molecules, or variants thereof, of the present invention can be stably expressed in cell lines to allow long term production of recombinant proteins in mammalian systems. Sequences encoding any one of the angiogenic genes of the invention can be transformed into cell lines using expression vectors which may contain viral origins of replication and/or endogenous expression elements and a selectable marker gene on the same or on a separate vector. The selectable marker confers resistance to a selective agent, and its presence allows growth and recovery of cells which successfully express the introduced sequences. Resistant clones of stably transformed cells may be propagated using tissue culture techniques appropriate to the cell type.

The protein produced by a transformed cell may be secreted or retained intracellularly depending on the sequence and/or the vector used. As will be understood by those of skill in the art, expression vectors containing polynucleotides which encode a protein may be designed to contain signal sequences which direct secretion of the protein through a prokaryotic or eukaryotic cell membrane.

In addition, a host cell strain may be chosen for its ability to modulate expression of the inserted sequences or to process the expressed protein in the desired fashion. Such modifications of the polypeptide include, but are not limited to, acetylation, glycosylation, phosphorylation, and acylation. Post-translational cleavage of a "prepro" form of the protein may also be used to specify protein targeting, folding, and/or activity. Different host cells having specific cellular machinery and characteristic mechanisms for post-translational activities (e.g., CHO or HeLa cells), are available from the American Type Culture Collection (ATCC) and may be chosen to ensure the correct modification and processing of the foreign protein.

There may be provided an expression vector comprising a nucleic acid molecule of the invention as described above.

There may be provided a cell comprising a nucleic acid molecule of the invention as described above.

When large quantities of protein are needed such as for antibody production, vectors which direct high levels of expression may be used such as those containing the T5 or T7 inducible bacteriophage promoter. Expression systems as described above may be used in generating and isolating fusion proteins which contain important functional domains of the protein. These fusion proteins are used for binding, structural and functional studies as well as for the generation of appropriate antibodies.

In order to express and purify the protein as a fusion protein, the appropriate polynucleotide sequences of the present invention are inserted into a vector which contains a nucleotide sequence encoding another peptide (for example, glutathionine succinyl transferase). The fusion protein is expressed and recovered from prokaryotic or eukaryotic cells. The fusion protein can then be purified by affinity chromatography based upon the fusion vector sequence and the relevant protein can subsequently be obtained by enzymatic cleavage of the fusion protein.

Fragments of polypeptides of the present invention may also be produced by direct peptide synthesis using solid-phase techniques. Automated synthesis may be achieved by using the ABI 431A Peptide Synthesizer (Perkin-Elmer). Various fragments of polypeptide may be synthesized separately and then combined to produce the full length molecule.

Partial gene sequences can be used to obtain the corresponding sequence of the full-length angiogenic gene. Full-length angiogenic genes may be cloned using the partial nucleotide sequences by methods known *per se* to those skilled in the art. For example, *in silico* analysis of sequence databases such as those hosted at the National Centre for Biotechnology Information (http://www.ncbi.nlm.nih.gov/) can be searched in order to obtain overlapping nucleotide sequence. This provides a "walking" strategy towards obtaining the full-length gene sequence. Appropriate databases to search at this site include the expressed sequence tag (EST) database (database of GenBank, EMBL and DDBJ sequences from their EST divisions) or the non redundant (nr) database (contains all GenBank, EMBL, DDBJ and PDB sequences but does not include EST, STS, GSS, or phase 0, 1 or 2 HTGS sequences). Typically searches are performed using the BLAST algorithm described in Altschul *et al* (1997) with the BLOSUM62 default matrix. In instances where *in silico* "walking" approaches fail to retrieve the complete gene sequence, additional strategies may be employed. These include the use of "restriction-site PCR" will allows the retrieval of unknown sequence adjacent to a portion of DNA whose sequence is known. In this technique universal primers are used to retrieve unknown sequence. Inverse PCR may also be used, in which primers based on the known sequence are designed to amplify adjacent unknown sequences. These upstream sequences may include promoters and regulatory elements. In addition, various other PCR-based techniques may be used, for example a kit available from Clontech (Palo Alto, California) allows for a walking PCR technique, the 5' RACE kit (Gibco-BRL) allows isolation of additional 5' gene sequence, while additional 3' sequence can be obtained using practised techniques (for example see Gecz et al., 1997).

There may be provided an isolated polypeptide as defined by SEQ ID Numbers: 51 to 58 and laid out in Table 1.

There are also provided isolated polypeptides, which have been shown to be up-regulated in their expression during angiogenesis (see Tables 1 and 2).

More specifically, following the realisation that these polypeptides are up-regulated in their expression during angiogenesis, the isolated polypeptides as defined by SEQ ID Numbers: 51 to 58, and as laid out in Tables 1 and 2, or fragments thereof, may play a role in an angiogenic process. Such a process may include, but is not restricted to, embryogenesis, menstrual cycle, wound repair, tumour angiogenesis and exercise induced muscle hypertrophy.

In addition, the isolated polypeptides as defined by SEQ ID Numbers: 51 to 58, and as laid out in Tables 1 and 2, or fragments thereof, may play a role in diseases associated with the angiogenic process. Diseases may include, but are not restricted to, cancer, rheumatoid arthritis, diabetic retinopathy, psoriasis, and cardiovascular diseases such as atherosclerosis, ischaemic limb disease and coronary artery disease.

An isolated polypeptide having at least 70%, preferably 85%, and more preferably 95%, identity to any one of SEQ ID Numbers: 51 to 58, may play a role in an angiogenic process.

Sequence identity is typically calculated using the BLAST algorithm, described in Altschul *et al* (1997) with the BLOSUM62 default matrix.

There is further provided a method of preparing a polypeptide as described above, comprising the steps of:
(1) culturing cells as described above under conditions effective for production of the polypeptide; and
(2) harvesting the polypeptide.

There is provided a polypeptide which is the product of the process described above.

Substantially purified protein or fragments thereof can then be used in further biochemical analyses to establish secondary and tertiary structure. Such methodology is known in the art and includes, but is not restricted to, X-ray crystallography of crystals of the proteins or by nuclear magnetic resonance (NMR). Determination of structure allows for the rational design of pharmaceuticals to interact with the protein, alter protein charge configuration or charge interaction with other proteins, or to alter its function in the cell.

The invention has provided a number of genes likely to be involved in angiogenesis and therefore enables methods for the modulation of angiogenesis. As angiogenesis is critical in a number of pathological processes, the invention therefore also enables therapeutic methods for the treatment of all angiogenesis-related disorders, and may enable the diagnosis or prognosis of all angiogenesis-related disorders associated with abnormalities in expression and/or function of any one of the angiogenic genes.

Examples of such disorders include, but are not limited to, cancer, rheumatoid arthritis, diabetic retinopathy, psoriasis, and cardiovascular diseases such as atherosclerosis, ischaemic limb disease and coronary artery disease.

### Therapeutic Applications

There is provided a method of treating an angiogenesis-related disorder as described above, comprising administering a selective antagonist or agonist of an angiogenic gene or protein of the invention to a subject in need of such treatment.

There is further provided the use of a selective antagonist or agonist of an angiogenic gene or protein of the invention in the manufacture of a medicament for the treatment of an angiogenesis-related disorder as described above.

For the treatment of angiogenesis-related disorders which result in uncontrolled or enhanced angiogenesis, including but not limited to, cancer, rheumatoid arthritis, diabetic retinopathy, psoriasis and cardiovascular diseases such as atherosclerosis, therapies which inhibit the expanding vasculature are desirable. This would involve inhibition of any one of the angiogenic genes or proteins that are able to promote angiogenesis, or enhancement, stimulation or re-activation of any one of the angiogenic genes or proteins that are able to inhibit angiogenesis.

For the treatment of angiogenesis-related disorders which are characterised by inhibited or decreased angiogenesis, including but not limited to, ischaemic limb disease and coronary artery disease, therapies which enhance or promote vascular expansion are desirable. This would involve inhibition of any one of the angiogenic genes or proteins that are able to restrict angiogenesis or enhancement, stimulation or re-activation of any one of the angiogenic genes or proteins that are able to promote angiogenesis.

For instance, decreasing the expression of BNO782 and BN0481 has been shown to disrupt endothelial cell activity leading to an inhibition of capillary tube formation and angiogenesis. Therefore, in the treatment of disorders where angiogenesis needs to be restricted, it would be desirable to inhibit the function of these genes. Alternatively, in the treatment of disorders where angiogenesis needs to be stimulated it may be desirable to enhance the function of these genes.

For each of these cases, the relevant therapy will be useful in treating angiogenesis-related disorders regardless of whether there is a lesion in the angiogenic gene.

### Inhibiting gene or protein function

Inhibiting the function of a gene or protein can be achieved in a variety of ways. Antisense nucleic acid methodologies represent one approach to inactivate genes that are causative of a disorder. Antisense or gene-targeted silencing strategies may include, but are not limited to, the use of antisense oligonucleotides, injection of antisense RNA, transfection of antisense RNA expression vectors, and the use of RNA interference (RNAi) or short interfering RNAs (siRNA). RNAi can be used *in vitro* and *in vivo* to silence a gene when its expression contributes to angiogenesis (Sharp and Zamore, 2000; Grishok et al., 2001). Still further, catalytic nucleic acid molecules such as DNAzymes and ribozymes may be used for gene silencing (Breaker and Joyce, 1994; Haseloff and Gerlach, 1988). These molecules function by cleaving their target mRNA molecule rather than merely binding to it as in traditional antisense approaches.

In one aspect of the invention an isolated nucleic acid molecule, which is the complement of any one of the relevant angiogenic nucleic acid molecules described above may be administered to a subject in need of such treatment. Typically, a complement to any relevant one of the angiogenic genes is administered to a subject to treat or prevent an angiogenesis-related disorder. In a further aspect the complement may encode an RNA molecule that hybridizes with the mRNA encoded by the relevant angiogenic gene of the invention or may be a short interfering oligonucleotide (siRNA) that hybridizes with the mRNA encoded by the relevant angiogenic gene of the invention.

There is provided the use of an isolated nucleic acid molecule which is the complement of any one of the relevant nucleic acid molecules of the invention and which encodes an RNA molecule or a short interfering oligonucleotide (siRNA) that hybridizes with the mRNA encoded by the relevant angiogenic gene of the invention, in the manufacture of a medicament for the treatment of an angiogenesis-related disorder.

Typically, a vector expressing the complement of a polynucleotide encoding any one of the relevant angiogenic genes may be administered to a subject to treat or prevent an angiogenesis-related disorder including, but not limited to, those described above. Many methods for introducing vectors into cells or tissues are available and equally suitable for use *in vivo, in vitro*, and ex vivo. For *ex vivo* therapy, vectors may be introduced into stem cells taken from the patient and clonally propagated for autologous transplant back into that same patient.

Delivery by transfection, by liposome injections, or by polycationic amino polymers may be achieved using methods which are well known in the art. (For example, see Goldman *et al*., 1997).

In a further aspect purified protein according to the invention may be used to produce antibodies which specifically bind any relevant angiogenic protein of the invention. These antibodies may be used directly as an antagonist or indirectly as a targeting or delivery mechanism for bringing a pharmaceutical agent (such as a cytotoxic agent) to cells or tissues that express the relevant angiogenic protein. Such antibodies may include, but are not limited to, polyclonal, monoclonal, chimeric and single chain antibodies as would be understood by the person skilled in the art.

For the production of antibodies, various hosts including rabbits, rats, goats, mice, humans, and others may be immunized by injection with a protein of the invention or with any fragment or oligopeptide thereof, which has immunogenic properties. Various adjuvants may be used to increase immunological response and include, but are not limited to, Freund's, mineral gels such as aluminum hydroxide, and surface-active substances such as lysolecithin. Adjuvants used in humans include BCG (bacilli Calmette-Guerin) and Corynebacterium parvum.

It is preferred that the oligopeptides, peptides, or fragments used to induce antibodies to the relevant angiogenic protein have an amino acid sequence consisting of at least about 5 amino acids, and, more preferably, of at least about 10 amino acids. It is also preferable that these oligopeptides, peptides, or fragments are identical to a portion of the amino acid sequence of the natural protein and contain the entire amino acid sequence of a small, naturally occurring molecule. Short stretches of amino acids from these proteins may be fused with those of another protein, such as KLH, and antibodies to the chimeric molecule may be produced.

Monoclonal antibodies to any relevant angiogenic protein may be prepared using any technique which provides for the production of antibody molecules by continuous cell lines in culture. These include, but are not limited to, the hybridoma technique, the human B-cell hybridoma technique, and the EBV-hybridoma technique. (For example, see Kohler and Milstein, 1975; Kozbor et al., 1985; Cote et al., 1983; Cole et al., 1984).

Monoclonal antibodies produced may include, but are not limited to, mouse-derived antibodies, humanised antibodies and fully-human antibodies. For example, antibodies are obtained from transgenic mice that have been engineered to produce specific human antibodies in response to antigenic challenge. In one example of this technique, elements of the human heavy and light chain loci are introduced into strains of mice derived from embryonic stem cell lines that contain targeted disruptions of the endogenous heavy and light chain loci. These transgenic mice can synthesise human antibodies specific for human antigens and can be used to produce human antibody-secreting hybridomas. Methods for obtaining human antibodies from transgenic mice are described for example in Lonberg et al., 1994; Green et al., 1994; Taylor et al., 1994.

Antibodies may also be produced by inducing *in vivo* production in the lymphocyte population or by screening immunoglobulin libraries or panels of highly specific binding reagents as disclosed in the literature. (For example, see Orlandi et al., 1989; Winter et al., 1991).

Antibody fragments which contain specific binding sites for any relevant angiogenic protein may also be generated. For example, such fragments include, F(ab')2 fragments produced by pepsin digestion of the antibody molecule and Fab fragments generated by reducing the disulfide bridges of the F(ab')2 fragments. Alternatively, Fab expression libraries may be constructed to allow rapid and easy identification of monoclonal Fab fragments with the desired specificity. (For example, see Huse et al., 1989).

Various immunoassays may be used for screening to identify antibodies having the desired specificity. Numerous protocols for competitive binding or immunoradiometric assays using either polyclonal or monoclonal antibodies with established specificities are well known in the art. Such immunoassays typically involve the measurement of complex formation between a protein and its specific antibody. A two-site, monoclonal-based immunoassay utilizing monoclonal antibodies reactive to two non-interfering epitopes is preferred, but a competitive binding assay may also be employed.

In a further aspect, antagonists may include peptides, phosphopeptides or small organic or inorganic compounds. These antagonists should disrupt the function of any relevant angiogenic gene of the invention so as to provide the necessary therapeutic effect.

Peptides, phosphopeptides or small organic or inorganic compounds suitable for therapeutic applications may be identified using nucleic acids and polypeptides of the invention in drug screening applications as described below.

### Enhancing gene or protein function

Enhancing, stimulating or re-activating a gene's or protein's function can be achieved in a variety of ways. Administration of an isolated nucleic acid molecule, as described above, to a subject in need of such treatment may be initiated. Typically, any relevant angiogenic gene of the invention can be administered to a subject to treat or prevent an angiogenesis-related disorder.

There is further provided the use of an isolated nucleic acid molecule, as described above, in the manufacture of a medicament for the treatment of an angiogenesis-related disorder.

Typically, a vector capable of expressing any relevant angiogenic gene, or a fragment or derivative thereof, may be administered to a subject to treat or prevent a disorder including, but not limited to, those described above. Transducing retroviral vectors are often used for somatic cell gene therapy because of their high efficiency of infection and stable integration and expression. Any relevant full-length gene, or portions thereof, can be cloned into a retroviral vector and expression may be driven from its endogenous promoter or from the retroviral long terminal repeat or from a promoter specific for the target cell type of interest. Other viral vectors can be used and include, as is known in the art, adenoviruses, adeno-associated viruses, vaccinia viruses, papovaviruses, lentiviruses and retroviruses of avian, murine and human origin.

Gene therapy would be carried out according to established methods (Friedman, 1991; Culver, 1996). A vector containing a copy of any relevant angiogenic gene linked to expression control elements and capable of replicating inside the cells is prepared. Alternatively the vector may be replication deficient and may require helper cells for replication and use in gene therapy.

Gene transfer using non-viral methods of infection *in vitro* can also be used. These methods include direct injection of DNA, uptake of naked DNA in the presence of calcium phosphate, electroporation, protoplast fusion or liposome delivery. Gene transfer can also be achieved by delivery as a part of a human artificial chromosome or receptor-mediated gene transfer. This involves linking the DNA to a targeting molecule that will bind to specific cell-surface receptors to induce endocytosis and transfer of the DNA into mammalian cells. One such technique uses poly-L-lysine to link asialoglycoprotein to DNA. An adenovirus is also added to the complex to disrupt the lysosomes and thus allow the DNA to avoid degradation and move to the nucleus. Infusion of these particles intravenously has resulted in gene transfer into hepatocytes.

Although not identified to date, it is possible that certain individuals with angiogenesis-related disorders contain an abnormality in any one of the angiogenic genes of the invention. In affected subjects that express a mutated form of any one of the angiogenic genes of the invention it may be possible to prevent the disorder by introducing into the affected cells a wild-type copy of the gene such that it recombines with the mutant gene. This requires a double recombination event for the correction of the gene mutation. Vectors for the introduction of genes in these ways are known in the art, and any suitable vector may be used. Alternatively, introducing another copy of the gene bearing a second mutation in that gene may be employed so as to negate the original gene mutation and block any negative effect.

There is provided a method of treating an angiogenesis-related disorder comprising administering a polypeptide, as described above, or an agonist thereof, to a subject in need of such treatment.

There is further provided the use of a polypeptide as described above, or an agonist thereof, in the manufacture of a medicament for the treatment of an angiogenesis-related disorder. Examples of such disorders are described above.

A suitable agonist may also include peptides, phosphopeptides or small organic or inorganic compounds that can mimic the function of any relevant angiogenic gene, or may include an antibody to any relevant angiogenic gene that is able to restore function to a normal level.

Peptides, phosphopeptides or small organic or inorganic compounds suitable for therapeutic applications may be identified using nucleic acids and polypeptides of the invention in drug screening applications as described below.

In further embodiments, any of the agonists, antagonists, complementary sequences, nucleic acid molecules, proteins, antibodies, or vectors of the invention may be administered in combination with other appropriate therapeutic agents. Selection of the appropriate agents may be made by those skilled in the art, according to conventional pharmaceutical principles. The combination of therapeutic agents may act synergistically to effect the treatment or prevention of the various disorders described above. Using this approach, therapeutic efficacy with lower dosages of each agent may be possible, thus reducing the potential for adverse side effects.

Any of the therapeutic methods described above may be applied to any subject in need of such therapy, including, for example, mammals such as dogs, cats, cows, horses, rabbits, monkeys, and most preferably, humans.

### Modulation of angiogenesis

Since a number of genes likely to be involved in angiogenesis have been identified this therefore enables methods for the modulation of angiogenesis. In a further aspect of the present invention, any of the methods described above used for the treatment of an angiogenesis-related disorder may be used for the modulation of angiogenesis in any system comprising cells. These systems may include but are not limited to, *in vitro* assay systems (e.g. Matrigel assays, proliferation assays, migration assays, collagen assays, bovine capillary endothelial cell assay etc), *in vivo* assay systems (e.g. *in vivo* Matrigel-type assays, chicken chorioallantoic membrane assay, isolated organs, tissues or cells etc), animal models (e.g. *in vivo* neovascularisation assays, tumour angiogenesis models etc) or hosts in need of treatment (e.g. hosts suffering from angiogenesis-related disorders as previously described.

### Drug screening

According to still another aspect of the invention, nucleic acid molecules of the invention as well as peptides of the invention, particularly any relevant purified angiogenic polypeptides or fragments thereof, and cells expressing these are useful for screening of candidate pharmaceutical compounds in a variety of techniques for the treatment of angiogenesis-related disorders.

Still further, it provides the use wherein high throughput screening techniques are employed.

Compounds that can be screened in accordance with the invention include, but are not limited to peptides (such as soluble peptides), phosphopeptides and small organic or inorganic molecules (such as natural product or synthetic chemical libraries and peptidomimetics).

In one embodiment, a screening assay may include a cell-based assay utilising eukaryotic or prokaryotic host cells that are stably transformed with recombinant nucleic acid molecules expressing the relevant angiogenic polypeptide or fragment, in competitive binding assays. Binding assays will measure for the formation of complexes between the relevant polypeptide or fragments thereof and the compound being tested, or will measure the degree to which a compound being tested will interfere with the formation of a complex between the relevant polypeptide or fragment thereof, and its interactor or ligand.

Non cell-based assays may also be used for identifying compounds that interrupt binding between the polypeptides of the invention and their interactors. Such assays are known in the art and include for example AlphaScreen technology (PerkinElmer Life Sciences, MA, USA). This application relies on the use of beads such that each interaction partner is bound to a separate bead via an antibody. Interaction of each partner will bring the beads into proximity, such that laser excitation initiates a number of chemical reactions ultimately leading to fluorophores emitting a light signal. Candidate compounds that disrupt the binding of the relevant angiogenic polypeptide with its interactor will result in loss of light emission enabling identification and isolation of the responsible compound.

High-throughput drug screening techniques may also employ methods as described in WO84/03564. Small peptide test compounds synthesised on a solid substrate can be assayed through relevant angiogenic polypeptide binding and washing. The relevant bound angiogenic polypeptide is then detected by methods well known in the art. In a variation of this technique, purified angiogenic polypeptides can be coated directly onto plates to identify interacting test compounds.

An additional method for drug screening involves the use of host eukaryotic cell lines that carry mutations in any relevant angiogenic gene of the invention. The host cell lines are also defective at the polypeptide level. Other cell lines may be used where the expression of the relevant angiogenic gene can be regulated (i.e. over-expressed, under-expressed, or switched off). The host cell lines or cells are grown in the presence of various drug compounds and the rate of growth of the host cells is measured to determine if the compound is capable of regulating the growth of defective cells.

The angiogenic polypeptides of the present invention may also be used for screening compounds developed as a result of combinatorial library technology. This provides a way to test a large number of different substances for their ability to modulate activity of a polypeptide. A substance identified as a modulator of polypeptide function may be peptide or non-peptide in nature. Non-peptide "small molecules" are often preferred for many *in vivo* pharmaceutical applications. In addition, a mimic or mimetic of the substance may be designed for pharmaceutical use. The design of mimetics based on a known pharmaceutically active compound ("lead" compound) is a common approach to the development of novel pharmaceuticals. This is often desirable where the original active compound is difficult or expensive to synthesise or where it provides an unsuitable method of administration. In the design of a mimetic, particular parts of the original active compound that are important in determining the target property are identified. These parts or residues constituting the active region of the compound are known as its pharmacophore. Once found, the pharmacophore structure is modelled according to its physical properties using data from a range of sources including x-ray diffraction data and NMR. A template molecule is then selected onto which chemical groups that mimic the pharmacophore can be added. The selection can be made such that the mimetic is easy to synthesise, is likely to be pharmacologically acceptable, does not degrade *in vivo* and retains the biological activity of the lead compound. Further optimisation or modification can be carried out to select one or more final mimetics useful for *in vivo* or clinical testing.

It is also possible to isolate a target-specific antibody and then solve its crystal structure. In principle, this approach yields a pharmacophore upon which subsequent drug design can be based as described above. It may be possible to avoid protein crystallography altogether by generating anti-idiotypic antibodies (anti-ids) to a functional, pharmacologically active antibody. As a mirror image of a mirror image, the binding site of the anti-ids would be expected to be an analogue of the original binding site. The anti-id could then be used to isolate peptides from chemically or biologically produced peptide banks.

Another alternative method for drug screening relies on structure-based rational drug design. Determination of the three dimensional structure of the polypeptides of the invention, or the three dimensional structure of the protein complexes which may incorporate these polypeptides allows for structure-based drug design to identify biologically active lead compounds.

Three dimensional structural models can be generated by a number of applications, some of which include experimental models such as x-ray crystallography and NMR and/or from *in silico* studies using information from structural databases such as the Protein Databank (PDB). In addition, three dimensional structural models can be determined using a number of known protein structure prediction techniques based on the primary sequences of the polypeptides (e.g. SYBYL - Tripos Associated, St. Louis, MO), de novo protein structure design programs (e.g. MODELER - MSI Inc., San Diego, CA, or MOE - Chemical Computing Group, Montreal, Canada) or *ab initio* methods (e.g. see US Patent Numbers 5331573 and 5579250).

Once the three dimensional structure of a polypeptide or polypeptide complex has been determined, structure-based drug discovery techniques can be employed to design biologically active compounds based on these three dimensional structures. Such techniques are known in the art and include examples such as DOCK (University of California, San Francisco) or AUTODOCK (Scripps Research Institute, La Jolla, California). A computational docking protocol will identify the active site or sites that are deemed important for protein activity based on a predicted protein model. Molecular databases, such as the Available Chemicals Directory (ACD) are then screened for molecules that complement the protein model.

Using methods such as these, potential clinical drug candidates can be identified and computationally ranked in order to reduce the time and expense associated with typical 'wet lab' drug screening methodologies.

Compounds identified from the screening methods described above form a part of the present invention, as do pharmaceutical compositions containing these and a pharmaceutically acceptable carrier.

### Pharmaceutical Preparations

Compounds identified from screening assays as indicated above can be administered to a patient at a therapeutically effective dose to treat or ameliorate a disorder associated with angiogenesis. A therapeutically effective dose refers to that amount of the compound sufficient to result in amelioration of symptoms of the disorder.

Toxicity and therapeutic efficacy of such compounds can be determined by standard pharmaceutical procedures in cell cultures or experimental animals. The data obtained from these studies can then be used in the formulation of a range of dosages for use in humans.

Pharmaceutical compositions can be formulated in a conventional manner using one or more physiological acceptable carriers, excipients or stabilisers which are well known. Acceptable carriers, excipients or stabilizers are non-toxic at the dosages and concentrations employed, and include buffers such as phosphate, citrate, and other organic acids; antioxidants including absorbic acid; low molecular weight (less than about 10 residues) polypeptides; proteins, such as serum albumin, gelatin, or immunoglobulins; binding agents including hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or non-ionic surfactants such as Tween, Pluronics or polyethylene glycol (PEG).

The formulation of pharmaceutical compositions will be based on the proposed route of administration. Routes of administration may include, but are not limited to, inhalation, insufflation (either through the mouth or nose), oral, buccal, rectal or parental administration.

### Diagnostic and prognostic applications

Should abnormalities in any one of the angiogenic genes of the invention exist, which alter activity and/or expression of the gene to give rise to angiogenesis-related disorders, the polynucleotides and polypeptides of the invention may be used for the diagnosis or prognosis of these disorders, or a predisposition to such disorders. Examples of such disorders include, but are not limited to, cancer, rheumatoid arthritis, diabetic retinopathy, psoriasis, cardiovascular diseases such as atherosclerosis, ischaemic limb disease and coronary artery disease. Diagnosis or prognosis may be used to determine the severity, type or stage of the disease state in order to initiate an appropriate therapeutic intervention.

In another embodiment of the invention, the polynucleotides that may be used for diagnostic or prognostic purposes include oligonucleotide sequences, genomic DNA and complementary RNA and DNA molecules. The polynucleotides may be used to detect and quantitate gene expression in biopsied tissues in which abnormal expression or mutations in any one of the angiogenic genes may be correlated with disease. Genomic DNA used for the diagnosis or prognosis may be obtained from body cells, such as those present in the blood, tissue biopsy, surgical specimen, or autopsy material. The DNA may be isolated and used directly for detection of a specific sequence or may be amplified by the polymerase chain reaction (PCR) prior to analysis. Similarly, RNA or cDNA may also be used, with or without PCR amplification. To detect a specific nucleic acid sequence, direct nucleotide sequencing, reverse transcriptase PCR (RT-PCR), hybridization using specific oligonucleotides, restriction enzyme digest and mapping, PCR mapping, RNAse protection, and various other methods may be employed. Oligonucleotides specific to particular sequences can be chemically synthesized and labelled radioactively or nonradioactively and hybridized to individual samples immobilized on membranes or other solid-supports or in solution. The presence, absence or excess expression of any one of the angiogenic genes may then be visualized using methods such as autoradiography, fluorometry, or colorimetry.

In a particular aspect, the nucleotide sequences of the invention may be useful in assays that detect the presence of associated disorders, particularly those mentioned previously. The nucleotide sequences may be labelled by standard methods and added to a fluid or tissue sample from a patient under conditions suitable for the formation of hybridization complexes. After a suitable incubation period, the sample is washed and the signal is quantitated and compared with a standard value. If the amount of signal in the patient sample is significantly altered in comparison to a control sample then the presence of altered levels of nucleotide sequences in the sample indicates the presence of the associated disorder. Such assays may also be used to evaluate the efficacy of a particular therapeutic treatment regimen in animal studies, in clinical trials, or to monitor the treatment of an individual patient.

In order to provide a basis for the diagnosis or prognosis of an angiogenesis-related disorder associated with a mutation in any one of the angiogenic genes of the invention, the nucleotide sequence of the relevant gene can be compared between normal tissue and diseased tissue in order to establish whether the patient expresses a mutant gene.

In order to provide a basis for the diagnosis or prognosis of a disorder associated with abnormal expression of any one of the angiogenic genes of the invention, a normal or standard profile for expression is established. This may be accomplished by combining body fluids or cell extracts taken from normal subjects, either animal or human, with a sequence, or a fragment thereof, encoding the relevant angiogenic gene, under conditions suitable for hybridization or amplification. Standard hybridization may be quantified by comparing the values obtained from normal subjects with values from an experiment in which a known amount of a substantially purified polynucleotide is used. Another method to identify a normal or standard profile for expression of any one of the angiogenic genes is through quantitative RT-PCR studies. RNA isolated from body cells of a normal individual, particularly RNA isolated from endothelial cells, is reverse transcribed and real-time PCR using oligonucleotides specific for the relevant gene is conducted to establish a normal level of expression of the gene. Standard values obtained in both these examples may be compared with values obtained from samples from patients who are symptomatic for a disorder. Deviation from standard values is used to establish the presence of a disorder.

Once the presence of a disorder is established and a treatment protocol is initiated, hybridization assays or quantitative RT-PCR studies may be repeated on a regular basis to determine if the level of expression in the patient begins to approximate that which is observed in the normal subject. The results obtained from successive assays may be used to show the efficacy of treatment over a period ranging from several days to months.

There is provided the use of an angiogenic polypeptide as described above in the diagnosis or prognosis of an angiogenesis-related disorder associated with any one of angiogenic genes of the invention, or a predisposition to such disorders.

When a diagnostic or prognostic assay is to be based upon any relevant angiogenic polypeptide, a variety of approaches are possible. For example, diagnosis or prognosis can be achieved by monitoring differences in the electrophoretic mobility of normal and mutant proteins. Such an approach will be particularly useful in identifying mutants in which charge substitutions are present, or in which insertions, deletions or substitutions have resulted in a significant change in the electrophoretic migration of the resultant protein. Alternatively, diagnosis or prognosis may be based upon differences in the proteolytic cleavage patterns of normal and mutant proteins, differences in molar ratios of the various amino acid residues, or by functional assays demonstrating altered function of the gene products.

In another aspect, antibodies that specifically bind the relevant angiogenic gene product may be used for the diagnosis or prognosis of disorders characterized by abnormal expression of the gene, or in assays to monitor patients being treated with the relevant angiogenic gene or protein or agonists, antagonists, or inhibitors thereof. Antibodies useful for diagnostic or prognostic purposes may be prepared in the same manner as described above for therapeutics. Diagnostic or prognostic assays may include methods that utilize the antibody and a label to detect the relevant protein in human body fluids or in extracts of cells or tissues. The antibodies may be used with or without modification, and may be labelled by covalent or non-covalent attachment of a reporter molecule.

A variety of assays for measuring the relevant angiogenic polypeptide based on the use of antibodies specific for the polypeptide are known in the art and provide a basis for diagnosing altered or abnormal levels of expression. Normal or standard values for expression are established by combining body fluids or cell extracts taken from normal mammalian subjects, preferably human, with antibody to the relevant protein under conditions suitable for complex formation. The amount of standard complex formation may be quantitated by various methods which are known in the art. Examples include, but are not limited to, enzyme-linked immunosorbent assays (ELISAs), radioimmunoassays (RIAs), immunofluorescence, flow cytometry, histology, electron microscopy, in situ assays, immunoprecipitation, Western blot etc. For example, using the ELISA technique an enzyme which is bound to the antibody will react with an appropriate substrate, preferably a chromogenic substrate, in such a manner as to produce a chemical moiety that can be detected for example by spectrophotomeric, fluorimetric or by visual means. Detection may also be accomplished by using other assays such as RIAs where the antibodies or antibody fragments are radioactively labelled. It is also possible to label the antibody with a fluorescent compound. When the fluorescently labelled antibody is exposed to light of a certain wavelength, its presence can then be detected due to fluorescence. The antibody can also be detectably labelled by coupling it to a chemiluminescent compound. The presence of the chemiluminescent-tagged antibody is then determined by detecting the presence of luminescence that arises during the course of a chemical reaction.

Quantities of protein expressed in subject, control, and disease samples from biopsied tissues are compared with the standard values. Deviation between standard and subject values establishes the parameters for diagnosing or prognosing disease.

Once an individual has been diagnosed or prognosed with a disorder, effective treatments can be initiated, as described above. In the treatment of angiogenesis-related diseases which are characterised by uncontrolled or enhanced angiogenesis, the expanding vasculature needs to be inhibited. This would involve inhibiting the relevant angiogenic genes or proteins of the invention that promote angiogenesis. In addition, treatment may also need to stimulate expression or function of the relevant angiogenic genes or proteins of the invention whose normal role is to inhibit angiogenesis but whose activity is reduced or absent in the affected individual.

In the treatment of angiogenesis-related diseases which are characterised by inhibited or decreased angiogenesis, approaches which enhance or promote vascular expansion are desirable. This may be achieved using methods essentially as described above but will involve stimulating the expression or function of the relevant angiogenic gene or protein whose normal role is to promote angiogenesis but whose activity is reduced or absent in the affected individual. Alternatively, inhibiting genes or proteins that restrict angiogenesis may also be an approach to treatment.

### Microarray

In further embodiments, complete cDNAs, oligonucleotides or longer fragments derived from any of the polynucleotide sequences described herein may be used as probes in a microarray. The microarray can be used to monitor the expression level of large numbers of genes simultaneously and to identify genetic variants, mutations, and polymorphisms. This information may be used to determine gene function, to understand the genetic basis of angiogenesis-related disorders, to diagnose or prognose angiogenesis-related disorders, and to develop and monitor the activities of therapeutic agents. Microarrays may be prepared, used, and analysed using methods known in the art. (For example, see Schena et al., 1996; Heller et al., 1997).

### Transformed hosts

The production of genetically modified (knock-out, knock-in and transgenic), non-human animal models comprising the nucleic acid molecules of the invention may also be effected. These animals are useful for the study of the function of the relevant angiogenic gene, to study the process of angiogenesis, to study the mechanisms of angiogenic disease as related to these genes, for the screening of candidate pharmaceutical compounds for the treatment of angiogenesis-related disorders, for the creation of explanted mammalian cell cultures which express the protein or mutant protein, and for the evaluation of potential therapeutic interventions.

Animal species which are suitable for use in the animal models of the present invention include, but are not limited to, rats, mice, hamsters, guinea pigs, rabbits, dogs, cats, goats, sheep, pigs, and non-human primates such as monkeys and chimpanzees. For initial studies, genetically modified mice and rats are highly desirable due to the relative ease in generating knock-in, knock-out or transgenics of these animals, their ease of maintenance and their shorter life spans. For certain studies, transgenic yeast or invertebrates may be suitable and preferred because they allow for rapid screening and provide for much easier handling. For longer term studies, non-human primates may be desired due to their similarity with humans.

To create an animal model based on any one of the angiogenic genes of the invention, several methods can be employed. These include, but are not limited to, generation of a specific mutation in a homologous animal gene, insertion of a wild type human gene and/or a humanized animal gene by homologous recombination, insertion of a mutant (single or multiple) human gene as genomic or minigene cDNA constructs using wild type, mutant or artificial promoter elements, or insertion of artificially modified fragments of the endogenous gene by homologous recombination. The modifications include insertion of mutant stop codons, the deletion of DNA sequences, or the inclusion of recombination elements (lox p sites) recognized by enzymes such as Cre recombinase.

To create transgenic mice in order to study gain of gene function *in vivo,* any relevant angiogenic gene can be inserted into a mouse germ line using standard techniques such as oocyte microinjection. Gain of gene function can mean the overexpression of a gene and its protein product, or the genetic complementation of a mutation of the gene under investigation. For oocyte injection, one or more copies of the wild type or mutant gene can be inserted into the pronucleus of a just-fertilized mouse oocyte. This oocyte is then reimplanted into a pseudo-pregnant foster mother. The liveborn mice can then be screened for integrants using analysis of tail DNA for the presence of the relevant human angiogenic gene sequence. The transgene can be either a complete genomic sequence injected as a YAC, BAC, PAC or other chromosome DNA fragment, a cDNA with either the natural promoter or a heterologous promoter, or a minigene containing all of the coding region and other elements found to be necessary for optimum expression.

To generate knock-out mice or knock-in mice, gene targeting through homologous recombination in mouse embryonic stem (ES) cells may be applied. Knock-out mice are generated to study loss of gene function *in vivo* while knock-in mice allow the study of gain of function or to study the effect of specific gene mutations. Knock-in mice are similar to transgenic mice however the integration site and copy number are defined in the former.

For knock-out mouse generation, gene targeting vectors can be designed such that they disrupt (knock-out) the protein coding sequence of the relevant angiogenic gene in the mouse genome. Knock-out animals of the invention will comprise a functional disruption of a relevant angiogenesis gene of the invention such that the gene does not express a biologically active product. It can be substantially deficient in at least one functional activity coded for by the gene. Expression of the polypeptide encoded by the gene can be substantially absent (i.e. essentially undetectable amounts are made) or may be deficient in activity such as where only a portion of the gene product is produced. In contrast, knock-in mice can be produced whereby a gene targeting vector containing the relevant angiogenic gene can integrate into a defined genetic locus in the mouse genome. For both applications, homologous recombination is catalysed by specific DNA repair enzymes that recognise homologous DNA sequences and exchange them via double crossover.

Gene targeting vectors are usually introduced into ES cells using electroporation. ES cell integrants are then isolated via an antibiotic resistance gene present on the targeting vector and are subsequently genotyped to identify those ES cell clones in which the gene under investigation has integrated into the locus of interest. The appropriate ES cells are then transmitted through the germline to produce a novel mouse strain.

In instances where gene ablation results in early embryonic lethality, conditional gene targeting may be employed. This allows genes to be deleted in a temporally and spatially controlled fashion. As above, appropriate ES cells are transmitted through the germline to produce a novel mouse strain, however the actual deletion of the gene is performed in the adult mouse in a tissue specific or time controlled manner. Conditional gene targeting is most commonly achieved by use of the cre/lox system. The enzyme cre is able to recognise the 34 base pair loxP sequence such that loxP flanked (or floxed) DNA is recognised and excised by cre. Tissue specific cre expression in transgenic mice enables the generation of tissue specific knock-out mice by mating gene targeted floxed mice with cre transgenic mice. Knock-out can be conducted in every tissue (Schwenk et al., 1995) using the 'deleter' mouse or using transgenic mice with an inducible cre gene (such as those with tetracycline inducible cre genes), or knock-out can be tissue specific for example through the use of the CD19-cre mouse (Rickert et al., 1997).

There is provided the use of genetically modified non-human animals for the screening of candidate pharmaceutical compounds.

It will be clearly understood that, although a number of prior art publications are referred to herein, this reference does not constitute an admission that any of these documents forms part of the common general knowledge in the art, in Australia or in any other country. Throughout this specification and the claims, the words "comprise", "comprises" and "comprising" are used in a non-exclusive sense, except where the context requires otherwise.

### Brief Description of the Drawings

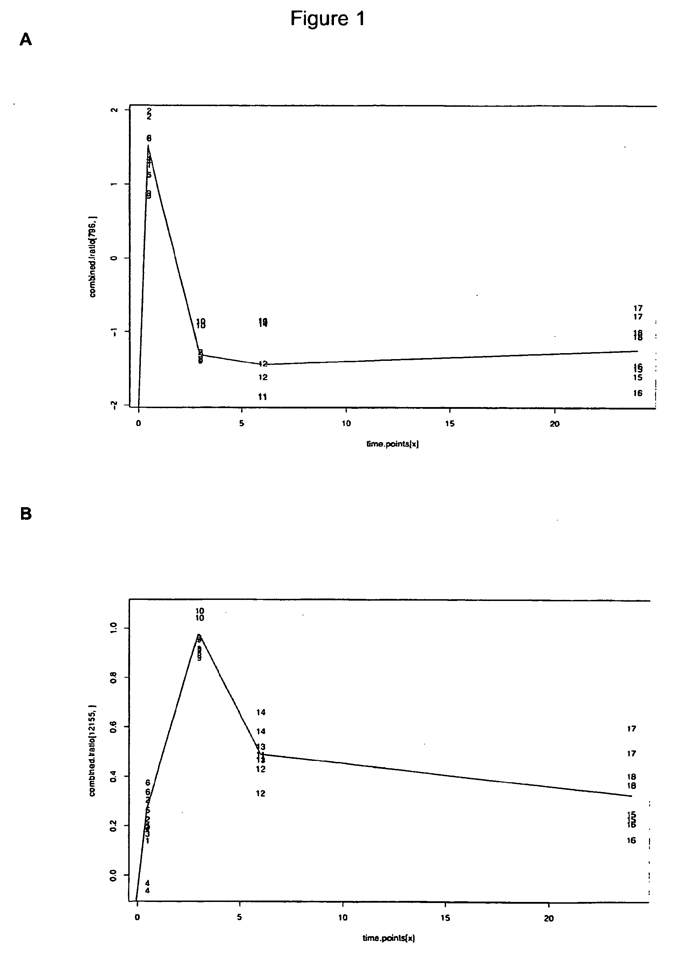

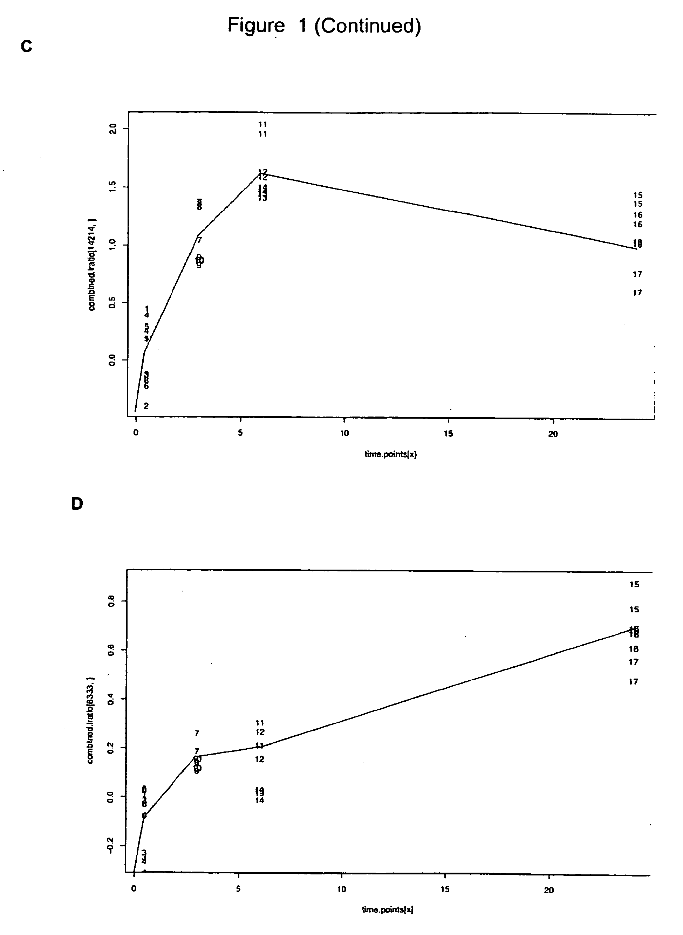

Figure 1. Example of the expression profile of selected differentially expressed clones during defined time points in the *in vitro* model of angiogenesis. Time points at the defined stages of 0.5 hours, 3 hours, 6 hours and 24 hours of the *in vitro* tube formation assay were plotted against the log ratio of cy5 (red) and cy3 (green) dyes used for microarray hybridizations. A: example of a clone with peak expression at the 0.5 hour time point; B: example of a clone with peak expression at the 3 hour time point; C: example of a clone with peak expression at the 6 hour time point; and D: example of a clone with peak expression at the 24 hour time point.

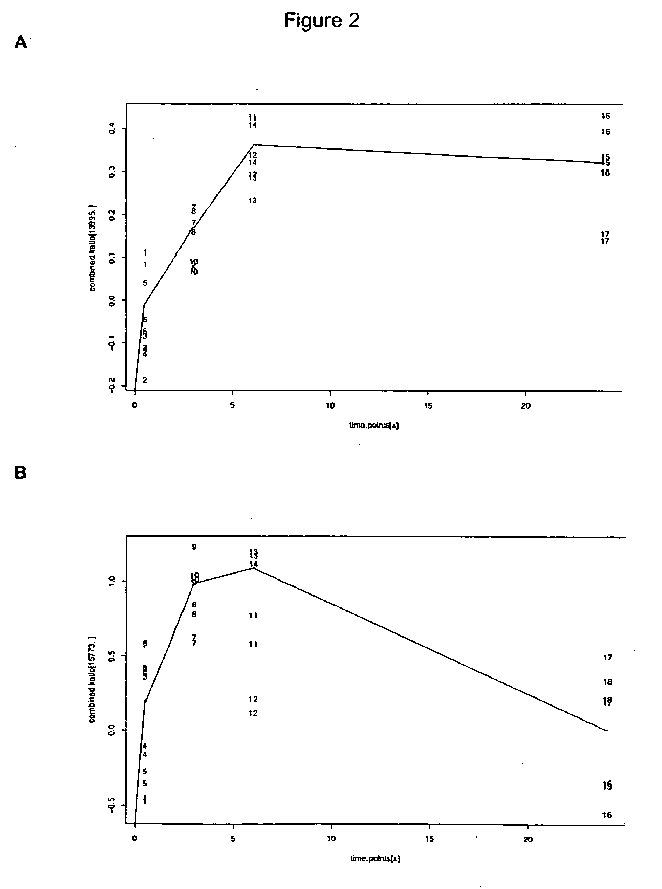

Figure 2. Expression profile of differentially expressed genes BN0782 and BNO481. Both genes show peak expression at the 6 hour time point of the *in vitro* tube formation assay. A: BNO782; B: BNO481.

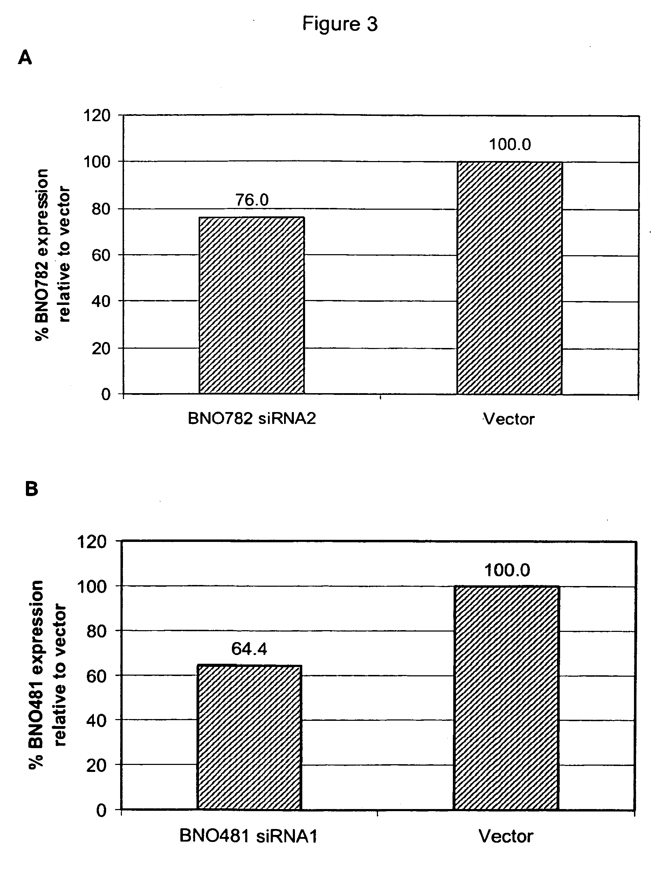

Figure 3. Analysis of the level of BNO782 expression knock-down mediated by BN0782 siRNA2 and BNO481 expression knock-down mediated by BNO481 siRNA1, as measured by real-time RT-PCR. The three siRNA oligonucleotides targeted to each gene were able to reduce expression of the gene to varying degrees with BNO781 siRNA2 inhibiting BNO781 expression by 24% (A) and BNO481 siRNA1 inhibiting expression of BN0481 by 36% (B).

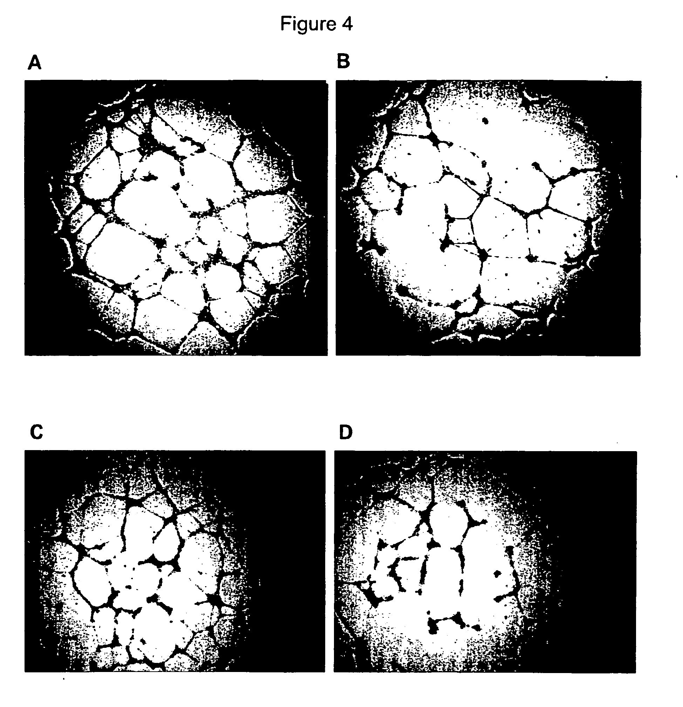

Figure 4. Reducing BN0782 or BN0481 mRNA expression inhibits HUVEC tube formation. HUVECs infected with BN0782 siRNA2, BNO481 siRNA1, or a vector control were plated on Matrigel for 24hrs. Vector infected cells formed extensive networks of tube structures (A and C). In contrast, cells infected with BNO782 siRNA2 or BNO481 siRNA1 exhibited tube structure networks of significantly reduced complexity with a high number of incomplete tube extensions (B and D).

### Modes for Performing the Invention

### Example 1: In vitro capillary tube formation

The *in vitro* model of angiogenesis is essentially as described in Gamble et al (1993). The assay was performed in collagen under the stimulation of phorbol myristate acetate (PMA) and the anti-integrin (α₂β₁) antibody, RMACII. Human umbilical vein endothelial cells (HUVECs) were used in all experiments between passages 2 to 4.

Cells were harvested from bulk cultures (t=0), replated onto the collagen gels with stimulation and then harvested from the collagen gels at 0.5, 3.0, 6.0 and 24 hours after commencement of the assay. These time points: were chosen since major morphological changes occur at these stages. Briefly, by 0.5 hours, cells have attached to the collagen matrix and have commenced migration into the gel. By 3.0 hours, small intracellular vesicles are visible. By 6.0 hours, these vesicles are coalescing together to form membrane bound vacuoles and the cells in the form of short sprouts have invaded the gel. After this time, these vacuoles fuse with the plasma membrane, thus expanding the intercellular space to generate the lumen (Meyer et al., 1997). The formation of these larger vacuoles is an essential requirement of lumen formation (Gamble et al., 1999). By 24 hours, the overall anastomosing network of capillary tubes has formed and has commenced degeneration.

### Example 2: RNA isolation, cDNA synthesis and amplification

Cells harvested at the specified time points were used for the isolation of total RNA using the Trizol reagent (Gibco BRL) according to manufacturers conditions. SMART (Switching mechanism at 5' end of RNA transcript) technology was used to convert small amounts of total RNA into enough cDNA to enable cDNA subtraction to be performed (see below). This was achieved using the SMART-PCR cDNA synthesis kit (Clontech-user manual PT3041-1) according to manufacturers recommendations. The SMART-PCR cDNA synthesis protocol generated a majority of full length cDNAs which were subsequently PCR amplified for cDNA subtraction.

### Example 3: Suppression subtractive hybridization (SSH)

SSH was performed on SMART amplified cDNA in order to enrich for cDNAs that were either up-regulated or downregulated between the cDNA populations defined by the selected time-points. This technique also allowed "normalization" of the regulated cDNAs, thereby making low abundance cDNAs (i.e. poorly expressed, but important, genes) more easily detectable. To do this, the PCR-Select cDNA synthesis kit (Clontech-user manual PT3041-1) and PCR-Select cDNA subtraction kit (Clontech-user manual PT1117-1) were used based on manufacturers conditions These procedures relied on subtractive hybridization and suppression PCR amplification. SSH was performed between the following populations: 0 - 0.5 hours; 0.5 - 3.0 hours; 3.0 - 6.0 hours; 6.0 - 24 hours.

### Example 4: Differential screening of cDNA clones

Following SSH, the cDNA fragments were digested with *Eag*I and cloned into the compatible unique *Not*I site in pBluescript KS⁺ using standard techniques (Sambrook et al., 1989). This generated forward and reverse subtracted libraries for each time period. Initially, the forward subtracted libraries were used in subsequent studies to identify those clones representing genes that were up-regulated in their expression during the *in vitro* model of angiogenesis. To do this, a microarray analysis procedure was adopted.

### Microarray slide preparation

A total of 10,000 clones from the 4 forward subtracted libraries (3,200 clones from 0-0.5 hr; 3,000 clones from 0.5-3 hr; 2,800 clones from 3-6 hr; 1,000 clones from 6-24 hr) were chosen to construct microarray slides. Inserts from these clones were amplified using standard PCR techniques with flanking T3 and T7 pBluescript KS⁺ vector primers. DNA from each clone was spotted in duplicate onto a single microarray slide. Appropriate positive and negative controls were also incorporated onto the plate.

### Probe labelling

Human umbilical vein endothelial cells harvested at the specified time points (0, 0.5, 3, 6, and 24 hr) were used for the isolation of total RNA using the Trizol reagent (Gibco BRL) according to manufacturers conditions. From each time point, 0.5 ug of total RNA was used as a template for the amplification of antisense RNA (aRNA) using the Ambion MessageAmp^{™} aRNA Kit. Briefly, total RNA was reversed transcribed with a T7 oligo(dT) primer in order to synthesize cDNA containing a T7 promoter sequence extending from the poly(A) tails of messages generated by reverse transcription. The cDNA was converted to a doublestranded DNA template and used for *in vitro* transcription of aRNA, incorporating 5-(3-aminoallyl)-UTP so as to allow coupling of fluorescent CyDyes. A typical amplification reaction would yield approximately 10 ug of mRNA (>400X amplification, assuming the initial total RNA contained <5% mRNA).

### Microarray hybridization

After coupling of CyDyes, the synthesized aRNA was used as a probe (3.0-3.5 ug) for hybridization to a microarray slide. The hybrizations performed were as follows:
1. 0 vs 0.5h (6 slides, 3 Dye swaps)
2. 0 vs 3h (4 slides, 2 Dye swaps)
3. 0 vs 6h (4 slides, 2 Dye swaps)
4. 0 vs 24h (4 slides, 2 Dye swaps)

Multiple slides were hybridized for each time point in order to verify the result from any one hybridization. Slides were hybridized in chambers for 16 hours, washed, and then scanned using the GenePix 2000 scanner. Those clones that were shown to be highly up-regulated were chosen for further analysis.

In summary, SSH was used in combination with microarray analyses to identify genes that are up-regulated and may be involved in biological processes underlying endothelial cell activation and blood vessel formation. This approach is novel in that it involves nucleotide hybridization steps that aim to reduce gene detection redundancy and enhance the chances of detecting genes that are of low overall representation in the endothelial cell transcriptome. The nucleotide-based sequential time-points aims to detect the timepoint at which the up-regulation of a particular gene takes place in a way that reduces redundancy of detection. For example, a gene that is up-regulated at 3hrs, and its expression remains up-regulated in subsequent time-points, will only be detected in the 0.5-3hr subtraction step. In contrast, if subtractions were done with the Ohr timepoint for all subsequent timepoints then this example gene would be detected at all subtraction steps following the 3hrs timepoint subtraction. This would introduce redundancy that could result in masking the possible detection of other genes of lower representation in the endothelial cell mRNA expression pool. The subsequent use of microarray analysis is based on the comparison subtraction hybridization in the SSH step involving each timepoint with the 0hrs timepoint. This enables the expression profiling of each gene across all timepoints in relation to 0hrs, irrespective of the timepoint at which it is up-regulated.

### Example 5: Clone selection

From analysis of the microarray hybridizations, a total of 1,963 clones were identified to be up-regulated in their expression at specified time points during the *in vitro* model of angiogenesis. Figure 1 provides an example of the expression profiles observed during defined time points in the *in vitro* model for a selection of clones. Each of the 1,963 clones were sequenced and subsequent in silico database analysis was used to remove clones containing vector sequences only and clones for which poor sequence was obtained. Following this, redundancy screens were used to group clones according to individual genes that they represented. This left a total of 523 genes that were found to be up-regulated in their expression during the process of angiogenesis.

Tables 1, 2 and 3 provide information on the up-regulated clones that were sequenced. Table 1 includes those clones which represent previously uncharacterised or novel genes, while Table 2 includes clones that correspond to previously identified genes which have not before been associated with angiogenesis. Also identified were a number of genes that have previously been shown to be involved in the process of angiogenesis (Table 3). The identification of these clones provides a validation or proof of principle of the effectiveness of the angiogenic gene identification strategy employed and suggests that the clones listed in Tables 1 and 2 are additional angiogenic gene candidates.

### Example 6: Analysis of the angiogenic genes

Further evidence for the involvement of the genes in Tables 1 and 2 in angiogenesis can be obtained through the functional analysis of each gene, for example by examining the effect that knock-down of their expression has on endothelial cell (EC) function and capillary tube formation.

A number of knock-down technologies and assays may be used. For example full-length coding sequences of the genes can be cloned into suitable expression vectors such as retroviruses or adenoviruses in both sense and antisense orientations and used for infection into ECs. Retrovirus infection gives long-term EC lines expressing the gene of interest whereas adenovirus infection gives transient gene expression. Infected cells can then be subjected to a number of EC assays including proliferation and capillary tube formation to confirm the role of each gene in angiogenesis.

In this study RNA interference (RNAi) gene knock-down technology was used for the analysis of gene function (see detailed description below). In this technique, short gene-specific RNA oligonucleotides are delivered to ECs in culture mediated by retroviral infection. These oligonucleotides bind to the gene transcript under study and induce its degradation resulting in silencing or reduction of gene expression. The consequences of this alteration to gene expression can be subsequently studied using assays that examine the ability of ECs to proliferate, migrate and form capillaries *in vitro*. The RNAi procedure adopted in this study is described below in detail and documents the analysis of two of the identified up-regulated angiogenesis genes. One of these genes is BN0782 shown in Table 1, a novel gene whose expression peaks at the 6 hour time point of the *in vitro* angiogenesis model (Figure 2A), while the other gene is BN0481 (KPNA4) as shown in Table 2, which is a previously identified gene that has not before been shown to have a role in angiogenesis. The expression of BNO481 also peaks at the 6 hour time point of the *in vitro* angiogenesis model (Figure 2B).

### RNAi oligonucleotide design

Short interfering RNA (siRNA) oligonucleotides for RNAi-mediated knock-down of BN0782 and BNO481 were identified through application of in-house computer software. This software incorporates a series of parameters for selecting appropriate siRNA oligonucleotides. These parameters ensure that the siRNA sequence starts after an AA dinucleotide, the siRNA is in the open reading frame of the gene and 100 bp downstream the ATG start codon, the GC content of the siRNA is between 35% and 60%, and the siRNA does not have stretches of more than three T, A, C or G nucleotides. siRNA sequences that harbour low complexity regions were not used. In addition, BLAST analysis was used to select against probes that cross-hybridize with a number of genes (Blastn_refseq at "expect 500" and "word size 7" and alignment scores accepted at 19>score>15 where: alignment score = length_match - (gap+mismatch). siRNAs were synthesised in hair-pin format for cloning into retroviral vectors. For each gene, three siRNA oligonucleotides were selected with each one being examined individually for their effects on gene-knock-down and EC function.

### Retroviral infection of HUVE cells

Each siRNA oligonucleotide was cloned into a retroviral vector for the delivery of the oligonucleotide to human umbilical vein endothelial cells (HUVECs). The siRNA vector was constructed through a modification of pMSCVpuro (BD Biosciences). Briefly, the 3' LTR of pMSCVpuro was inactivated by removal of the XbaI/NheI fragment. A H1-RNA Polymerase III promoter cassette was then inserted into the MCS of the vector. Annealed siRNA primers were ligated into the modified vector (pMSCVpuro(H1)) digested with BglII and HindIII restriction enzymes.

For virus production prior to infection of HUVECs, 293T cells were plated at a density of 1 x 10⁶ cells per well of a 6 well plate 18-24 hours before transfection in RPMI media (Invitrogen) supplemented with 10% FCS (Invitrogen) and 1.0 M Hepes (Invitrogen) without antibiotics. Cells were co-transfected with 2 µg retroviral DNA and 1.5 µg pVPack-VSV-G- (Stratagene), 1.5 µg pVPack-GP (Stratagene) using Lipofectamine 2000 reagent (Invitrogen). Transfected cells were incubated overnight in 5% CO₂ at 37°C. The following day, media containing the DNA/LF2000 complexes was removed and replaced with RPMI supplemented with 10% FCS, 1.0 M Hepes and 1% PSG (Invitrogen). Virus containing supernatants were collected 48-72 hours post transfection and filtered using a 0.45 µM filter. Virus was aliquoted and stored at -80°C.

For the retroviral infection of HUVECs (Clonetics), cells were plated 24 hours before infection in EGM-2 media (Clonetics) at a density of 1.3 x 10⁵ cells per well of a 6 well plate. The following day, 500 µl of virus supernatant was combined with 500 µl of EGM-2 complete media. Polybrene (Sigma) was added to a final concentration of 8.0 µg/ml. Media was aspirated from the cells and replaced with the viral mix. Cells were incubated with the viral mix in 5% CO₂ at 37°C. After 3 hours incubation, an additional 1.0 ml of EGM-2 media was added and cells were incubated for a further 24 hours. After this time HUVE cells were split 1:2 and replated into a 6 well plate. Cells were incubated for 24 hours following splitting to allow them to recover and adhere. To select for infected cells, medium was replaced with EGM-2 complete medium containing puromycin (Sigma) at a 0.4 µg/ml final concentration. Cells were incubated until uninfected cells treated with puromycin had died and infected resistant cells had grown to confluence. Media containing puromycin was replaced every 48 hours to replenish puromycin and remove cell debris. Once resistant cells were grown to confluence (approximately 4-5 days after starting selection), cells were washed in PBS, trypsinised and their properties analysed using the Matrigel capillary tube formation assay.

### Capillary tube formation assay

96 well tissue culture plates were coated with 50 µl of cold Matrigel (BD Biosciences) at 4°C in a two layer process. Matrigel was allowed to polymerize at 37°C for a minimum of 30 minutes before being used. Trypsinised cells were collected in 500 µl of EGM-2 media then centrifuged at 400 rcf for 3 minutes to pellet cells. This allows for the removal of trypsin that may interfere with the assay. Cell pellets were resuspended in 500 µl EGM-2 media then counted using a heamocytometer. Cells were diluted to 2.5 x 10⁵ cells/ml in EGM-2 media. 100µl of the diluted cell suspension was added to duplicate Matrigel coated wells. The final cell density was 25,000 cells/well. Plates were incubated for 22 hours in a humidified incubator at 37°C with 5% CO₂. Images were obtained using an Olympus BX-51 microscope with a 4x objective and Optronics MagnaFire software. Remaining cells were pelleted at 400 rcf for 3 minutes, then media was removed and pellets stored at -80°C for extraction of RNA for real-time RT-PCR analysis (see below). For all assays performed, a vector control was included. This consisted of HUVECs undergoing the infection and selection process with virus made for the vector containing no siRNA insert. This allows for comparison of capillary tube formation ability between a control (vector) and the individual siRNA under analysis.

### Real-time RT-PCR analysis

To determine the level of gene knock-down (mediated by the siRNAs) occurring in the HUVECs, real-time RT-PCR was employed. This involved isolation of RNA from infected cells using the RNeasy Mini or Midi kits (Qiagen) as per manufacturer's instructions (including the on-column DNase treatment). Total RNA was visualised on a 1.2% TBE agarose gel containing ethidium bromide to check for quality and purity. Total RNA concentration was determined by A₂₆₀ on a spectrophotometer.

For the synthesis of cDNA, total RNA (at least lug and preferably at a concentration >1.0 ug/ul) was reverse transcribed using M-MLV (Promega) as per manufacturer's directions. Briefly, the RNA sample to be analysed was made up to 13 ul with water and 1.0 ul of oligo-dT primer (500ng/ul) was added. After incubating at 70°C for 5 minutes, the tubes were placed on ice for 5 minutes and 11 ul of a pre-made master mix containing 5.0 ul M-MLV RT 5x Reaction Buffer, 1.25 ul 10 mM dNTP mix, 1.0 ul of M-MLV RT (H⁻ point mutant) enzyme, and 3.75 ul water was added. This mix was incubated at 40°C for one hour, and the reaction terminated by incubating at 70°C for 15 minutes.

Real-Time PCRs were run on the RotorGene™ 2000 system (Corbett Research). Reactions used AmpliTaq Gold enzyme (Applied Biosystems) and followed the manufacturers instructions. Real-Time PCR reactions were typically performed in a volume of 25 ul and consisted of 1X AmpliTaq Gold Buffer, 200 nM dNTP mix, 2.0 mM MgCl₂ (may vary for primer combination used), 0.3 uM of each primer, 1X SYBR Green mix (Cambrex BioScience Rockland Inc), 1.2 ul of AmpliTaq Gold Enzyme, and 10 ul of a 1 in 5 dilution of the cDNA template.

Cycling conditions were typically performed at 94°C for 12 minutes, followed by 35 cycles of 94°C for 15 seconds, 60°C for 15 seconds, and 72°C for 20 seconds. The annealing temperature of the primers may vary depending on the properties of the primers used.

The PCR cycling was followed by the generation of a melt curve using the RotorGene™ 2000 software where the amount of annealed product was determined by holding at each degree between 50°C and 99°C and measuring the absorbance. All products were run on a 1.2% agarose gel containing ethidium bromide to check specificity in addition to observing the melt curve.

The level of knock-down of a particular gene was then measured by a comparison of its expression level in HDVECs infected with the relevant siRNA under investigation as opposed to HUVECs infected with the retroviral vector alone.

### In vitro regulation of HUVEC function - BNO782 and BNO481

The siRNA oligonucleotides designed to knock-down BNO782 and BNO481 expression are represented by SEQ ID Numbers: 45-47 and SEQ ID Numbers: 48-50 respectively. Real-time RT-PCR analysis of HUVECs retrovirally infected with these siRNAs revealed that each siRNA was able to knock-down the expression of BN0782 or HNO481 to varying degrees. The level of BNO782 expression knock-down mediated by BNO782 siRNA2 (SEQ ID NO: 46) was 24% (Figure 3A), while expression of BN0481 was reduced by 36% (Figure 3B) using BN0481 siRNA1 (SEQ ID NO: 48). Both of these siRNAs were subsequently used separately in Matrigel assays to examine the effects that this level of knock-down for each gene had on the ability of HUVECs to participate in capillary tube formation. As can be seen in Figure 4, reducing BNO782 or BNO481 mRNA levels inhibits HUVEC tube formation. Vector infected cells formed extensive networks of tube structures (Figure 4A and 4C) while cells infected with BNO782 siRNA2 or BNO481 signal exhibited tube structure networks of significantly reduced complexity with a high number of incomplete tube extensions (Figure 4B and 4D). This result confirms a role for both BN0782 and BNO481 in the process of angiogenesis.

### Protein interaction studies

The ability of any one of the angiogenic proteins of the invention, including BNO782 and BNO481, to bind known and unknown proteins can be examined. Procedures such as the yeast two-hybrid system are used to discover and identify any functional partners. The principle behind the yeast two-hybrid procedure is that many eukaryotic transcriptional activators, including those in yeast, consist of two discrete modular domains. The first is a DNA-binding domain that binds to a specific promoter sequence and the second is an activation domain that directs the RNA polymerase II complex to transcribe the gene downstream of the DNA binding site. Both domains are required for transcriptional activation as neither domain can activate transcription on its own. In the yeast two-hybrid procedure, the gene of interest or parts thereof (BAIT), is cloned in such a way that it is expressed, as a fusion to a peptide that has a DNA binding domain. A second gene, or number of genes, such as those from a cDNA library (TARGET), is cloned so that it is expressed as a fusion to an activation domain. Interaction of the protein of interest with its binding partner brings the DNA-binding peptide together with the activation domain and initiates transcription of the reporter genes. The first reporter gene will select for yeast cells that contain interacting proteins (this reporter is usually a nutritional gene required for growth on selective media). The second reporter is used for confirmation and while being expressed in response to interacting proteins it is usually not required for growth.

The nature of the interacting genes and proteins can also be studied such that these partners can also be targets for drug discovery.

### Structural studies

Recombinant angiogenic proteins of the invention can be produced in bacterial, yeast, insect and/or mammalian cells and used in crystallographical and NMR studies. Together with molecular modeling of the protein, structure-driven drug design can be facilitated.

**(Equation 1)**

| Novel Angiogenesis Genes | | | | | |
|---|---|---|---|---|---|
| **BNO Number** | **Symbol** | **Gene Description - Homology** | **UniGene Number** | **GenBank Number** | **Peak Expression (h)** |
| BNO605 | BNO605 | EST, UI-HF-BROp-ajy-c-08-0-UI.s1 Homo sapiens cDNA | None | AW576601 | |
| BNO612 | FLJ20445 | hypothetical protein FLJ20445 | Hs.343748 | NM_017824 | 6 |
| BNO616 | MGC2747 | hypothetical protein MGC2747 | Hs.194017 | NM_024104 | 0.5, 6 |
| BNO617 | FLJ20986 | hypothetical protein FLJ20986 | Hs.324507 | NM_024524 | 6 |
| BNO618 | FLJ14834 | hypothetical protein FLJ14834 | Hs.62905 | NM_032849 | 3 |
| BNO620 | FLJ22746 | hypothetical protein FLJ22746 | Hs.147585 | NM_024785 | 0.5 |
| BNO622 | KIAA1376 | KIAA1376 protein | Hs.24684 | BC015928 | 3, 24 |
| BNO627 | BNO627 | EST, AV756199 BM Homo sapiens cDNA clone BMFAUH02 5' | None | SEQ ID NO: 1 | 6 |
| BNO628 | BNO628 | EST, QV1-BT0631-130300-111-e03 BT0631 Homo sapiens cDNA | None | SEQ ID NO: 2 | 6 |
| BNO629 | BNO629 | EST, Homo sapiens cDNA clone IMAGE:2664022 3' | None | SEQ ID NO: 3 | 6 |
| BNO630 | BNO630 | EST, Homo sapiens cDNA clone IMAGE:2357465 3' | None | SEQ ID NO: 4, 51 | 6 |
| BNO632 | BNO632 | ESTs | Hs.404198 | SEQ ID NO: 5 | 6 |
| BNO633 | BNO633 | ESTs, Weakly similar to hypothetical protein FLJ20378 | Hs.310598 | SEQ ID NO: 6 | 24 |
| BNO634 | BNO634 | ESTs | Hs.345443 | SEQ ID NO: 7 | 6 |
| BNO635 | BNO635 | Hypothetical protein | Hs.54347 | BC057847 | 6 |
| BNO636 | BNO636 | ESTs | Hs.105636 | SEQ ID NO: 8 | 3 |
| BNO637 | BNO637 | ESTs | Hs.486928 | SEQ ID NO: 9, 52 | 6 |
| BNO638 | BNO638 | EST | None | SEQ ID NO: 10 | 6 |
| BNO639 | BNO639 | None | None | SEQ ID NO: 11, 53 | 6 |
| BNO640 | BNO640 | None | None | SEQ ID NO: 12 | 6 |
| BNO645 | FLJ10498 | hypothetical protein FLJ10498 | Hs.270107 | NM_018115 | 24 |
| BNO648 | LOC57146 | hypothetical protein from clone 24796 | Hs.27191 | NM_020422 | 0.5 |
| BNO652 | FLJ31051 | hypothetical protein FLJ31051 | Hs.406199 | NM_153687 | 6 |
| BNO655 | LOC51122 | HSPC042 protein | Hs.432729 | NM_016094 | 3 |
| BNO659 | FLJ32123 | FLJ32123 | Hs.349397 | AK056685 | 6 |
| BNO662 | BNO662 | ESTs | Hs.444495 | BX47355 | 6 |
| BNO664 | FLJ10312 | FLJ10312 | None | NM_030672 | 3 |
| BNO669 | BNO669 | ESTs | Hs.172998 | BC030094 | 3 |
| BNO671 | KIAA0882 | KIAA0882 protein, | Hs.411317 | AB020689 | 3 |
| BNO673 | BNO673 | hypothetical protein DKFZp434L142 | Hs.323583 | NM_016613 | 6 |
| BNO675 | FLJ10700 | hypothetical protein FLJ10700 | Hs.295909 | NM_018182 | 3 |
| BNO677 | FLJ30135 | FLJ30135 | Hs.34906 | BC020494 | 3, 24 |
| BNO685 | FLJ10849 | hypothetical protein FLJ10849 | Hs.386784 | NM_018243 | |
| BNO687 | MGC45416 | hypothetical protein MGC45416 | Hs.95835 | NM_152398 | 24 |
| BNO690 | C15orf15 | chromosome 15 open reading frame 15 | Hs.274772 | NM_016304 | 3 |
| BNO694 | BNO694 | cDNA DKFZp588E0124 | None | AL050030 | 6 |
| BNO697 | BNO697 | Hypothetical protein MGC45871 | Hs.345588 | BC014203 | 24 |
| BNO700 | C7orf30 | chromosome 7 open reading frame 30 | Hs.87385 | NM_138446 | 24 |
| BNO704 | KIAA1102 | KIAA1102 protein | Hs.156761 | AB029025 | |
| BNO705 | BNO705 | ESTs | Hs.30280 | SEQ ID NO: 13 | 3 |
| BNO706 | LOC116441 | hypothetical protein BC014339 | Hs.22026 | NM_138786 | 24 |
| BNO708 | BNO708 | ESTs | Hs.12876 | SEQ ID NO: 14 | 6 |
| BNO710 | BNO710 | FLJ23228 | Hs.170623 | AK026881 | 6 |
| BNO712 | BNO712 | FLJ21592 | Hs.5921 | AK025245 | 3 |
| BNO713 | KIAA0970 | KIAA0970 protein | Hs.103329 | NM_014923 | 6 |
| BNO714 | KIAA0121 | KIAA0121 gene product | Hs.155584 | D50911 | 6 |
| BNO723 | C14orf123 | chromosome 14 open reading frame 123 | Hs.279761 | NM_014169 | 6 |
| BNO725 | KIAA0582 | KIAA0582 protein | Hs.146007 | NM_015147 | 24 |
| BNO730 | BNO730 | ESTs | Hs.158753 | SEQ ID NO: 15 | 6 |
| BNO731 | C6orf166 | chromosome 6 open reading frame 166 | Hs.201864 | NM_018064 | 3 |
| BNO735 | FLJ32029 | Unnamed protein product | Hs.26612 | NM_173582 | 6 |
| BNO737 | BNO737 | hypothetical protein DKFZp434F0318 | Hs.23388 | NM_030817 | |
| BNO740 | KIAA1728 | KIAA1728 protein | Hs.437362 | AB051515 | 24 |
| BNO742 | BNO742 | hypothetical protein FLJ11795 | Hs.84560 | NM_024669 | 24 |
| BNO745 | BNO745 | hypothetical protein DKFZp547A023 | Hs.374649 | NM 018704 | 6 |
| BNO747 | MGC23937 | hypothetical protein MGC23937 similar to CG4798 | Hs.91612 | NM_145052 | 6 |
| BNO753 | BNO753 | cDNA DKFZp667P1024 | Hs.127811 | AL832835 | 3 |
| BNO754 | KJAA0303 | KIAA0303 protein | Hs.212787 | AB002301 | 3 |
| BNO756 | BNO756 | ESTs | Hs.443155 | SEQ ID NO: 16, 54 | |
| BNO759 | KIAA1416 | KIAA1416 protein | Hs.397426 | AB037837 | 6 |
| BNO761 | C7orf24 | chromosome 7 open reading frame 24 | Hs.444840 | NM_024051 | 6 |
| BNO762 | FLJ11223 | cDNA FLJ11223 | Hs.92308 | AL832083 | 3 |
| BNO768 | FLJ30478 | cDNA FLJ30478 | Hs.298258 | AK092048 | 6 |
| BNO772 | FLJ10525 | Hypothetical protein FLJ10525 | Hs.31082 | NM 018126 | 6 |
| BNO780 | LOC58489 | hypothetical protein from EUROIMAGE 588495 | Hs.26765 | AL390079 | 3 |
| BNO782 | MGC26717 | Hypothetical protein | Hs.406060 | BC024188 | 6 |
| BNO791 | KIAA1053 | KIAA1053 protein | Hs.98259 | NM_015589 | 6 |
| BNO793 | KIAA0766 | KIAA0766 gene product | Hs.28020 | NM_014805 | 24 |
| BNO795 | BNO795 | ESTs moderately similar to MDC-3.13 isoform 2 mRNA | Hs.306343 | AK123281 | 6 |
| BNO800 | KIAA1577 | KIAA1 577 protein | Hs.449290 | AB046797 | 6 |
| BNO802 | KIAA0877 | KIAA0877 protein | Hs.408623 | AB020684 | 24 |
| BNO812 | KIAA0372 | KIAA0372 gene product | Hs.435330 | NM_014639 | 6 |
| BNO816 | BNO816 | cDNA clone 4052238 | Hs.348514 | BC014384 | 6 |
| BNO818 | MGC10067 | hypothetical protein MGC10067 | Hs.42251 | NM_145049 | 3 |
| BNO819 | KIAA1191 | KIAA1191 protein | Hs.8594 | NM_020444 | 24 |
| BNO821 | BNO821 | ESTs | Hs.87606 | SEQ ID NO: 17 | 24 |
| BNO825 | FBXO30 | F-box protein 30 | Hs.421095 | NM_032145 | 3 |
| BNO831 | C8orf1 | chromosome 8 open reading frame 1 | Hs.436445 | NM_004337 | 24 |
| BNO833 | C6orf115 | Chromosome 6 open reading frame 115 | Hs.238205 | BC014953 | 24 |
| BNO838 | BNO838 | ESTs | Hs.319095, | SEQ ID NO: 18 | 3 |
| BNO845 | FLJ23728 | cDNA FLJ23728 | Hs.191094 | AK074308 | 6 |
| BNO848 | C10orf45 | Chromosome 10 open reading frame 45 | Hs.103378 | NM 031453 | 24 |
| BNO849 | BisO849 | cDNA DKFZp434G0972 | Hs.106148 | AL133577 | 24 |
| BNO852 | CGI-111 | CGI-111 protein | Hs.11085 | NM_016048 | 6 |
| BNO856 | LOC116068 | hypothetical protein LOC116068 | Hs.136235 | AL832721 | 24 |
| BNO857 | C12orP2 | chromosome 12 open reading frame 2 | Hs. 140821 | NM_007211 | 6 |
| BNO862 | BNO862 | DKFZP434C212 protein | Hs.287266 | AK023841 | |
| BNO868 | BNO868 | DKFZP566C134 protein | Hs.20237 | AB040922 | 3 |
| BNO870 | LOC57228 | hypothetical protein from clone 643 | Hs.206501 | NM_020467 | 24 |
| BNO871 | KIAA1463 | KIAA1463 protein | Hs.21104 | AB040896 | 6 |
| BNO873 | KIAA1376 | KIAA1376 protein | Hs.24684 | NM_020801 | 0.5, 24 |
| BNO876 | FLJ10326 | hypothetical protein FLJ10326 | Hs.262823 | NM_018060 | 24 |
| BNO878 | BNO878 | hypothetical protein DKFZp761L1417 | Hs.270753 | NM_152913 | 6 |
| BNO881 | MGC11349 | hypothetical protein MGC11349 | Hs.288697 | NM_025112 | 6 |
| BNO883 | FLJ39541 | similar to RIKEN cDNA 9130404H11 gene | Hs.21388 | NM_178566 | 6 |
| BNO886 | BNO886 | cDNA DKFZp686D04119 | Hs.30258 | BX537597 | 6 |
| BNO887 | KIAA0648 | KIAA0648 protein | Hs.31921 | NM_015200 | 24 |
| BNO890 | KIAA1160 | KIAA1160 protein | Hs.512661 | NM_020701 | 3 |
| BNO892 | C20orf108 | chromosome 20 open reading frame 108 | Hs.143736 | NM_080821 | 3 |
| BNO894 | KIAA0205 | KIAA0205 gene product | Hs.528724 | NM_014873 | 6 |
| BNO895 | C20orf112 | chromosome 20 open reading frame 112 | Hs.335142 | NM_080616 | 0.5 |
| BNO898 | BNO898 | clone IMAGE:5243590 | Hs.454832 | BC036880 | 6 |
| BNO905 | KIAA1462 | KIAA1462 protein | Hs.192726 | AB040895 | 3 |
| BNO906 | KIAA1199 | KIAA1199 protein | Hs.212584 | AB033025 | 6 |
| BNO908 | C15orf12 | chromosome 15 open reading frame 12 | Hs.513041 | NM_018285 | |
| BNO910 | BNO910 | cDNA OKFZp564F053 | Hs.529772 | AL049265 | 6 |
| BNO917 | BNO917 | hypothetical protein dJ465N24.2.1 | Hs.259412 | NM_020317 | 24 |
| BNO926 | KIAA1238 | KIAA1238 protein | Hs.372288 | AB033064 | |
| BNO928 | BNO928 | EST | None | SEQ ID NO: 19 | 3 |
| BNO929 | BNO929 | EST | None | SEQ ID NO: 20 | 6 |
| BNO930 | BNO930 | EST | Hs.478376 | SEQ ID NO: 21 1 | 6 |
| BNO932 | BNO932 | EST | Hs.492501 | SEQID NO: 22, 55 | 3 |
| BNO933 | BNO933 | EST | None | SEQ ID NO: 23 | 6 |
| BNO934 | BNO934 | EST | None | SEQ ID NO: 24 | 6 |
| BNO935 | BNO935 | EST | None | SEQ ID NO: 25 | 6 |
| BNO936 | BNO936 | EST | None | SEQ ID NO: 26, 56 | 6 |
| BNO937 | BNO937 | alpha gene sequence | None | AF203815 | 6 |
| BNO938 | BNO938 | EST | None | SEQ ID NO: 27 | 0.5 |
| BNO939 | BNO939 | EST | None | SEQ ID NO: 28 | 6 |
| BNO940 | BNO940 | EST | None | SEQ ID NO: 29 | 6 |
| BNO941 | BNO941 | EST | None | SEQ ID NO: 30 | 3 |
| BNO942 | BNO942 | EST | None | SEQID NO: 31 | 6 |
| BNO943 | BNO943 | EST | None | SEQ ID NO: 32 | 6 |
| BNO944 | BNO944 | EST | None | SEQ ID NO: 33 | 6 |
| BNO945 | BNO945 | EST | None | SEQ ID NO: 34 | 6 |
| BNO946 | BNO946 | EST | None | SEQ ID NO: 35, 57 | 6 |
| BNO948 | BNO948 | EST | None | SEQ ID NO: 36 | 6 |
| BNO949 | BNO949 | EST | None | SEQ ID NO: 37, 58 | 3 |
| BNO950 | BNO950 | EST | None | SEQ ID NO: 38 | 24 |
| BNO951 | BNO951 | EST | None | SEQ ID NO: 39 | 24 |
| BNO953 | BNO953 | EST | None | SEQ ID NO: 40 | 24 |
| BNO961 | BNO961 | FLJ00138 protein | Hs.199749 | AK074067 | 3, 24 |
| BNO1018 | BNO1018 | EST | Hs.485935 | SEQ ID NO: 41 | 3 |
| BNO1019 | BNO1019 | EST | None | SEQ ID NO: 42 | 24 |
| BNO1020 | BNO1020 | EST | None | SEQ ID NO: 43 | 3 |
| BNO1021 | BNO1021 | EST | None | SEQ ID NO: 44 | 3 |

**TABLE 2**

| Genes with a Previously Unknown Role in Angiogenesis | | | | | |
|---|---|---|---|---|---|
| BNO Number | Symbol | Gene Description - Homology | UniGene Number | GenBanlc Number | Peak Expression (h) |
| BNO436 | NP | nucleoside phosphorylase | Hs.75514 | NM 000270 | 6 |
| BNO438 | CD59 | CD59 antigen p18-20 | Hs.278573 | NM_000611 | 24 |
| BNO441 | BIRC3 | baculoviral IAP repeat-containing 3 | Hs.127799 | NM_001165 | 3 |
| BNO442 | FABP5 | fatty acid binding protein 5 (psoriasis-associated) | Hs.408061 | NM_001444 | 24 |
| BNO443 | CBFB | core-binding factor, beta subunit | Hs.179881 | NM_001755 | 6 |
| BNO446 | INHBA | inhibin, beta A (activin A, activin AB alpha polypeptide) | Hs.727 | NM_002192 | 6 |
| BNO447 | MGST2 | microsomal glutathione S-transferase 2 | Hs.81874 | NM_002413 | 24 |
| BNO448 | RAB6A | RAB6A, member RAS oncogene family | Hs.5636 | NM_002869 | 6 |
| BNO449 | SAT | spermidine/spermine N1-acetyltransferase | Hs.28491 | NM_002970 | 6 |
| BNO451 | TXNRD1 | thioredoxin reductase 1 | Hs.13046 | NM_003330 | 6 |
| BNO452 | SLC4A7 | solute carrier family 4, sodium bicarbonate cotransporter, member 7 | Hs.132904 | NM_003615 | 6 |
| BNO453 | PPAP2B | phosphatidic acid phosphatase type 2B | Hs.432840 | NM_003713 | 3 |
| BNO454 | BCL10 | B-cell CLUlymphoma 10 | Hs.193516 | NM_003921 | 3 |
| BNO455 | DUSP1 | dual specificity phosphatase 1 | Hs.171695 | NM_004417 | 0.5 |
| BNO456 | KIF5B | kinesin family member 5B | Hs.149436 | NM_004521 | 6 |
| BNO457 | WTAP | Wilms' tumour 1-associating protein | Hs.119 | M_004906 | 0.5 |
| BNO459 | FOS | v-fos FBJ murine osteosarcoma viral oncogene homolog | Hs.25647 | NM_005252 | 0.5 |
| BNO460 | GATA6 | GATA binding protein 6 | Hs.50924 | NM_005257 | 3 |
| BNO461 | HRY | hairy and enhancer of split 1, (Drosophila) | Hs.250666 | NM_005524 | 0.5 |
| BNO462 | SGK | serum/glucocorticoid regulated kinase | Hs.296323 | NM_005627 | 3 |
| BNO463 | TIEG | TGFB Inducible early growth response | Hs.82173 | NM_005655 | 0.5 |
| BNO464 | BCAP31 | B-cell receptor-associated protein 31 | Hs.381232 | NM_005745 | |
| BNO465 | CALCRL | calcitonin receptor-like | Hs.152175 | NM_005795 | 24 |
| BNO466 | SUI1 | putative translation initiation factor | Hs.150580 | NM_005801 | 3 |
| BNO467 | TSC22 | transforming growth factor beta-stimulated protein TSC-22 | Hs.114360 | NM_006022 | 6 |
| BNO468 | RAN | RAN, member RAS oncogene family | Hs.426035 | NM_006325 | |
| BNO469 | LYPLA1 | lysophospholipase I | Hs.12540 | NM_006330 | 6 |
| BNO470 | SSFA2 | sperm specific antigen 2 | Hs.351355 | NM_006751 | 6 |
| BNO472 | CLIC4 | chloride intracellular channel 4 | Hs.25035 | NM_013943 | 24 |
| BNO473 | SLC7A11 | solute carrier family 7, member 11 | Hs.6682 | NM_014331 | 3 |
| BNO474 | RAI14 | retinoic acid induced 14 | Hs.15165 | NM_015577 | 6 |
| BNO475 | HSPC014 | chromosome 13 open reading frame 12 | Hs.279813 | NM_015932 | 24 |
| BNO476 | UMP-CMPK | UMP-CMP kinase | Hs.11463 | NM_01630B | 3 |
| BNO477 | SLC38A2 | solute carrier family 38, member 2 | Hs.298275 | NM_018976 | 3 |
| BNO478 | ZNF317 | zinc finger protein 317 | Hs.18587 | NM_020933 | 24 |
| BNO479 | RAB6C | RAB6C, member RAS oncogene family | Hs.333139 | NM_032144 | 24 |
| BNO480 | MKI67IP | MKI67 (FHA domain) interacting nucleolar phosphoprotein | Hs.142838 | NM_032390 | 3 |
| BNO481 | KPNA4 | karyopherin alpha 4 (importin alpha 3) | Hs.288193 | NM_002268 | 3 |
| BNO483 | C14orf32 | chromosome 14 open reading frame 32 | Hs.406401 | NM_144578 | 3 |
| BNO484 | SMARCA2 | SWI/SNF related, matrix associated, regulator of chromatin, A2 | Hs.198296 | NM_003070 | 0.5 |
| BNO485 | SOX4 | Homo sapiens SRY (sex determining region Y)-box 4 (SOX4), mRNA | Hs.83484 | NM_003107 | 3 |
| BNO487 | NR4A3 | nuclear receptor subfamily 4, group A, member 3 | Hs.80561 | NM 006981 | 0.5 |
| BNO488 | NTN4 | netrin 4 | Hs.102541 | NM 021229 | |
| BNO489 | DNCI2 | dynein, cytoplasmic, intermediate polypeptide 2 (DNCI2), mRNA | Hs.66881 | XM_027780 | 0.5 |
| BNO490 | UGCG | UDP-glucose ceramide glucosyltransferase | Hs.432605 | NM_003358 | 0.5, 24 |
| BNO491 | P125 | Sec23-interacting protein p125 | Hs.300208 | NM 007190 | 3 |
| BNO492 | NUDT4 | nudix (nucleoside diphosphate linked moiety X)-type motif 4 | Hs.355399 | NM_019094 | 6 |
| BNO495 | SATB1 | special AT-rich sequence binding protein 1 | Hs.74592 | NM_002971 | 6 |
| BNO496 | BZW1 | basic leucine zipper and W2 domains 1 | Hs.155291 | NM_014670 | 3 |
| BNO497 | TDG | thymine-DNA glycosylase | Hs.173824 | NM_003211 | 6 |
| BNO498 | ACTR3 | ARP3 actin-related protein 3 homolog (yeast) | Hs.380096 | NM_005721 | 24 |
| BNO499 | LAMP2 | lysosomal-associated membrane protein 2 | Hs.8262 | NM_013995 | 6 |
| BNO500 | ERBB21P | erbb2 interacting protein | Hs.8117 | NM_018695 | 6 |
| BNO501 | DNAJB6 | DnaJ (Hsp40) homolog, subfamily B, member 6 | Hs.181195 | NM 005494 | 3 |
| BNO502 | EMP1 | epithelial membrane protein 1 | Hs.79368 | NM_001423 | 6 |
| BNO503 | MAPK1 | mitogen-activated protein kinase 1 | Hs.324473 | NM_002745 | 24 |
| BNO504 | CYP1A1 | cytochrome P450, subfamily 1, polypeptide 1 | Hs.72912 | NM_000499 | 6 |
| BNO505 | ACVR1 | activin A receptor, type I | Hs.150402 | NM_001105 | 3 |
| BNO506 | TPT1 | tumor protein, translationally-controlled 1 | Hs.401448 | NM_003295 | 0.5, 24 |
| BNO507 | VAV3 | vav 3 oncogene | Hs.267659 | NM_006113 | 3 |
| BNO508 | CAP | adenylyl cyclase-associated protein | Hs.104125 | NM_006367 | 24 |
| BNO509 | HSPA5 | Heat shock 70kDa protein 5 (glucose-regulated protein, 78kDa) | Hs.75410 | NM_005347 | 6 |
| BNO510 | TIA1 | TIA1 cytotoxic granule-associated RNA binding protein | Hs.239489 | NM_022173 | 6 |
| BNO511 | CCNT2 | cyclin T2 | Hs.155478 | NM_001241 | 6 |
| BNO512 | CHC1L | chromosome condensation 1-like | Hs.27007 | M_001268 | 0.5 |
| BNO513 | SFPQ | splicing factor proline/glutamine rich | Hs.180610 | NM_005066 | 3 |
| BNO514 | PRKAR1A | protein kinase, cAMP-dependent, regulatory, type I, alpha | Hs.183037 | NM_002734 | 24 |
| BNO515 | RALA | v-ral simian leukemia viral oncogene homolog A (ras related) | Hs.6906 | NM_005402 | |
| BNO516 | ANXA2 | annexin A2 | Hs.217493 | NM_004039 | 0.5 |
| BNO517 | NUP153 | nucleoporin 153kDa | Hs.211608 | NM_005124 | 3 |
| BNO518 | RANBP9 | RAN binding protein 9 | Hs.279886 | NM_005493 | 24 |
| BNO519 | PRPF4B | PRP4 pre-mRNA processing factor 4 homolog B (yeast) | Hs.198891 | NM_003913 | 6 |
| BNO520 | TSN | translin | Hs.75066 | NM_004622 | 6 |
| BNO521 | H3F3A | H3 histone, family 3A | Hs.181307 | NM_002107 | 24 |
| BNO523 | PROS1 | protein S (alpha) | Hs.64016 | NM_000313 | 6 |
| BNO524 | DDX3 | DEAD/H (Asp-Glu-Ala-Asp/His) box polypeptide 3 | Hs.380774 | NM 001356 | 3 |
| 8NO525 | TCF4 | transcription factor 4 | Hs.359289 | M_003199 | 6 |
| BNO526 | PTP4A1 | Protein tyrosine phosphatase type IVA, member 1 | Hs.227777 | NM 003463 | 6 |
| BNO527 | BMPR2 | bone morphogenetic protein receptor, type II(serine/threonine kinase) | Hs.53250 | NM_001204 | 3 |
| BNO528 | NFE2L2 | nuclear factor (erythroid-derived 2)-like 2 | Hs.155396 | NM_006164 | 3 |
| BNO531 | AHR | aryl hydrocarbon receptor | His.170087 | NM_001621 | 3 |
| BNO532 | RANBP7 | RAN binding protein 7 | Hs.5151 | NM_006391 | 3 |
| BNO533 | ARF6 | ADP-ribosylation factor 6 | Hs.89474 | NM 001663 | 3 |
| BNO534 | SCARF1 | SCARF1 Scavenger receptor class F, member 1 | Hs.57735 | NM 003693E | 24 |
| BNO535 | PLU-1 | putative DNA/chromatin binding motif | Hs.143323 | NM_006618 | 24 |
| BNO536 | TOMM20 | translocase of outer mitochondrial membrane 20 (yeast) homolog | Hs.75187 | NM_014765 | 6 |
| BNO537 | B2M | bete-2-micmglobulin | Hs.48516 | NM_004048 | 24 |
| BNO538 | zizimin1 | zizimin1 | Hs.8021 | NM_015296 | 6 |
| BNO539 | ARPP-19 | cyclic AMP phosphoprotein, 19 kD | Hs.7351 | NM_006628 | 3 |
| BNO540 | RAP1B | RAP1B, member of RAS oncogene family | Hs.156764 | NM_015646 | 3 |
| BNO541 | MCP | membrane cofactor protein | Hs.83532 | NM_153826 | 6 |
| BNO542 | IF116 | interferon, gamma-inducible protein 16 | Hs.155530 | NM_005531 | 0.5 |
| BNO543 | PRG1 | proteoglycan 1, secretory granule | Hs.1908 | NM_002727 | |
| BNO544 | KIT | v-kit Hardy-Zuckerman 4 feline sarcoma viral oncogene homolog | Hs.81665 | NM_000222 | 0.5, 24 |
| BNO545 | SYBL1 | synaptobrevin-like 1 | Hs.24167 | NM_005638 | 6 |
| BNO546 | TCF8 | transcription factor 8 (represses interleukin 2 expression) | Hs.232068 | NM_030751 Ë | 6 |
| BNO548 | NXF1 | nuclear RNA export factor 1 | Hs.323502 | NM_006362 | 3, 24 |
| BNO549 | RAP2B | RAP2B, member of RAS oncogene family | Hs.239527 | NM_002886 | 3 |
| BNO551 | IL6ST | interleukin 6 signal transducer (gp130, oncostatin M receptor) | Hs.82065 | NM_002184 | 6 |
| BNO552 | REST | RE1-silencing transcription factor | Hs.401145 | NM_005612 | 6 |
| BNO553 | SLC19A2 | solute carrier family 19 (thiamine transporter), member 2 | Hs.30246 | NM_006996 | 3 |
| BNO554 | EIF4G2 | eukaryotic translation initiation factor 4 gamma, 2 | Hs.183684 | NM_001418 8 | 3 |
| BNO555 | PTPRE | protein tyrosine phosphatase, receptor type, E | Hs.31137 | NM_006504 | 3 |
| BNO556 | PDE3A | phosphodiesterase 3A, cGMP-inhibited | Hs.777 | NM_000921 | 3 |
| BNO557 | C1QR1 | complement component 1, q subcomponent, receptor 1 | Hs.97199 | NM_012072 | 24 |
| BNO558 | RANBP2 | RAN binding protein 2 | Hs.199179 | NM_006267 | |
| BNO559 | KIS | kinase interacting with leukemia-associated gene (stathmin) | Hs.127310 | NM_144624 | 24 |
| BNO560 | HMGCR | 3-hydroxy-3-methylglutaryl-Coenzyme A reductase | Hs.11899 | NM_000859 | 6 |
| BNO561 | PDCD4 | programmed cell death 4 (neoplastic transformation inhibitor) | Hs.326248 | NM_145341 | 3 |
| BNO562 | TACC1 | transforming, acidic coiled-coil containing protein 1 | Hs.173159 | NM_006283 | 0.5 |
| BNO564 | DIS3 | mitotic control protein dis3 homolog | Hs.323346 | NM_014953 | 6 |
| BNO565 | TOP2A | topoisomerase (DNA) II alpha 170kDa | Hs.156346 | NM_001067 | 6 |
| BNO566 | SLC7A2 | solute carrier family 7, member 2 | Hs.153985 | NM_003046 | 6 |
| BNO567 | FH | fumarate hydratase | Hs.75653 | NM_000143 | 6 |
| BNO568 | IL1RL1 | interleukin 1 receptor-like 1 | Hs.66 | NM_003856 | 6 |
| BNO569 | HPRP3P | U4/U6-associated RNA splicing factor | Hs.11776 | NM_004698 | 6 |
| BNO570 | DDX5 | DEAD/H (Asp-Glu-Ala-Asp/His) box polypeptide 5 | Hs.76053 | NM_004396 | |
| BNO571 | MAD2L1 | MAD2 mitotic arrest deficient-like 1 (yeast) | Hs.79078 | NM_002358 | 0.5, 24 |
| BNO572 | MADH7 | MAD, mothers against decapentaplegic homolog 7 (Drosophila) | Hs.100602 | NM_005904 | 3 |
| BNO573 | E2F3 | E2F transcription factor 3 | Hs.1189 | NM_001949 | 3 |
| BNO574 | CSNK2A2 | CSNK2A2 Casein kinase 2, alpha prime polypeptide | Hs.82201 | NM_001896 | 6 |
| BNO575 | MAX | MAX protein | Hs.42712 | NM_002382 | 6 |
| BNO576 | ERAP140 | 140 kDa estrogen receptor associated protein | Hs.339283 | AF493978 | 3 |
| BNO577 | CD9 | CD9 antigen (p24) | Hs.1244 | NM_001769 | 24 |
| BNO578 | ATRX | alpha thalassemia/mental retardation syndrome X-linked | Hs.96264 | NM_000489 | 6 |
| BNO579 | YWHAZ | tyrosine/tryptophan activation protein, zeta polypeptide | Hs.75103 | NM_003406 | 3 |
| BNO580 | IDS | iduronate 2-sulfatase (Hunter syndrome) | Hs.172458 | NM_000202 | 24 |
| BNO581 | SERPINE2 | serine (or cysteine) proteinase inhibitor, clade E, member 2 | Hs.21858 | NM_006216 | 6 |
| BNO582 | DDEF1 | development and differentiation enhancing factor 1 | Hs. 10669 | NM_018482 | 6 |
| BNO583 | GLRX | glutaredoxin (thioltransferase) | Hs.28988 | NM_002064 | 24 |
| BNO584 | MAP3K1 | MAP3K1 Mitogen-activated protein kinase kinase kinase 1 | Hs.170610 | XM_042066 | 3 |
| BNO585 | ANKH | ankylosis, progressive homolog (mouse) | Hs.168640 | NM_054027 | 3 |
| BNO586 | RBX1 | ring-box 1 | Hs.279919 | NM_014248 | 24 |
| BNO587 | NAB1 | NGFI-A binding protein 1 (EGR1 binding protein 1) | Hs.107474 | NM_005966 | 3 |
| BNO588 | TNFSF10 | tumor necrosis factor (ligand) superfamily, member 10 | Hs.83429 | NM_003810 | 3 |
| BNO589 | PRDX3 | peroxiredoxin 3 | Hs.75454 | NM_006793 | 6 |
| BNO590 | MAP2K1 | mitogen-activated protein kinase kinase 1 | Hs.3446 | NM_002755 | 3 |
| BNO591 | NFATC1 | nuclear factor of activated T-cells, calcineurin-dependent 1 | Hs.96149 | NM_006162 | 24 |
| BNO594 | USP7 | ubiquitin specific protease 7 (herpes virus-associated) | Hs.78683 | NM_003470 | |
| BNO595 | ARHB | ras homolog gene family, member B | Hs.406064 | NM_004040 | 3 |
| BNO596 | PTEN | phosphatase and tensin homolog | Hs.10712 | NM_000314 | |
| BNO597 | UBL1 | ubiquitin-like 1 (sentrin) | Hs.81424 | NM_003352 | 24 |
| BNO598 | RAB5A | RAB5A, member RAS oncogene family | Hs.73957 | NM_004162 | 3 |
| BNO599 | ITGB1 | integrin, beta 1 | Hs.287797 | NM_002211 | 24 |
| BNO600 | PRDM2 | PR domain containing 2, with ZNF domain | Hs.26719 | NM_012231 | 6 |
| BNO602 | ITGA2 | integrin, alpha 2 (CD49B, alpha 2 subunit of VLA-2 receptor) | Hs.271986 | NM_002203 | 6 |
| BNO603 | ETV5 | ets variant gene 5 (ets-related molecule) | Hs.43697 | NM_004454 | 3 |
| BNO604 | ZFHX1B | zinc finger homeobox 1b | Hs.34871 | NM_014795 | 3 |
| BNO606 | LOC157713 | lysophospholipase I-like pseudogene on chromosome 6 | None | NG_001063 | |
| BNO607 | RBM3 | RNA binding motif protein 3 | Hs.301404 | NM_006743 | 0.5 |
| BNO609 | NET-6 | transmembrane 4 superfamily member tetraspan NET-6 | Hs.364544 | NM_014399 | 6 |
| BNO610 | EHD3 | EH-domain containing 3 | Hs.87125 | NM_014600 | 24 |
| BNO611 | KIAA0992 | palladin | Hs.194431 | NM_016081 | 6 |
| BNO613 | METL | methyltransferase like 2 | Hs.433213 | NM_018396 | 3 |
| BNO614 | HT010 | uncharacterized hypothalamus protein HT010 | Hs.6375 | NM_018471 | 0.5 |
| BNO615 | C3orf4 | chromosome 3 open reading frame 4 | Hs.107393 | NM_019895 | 6 |
| BNO619 | RPL27A | ribosomal protein L27a | Hs.76064 | NM_000990 | 6 |
| BNO621 | MIB | Ubiquitin ligase mind bomb | Hs.34892 | AY149908 | 0.5 |
| BNO623 | KIAA0261 | KlAA0261 protein | Hs.154978 | XM_042946 | 24 |
| BNO624 | KIAA1199 | KlAA1199 protein | Hs.50081 | XM_051860 | 6 |
| BNO625 | HIF1 | huntingtin interacting protein B | Hs.6947 | NM_014159 | |
| BNO642 | ETL | EGF-TM7-latrophilin-related protein | Hs.57958 | NM_022159 | 24 |
| BNO643 | VMP1 | likely ortholog of rat vacuole membrane protein 1 | Hs.166254 | NM_030938 | 3 |
| BNO644 | TAF9 | TATA box binding protein (TBP)-associated factor, 32kDa | Hs.60679 | NM_16283 | 24 |
| BNO646 | MAN1A1 | mannosidase, alpha, class 1A, member 1 | Hs.432931 | NM_005907 | 6 |
| BNO647 | DOCK4 | Dedicator of cytokinesis 4 | Hs.118140 | NM_014705 | 24 |
| BNO649 | ADAMTS9 | a disintegrin-like and metalloprotease (thrombospondin type 1 motif, 9) | Hs.126855 | NM_020249 | 24 |
| BNO650 | CSNK2A2 | Casein kinase 2, alpha prime polypeptide | Hs.82201 | NM_001896 | 6 |
| BNO651 | RPLP0 | ribosomal protein, large, P0 | Hs.406511 | NM_001002 | 6 |
| BNO653 | GALNT4 | N-acetylgalactosaminyltransferase 4 | Hs.271923 | NM_003774 | 3 |
| BNO654 | GNG2 | guanine nucleotide binding protein (G protein), gamma 2 | Hs.289026 | BC020774 | 6 |
| BNO656 | MBNL | muscleblind-like (Drosophila) | Hs.28578 | NM_021 038 | |
| BNO657 | ARL8 | ADP-ribosylation factor-like 8 | Hs.25362 | BC024163 | 3 |
| BNO658 | ASB3 | ankyrin repeat and SOCS box-containing 3 | Hs.9893 | NM_016115 | 6 |
| BNO660 | GG2-1 | TNF-induced protein | Hs.17839 | NM_014350 | 3 |
| BNO661 | ELL2 | ELL-related RNA polymerase II, elongation factor | Hs.98124 | NM_012081 | 3 |
| BNO663 | ATP5J2 | ATP synthase, H+ transporting, mitochondrial F0 complex, subunit f 2 | Hs.235557 | NM_004889 | 24 |
| BNO665 | SDCBP | syndecan binding protein (syntenin) | Hs.8180 | NM_005625 | 3 |
| BNO666 | KIAA1959 | Nm23-phosphorylated unknown substrate | Hs.55067 | NM_032873 | 3 |
| BNO667 | GNPNAT1 | glucosamine-phosphate N-acetyltransferase 1 | Hs.478025 | NM_198066 | 6 |
| BNO668 | SPRED1 | Sprouty-related, EVH1 domain containing 1 | Hs.132804 | NM_152594 | 3, 24 |
| BNO670 | Nbak2 | homeodomain interacting protein kinase 1-like protein | Hs.12259 | NM_152696 | 6 |
| BNO672 | GABPA | GA binding protein transcription factor, alpha subunit 60kDa | Hs.78 | NM_002040 | 3 |
| BNO674 | V-1 | likely ortholog of rat V-1 protein | Hs.21321 | NM_145808 | 24 |
| BNO676 | C8FW | phosphoprotein regulated by mitogenic pathways | Hs.7837 | NM_025195 | 3 |
| BNO678 | TBC1D4 | TBC1 domain family, member 4 | Hs.173802 | NM_014832 | 6 |
| BNO679 | ACATE2 | likely ortholog of mouse acyl-Coenzyme A thioesterase 2 | Hs.18625 | NM_012332 | 24 |
| BNO680 | CRYZ | crystallin, zeta (quinone reductase) | Hs.83114 | NM_001889 | 6 |
| BNO681 | KPNB1 | karyopherin (importin) beta 1 | Hs.180446 | NM_002265 | 24 |
| BNO682 | RPL23A | ribosomal protein L23a | Hs.350046 | NM_000984 | 0.5 |
| BNO683 | LIMS1 | LIM and senescent cell antigen-like domains 1 | Hs.112378 | NM_004987 | 6 |
| BNO684 | WW45 | WW45 protein | Hs.288906 | NM_021818 | 3 |
| BNO686 | ST3GALVI | alpha2,3-sialyltransferase | Hs.34578 | NM_006100 | 6 |
| BNO688 | CPR8 | cell cycle progression 8 protein | Hs.283753 | NM_004748 | 24 |
| BNO689 | HDCL | hHDC for homolog of Drosophila headcase | Hs.6679 | NM_016217 | 3 |
| BNO691 | UBC | ubiquitin C | Hs.183704 | NM_021 009 | 3 |
| BNO692 | RDX | radixin | Hs.263671 | NM_002906 | 24 |
| BNO693 | PELI1 | pellino homolog 1 (Drosophila) | Hs.7886 | NM_020651 | 3 |
| BNO695 | MCC | mutated in colorectal cancers | Hs.1345 | NM_002387 | 6 |
| BNO696 | RetSDR2 | RetSDR2 Retinal short-chain dehydrogenase/reductase 2 | Hs.282984 | NM_016245 | 3 |
| BNO698 | CSS3 | Chondroitin sulfate synthase 3 | Hs.165050 | AB086062 | 3 |
| BNO699 | BRE | brain and reproductive organ-expressed (TNFRSF1A modulator) | Hs.80426 | NM_004899 | 6 |
| BNO701 | BAZ1A | bromodomain adjacent to zinc finger domain, 1A | Hs.8858 | NM_013448 | 3 |
| BNO702 | HNRPDL | heterogeneous nuclear ribonucleoprotein D-like | Hs.372673 | NM_005463 | 3 |
| BNO703 | PREI3 | preimplantation protein 3 | Hs.107942 | NM_015387 | 6 |
| BNO707 | BNO707 | Human XIST, coding sequence "a" | Hs.83623 | X56199 | 3 |
| BNO709 | ROD1 | ROD1 regulator of differentiation 1 (S. pombe) | Hs.374634 | NM_005156 | 6 |
| BNO711 | SMAP-5 | golgi membrane protein SB140 | Hs.5672 | NM_030799 | 6 |
| BNO715 | M-RIP | Myosin phosphatase-Rho interacting protein | Hs.430725 | AB020671 | 0.5, 24 |
| BNO716 | HIVEP2 | human immunodeficiency virus type I enhancer binding protein 2 | Hs.75063 | NM_006734 | 3 |
| BNO717 | DC42 | hypothetical protein DC42 | None | NM_030921 | 3 |
| BNO718 | GRPEL2 | GrpE-like 2, mitochondrial | Hs.17121 | NM_152407 | 6 |
| BNO719 | PCMF | potassium channel modulatory factor | Hs.5392 | NM_020122 | 3 |
| BNO720 | UBE2E1 | ubiquitin-conjugating enzyme E2E 1 (UBC4/5 homolog, yeast) | Hs.163546 | NM_003341 | 24 |
| BNO721 | KLHL4 | kelch-like 4 (Drosophila) | Hs.49075 | NM_019117 | |
| BNO722 | MANEA | Mannosidase, endo-alpha | Hs.46903 | NM_024641 | 3 |
| BNO724 | TCF12 | transcription factor 12 (HTF4, helix-loop-helix transcription factors 4) | Hs.21704 | NM_003205 | 6 |
| BNO726 | STAF42 | SPT3-associated factor 42 | Hs.435967 | NM_053053 | 6 |
| BNO727 | CYFIP1 | cytoplasmic FMR1 interacting protein 1 | Hs.77257 | NM_014608 | 6 |
| BNO728 | NOL5A | nucleolar protein 5A (56kDa with KKE/D repeat) | Hs.376064 | NM_006392 | 6 |
| BNO729 | GSA7 | ubiquitin activating enzyme E1-like protein | Hs.278607 | NM_006395 | 6 |
| BNO732 | P66 Alpha | P66 Alpha | Hs.118964 | NM_017660 | |
| BNO733 | STAG1 | stromal antigen 1 | Hs.286148 | NM_005862 | |
| BNO734 | MYCT1 | Myc target 1 | Hs.18160 | NM_025107 | 6 |
| BNO736 | SCAMP1 | secretory carrier membrane protein 1 | Hs.31218 | NM_004866 | 3 |
| BNO738 | ACTG1 | actin, gamma 1 | Hs.14376 | NM_001614 | 0.5 |
| BNO739 | HRB2 | HIV-1 rev binding protein 2 | Hs.154762 | NM_007043 | 6 |
| BNO741 | VMP1 | Likely orthologue of rat vacuole membrane protein 1 | Hs.166254 | NM_030938 | 6 |
| BNO743 | BCAT1 | branched chain aminotransferase 1, cytosolic | Hs.438993 | NM_005504 | 0.5, 24 |
| BNO744 | PJA2 | Praja 2, RING-H2 motif containing | Hs.224262 | NM_014819 | |
| BNO746 | FKSG14 | leucine zipper protein FKSG14 | Hs.192843 | NM_022145 | 6 |
| BNO748 | KLHL6 | kelch-like 6 (Drosophila) | Hs.43616 | NM_130446 | 6 |
| BNO749 | TTL | Tubulin tyrosine ligase | Hs.358997 | NM_153712 | 6 |
| BNO750 | CDC23 | CDC23 (cell division cycle 23, yeast, homolog) | Hs.153546 | NM_004661 | 24 |
| BNO751 | ULK2 | unc-51-like kinase 2 (C. elegans) | Hs.151406 | NM_014683 | 3 |
| BNO752 | SCARB2 | SCARB2 Scavenger receptor class B, member 2 | Hs.323567 | NM_005506E | 3 |
| BNO755 | ZMPSTE24 | zinc metalloproteinase (STE24 homolog, yeast) | Hs.25846 | NM_005857 | |
| BNO757 | U5-100K | prp28, U5 snRNP 100 kd protein | Hs.184771 | NM_004818 | |
| BNO758 | CHD4 | chromodomain helicase DNA binding protein 4 | Hs.74441 | NM_001273 | 6 |
| BNO760 | CGI-127 | yippee protein | Hs.184542 | NM_016061 | 3,24 |
| BNO763 | BET1 | BET1 homolog (S. cerevisiae) | Hs.23103 | NM_005868 | 24 |
| BNO764 | ARHGAP5 | Rho GTPase activating protein 5 | Hs.267831 | NM_001173 | |
| BNO765 | TUBA | Scaffold protein TUBA | Hs.429994 | NM_015221 | 3 |
| BNO766 | NUMB | numb homolog (Drosophila) | Hs.78890 | NM_003744 | 6 |
| BNO767 | P5 | protein disulfide isomerase-related protein | Hs.182429 | NM_005742 | 0.5 |
| BNO769 | SFRS21P | splicing factor, arginine/serine-rich 2, interacting protein | Hs.51957 | NM 004719 | 6 |
| BNO770 | OXA1L | oxidase (cytochrome c) assembly 1-like | Hs.151134 | NM_005015 | 0.5,24 |
| BNO771 | POH1 | 26S proteasome-associated pad1 homolog | Hs.178761 | NM_005805 | 6 |
| BNO773 | AHCYL1 | S-adenosylhomocysteine hydrolase-like 1 | Hs.4113 | NM_006621 | 3 |
| BNO774 | UAP1 | UDP-N-acteylglucosamine pyrophosphorylase 1 | Hs.21293 | NM_003115 | 3 |
| BNO775 | PLS3 | plastin 3 (T isoform) | Hs.4114 | NM_005032 | 6 |
| BNO776 | TSNAX | translin-associated factor X | Hs.96247 | NM_005999 | 0.5 |
| BNO777 | HEL01 | homolog of yeast long chain polyunsaturated fatty acid elong. enz. 2 | Hs.250175 | NM_021814 | 6 |
| BNO778 | MAN2A1 | mannosidase, alpha, class 2A, member 1 | Hs.377915 | NM_002372 | 3 |
| BNO779 | RAB21 | RAB21, member RAS oncogene family | Hs.184627 | NM_014999 | 6 |
| BNO781 | WAC | WW domain-containing adapter with a coiled-coil region | Hs.70333 | NM_016628 | 3 |
| BNO783 | POSH | likely ortholog of mouse plenty of SH3 domains | Hs.301804 | AB040927 | 6 |
| BNO784 | RBM9 | RNA binding motif protein 9 | Hs.433574 | NM_014309 | |
| BNO785 | CSRP2 | cysteine and glycine-rich protein 2 | Hs.10526 | NM_001321 | 3 |
| BNO786 | COPA | coatomer protein complex, subunit alpha | Hs.75887 | NM_004371 | 6 |
| BNO787 | TIMM17A | translocase of inner mitochondrial membrane 17 homolog A (yeast) | Hs.20716 | NM_006335 | 6 |
| BNO788 | RIN2 | Ras and Rab interactor2 | Hs.62349 | NM_018993 | 24 |
| BNO789 | KLHL5 | kelch-like 5 (Drosophila) | Hs.272239 | NM_015990 | 24 |
| BNO790 | IPLA2(γ) | intracellular memb.-assoc. calcium-independent phospholipase A2 γ | Hs.44198 | AF263613 | 6 |
| BNO794 | SMARCA5 | SWI/SNF related regulator of chromatin, a5 | Hs.9456 | NM_003601 | |
| BNO796 | FBXL3A | F-box and leucine-rich repeat protein 3A | Hs.7540 | NM_012158 | 24 |
| BNO797 | SART2 | squamous cell carcinoma antigen recognized by T cell | Hs.58636 | NM_013352E | 6 ' |
| BNO798 | YWHAZ | 14-3-3zeta | Hs.386834 | NM_145690 | |
| BNO799 | SH3BGRL2 | SH3 domain binding glutamic acid-rich protein like 2 | Hs.9167 | NM_031469 | 3, 24 |
| BNO801 | PUM1 | pumilio homolog 1 (Drosophila) | Hs.153834 | NM_014676 | 3 |
| BNO803 | CCT2 | chaperonin containing TCP1, subunit 2 (beta) | Hs.432970 | NM_006431 | 6 |
| BNO804 | PTPRK | protein tyrosine phosphatase, receptor type, K | Hs.79005 | NM_002844 | 6 |
| BNO806 | TM4SF1 | transmembrane 4 superfamily member 1 | Hs.351316 | NM_014220 | 6 |
| BNO807 | CHSY1 | carbohydrate (chondroitin) synthase 1 | Hs.110488 | NM_014918 | 24 |
| BNO808 | TERF21P | telomeric repeat binding factor 2, interacting protein | Hs.274428 | NM_018975 | 6 |
| BNO809 | RDC1 | G protein-coupled receptor | Hs.23016 | BC036661 | 3 |
| BNO810 | CD59 | CD59 antigen p18-20 | Hs.278573 | AK095453 | 0.5,6 |
| BNO811 | UBE2D1 | ubiquitin-conjugating enzyme E2D 1 (UBC4/5 homolog, yeast) | Hs.129683 | NM_003338 | 6 |
| BNO813 | CUL4B | cullin 4B | Hs.155976 | NM_003588 | 24 |
| BNO814 | LCHN | LCHN protein | Hs.233044 | AB032973 | 3 |
| BNO815 | PELO | pelota homolog (Drosophila) | Hs.5798 | NM_015946 | 3 |
| BNO817 | MRPS10 | mitochondrial ribosomal protein S10 | Hs.380887 | NM_018141 | 6 |
| BNO820 | EIF3S2 | eukaryotic translation initiation factor 3, subunit 2 beta, 36kDa | Hs.192023 | NM_003757 | 3 |
| BNO822 | UBQLN1 | ubiquilin 1 | Hs.9589 | NM_013438 | 3 |
| BNO823 | PSMB3 | proteasome (prosome, macropain) subunit, beta type, 3 | Hs.82793 | NM_002795 | 0.5, 24 |
| BNO826 | UBE2J1 | ubiquitin-conjugating enzyme E2, J1 (UBC6 homolog, yeast) | Hs.184325 | NM_016336 | 24 |
| BNO827 | CDK2AP1 | CDK2-associated protein 1 | Hs.433201 | NM_004642 | 24 |
| BNO828 | CRY1 | cryptochrome 1 (photolyase-like) | Hs.151573 | NM_004075 | 3 |
| BNO830 | HSPC051 | ubiquinol-cytochrome c reductase complex (7.2 kD) | Hs.284292 | NM_013387 | 6 |
| BNO832 | GNG11 | guanine nucleotide binding protein (G protein), gamma 11 | Hs.83381 | NM_004126 | 0.5, 24 |
| BNO834 | ZNF198 | zinc finger protein 198 | Hs.109526 | NM_003453 | 6 |
| BNO835 | RAB11A | RAB11A, member RAS oncogene family | Hs.75618 | NM_004663 | 6 |
| BNO836 | SMAP1 | stromal membrane-associated protein | Hs.373517 | NM_021940 | 6 |
| BNO837 | COPG | Coatomer protein complex, subunit gamma | Hs.368056 | NM_016128 | 3 |
| BNO839 | MTHFD2 | methylene tetrahydrofolate dehydrogenase (NAD+ dependent) | Hs.154672 | NM_006636 | 3 |
| BNO840 | PODXL | podocalyxin-like | Hs.16426 | NM_005397 | 6 |
| BNO841 | SLC30A7 | Solute carrier family 30 (zinc transporter), member 7 | Hs.38856 | NM_133496 | 3 |
| BNO842 | API5 | apoptosis inhibitor 5 | Hs.227913 | NM_006595 | 3 |
| BNO843 | ERdj5 | ER-resident protein ERdj5 | Hs.1098 | NM_018981 | 3 |
| BNO844 | HDGFRP3 | Hepatoma-derived growth factor, related protein 3 | Hs.127842 | NM_016073 | 6 |
| BNO847 | TUCAN | tumor up-regulated CARD-containing antagonist of caspase nine | Hs.10031 | NM_014959 | 6 |
| BNO850 | PCDH17 | protocadherin 17 | Hs.106511 | NM_014459 | 24 |
| BNO851 | GALNT10 | N-acetylgalactosaminyltransferase 10 | Hs.107260 | NM_017540 | 24 |
| BNO853 | UQCRC1 | ubiquinol-cytochrome c reductase core protein I | Hs.119251 | NM_003365 | 6 |
| BNO854 | RPL3 | ribosomal protein L3 | Hs.119598 | NM_000967 | 24 |
| BNO855 | CMT2 | gene predicted from cDNA with a complete coding sequence | Hs.124 | NM_014628 | 24 |
| BNO858 | PSMD7 | proteasome 26S subunit, non-ATPase, 7 | Hs.155543 | NM_002811 | 6 |
| BNO859 | CCT5 | chaperonin containing TCP1, subunit 5 (epsilon) | Hs.1600 | NM_012073 | 3 |
| BNO860 | SEC5 | homolog of yeast Sec5 | Hs.16580 | NM_018303 | 6 |
| BNO861 | SKP1A | S-phase kinase-associated protein 1A (p19A) | Hs.171626 | NM_006930 | 24 |
| BNO863 | CAPZA1 | capping protein (actin filament) muscle Z-line, alpha 1 | Hs.184270 | NM_006135 | 24 |
| BNO864 | YES1 | v-yes-1 Yamaguchi sarcoma viral oncogene homolog 1 | Hs.194148 | NM_005433 | 24 |
| BNO865 | DAAM1 | dishevelled associated activator of morphogenesis 1 | Hs.197751 | NM_014992 | 6 |
| BNO866 | BCL6B | B-cell CLUlymphoma 6, member B (zinc finger protein) | Hs.22575 | NM_181844 | 6 |
| BNO872 | AF5Q31 | ALL1 fused gene from 5q31 | Hs.231967 | NM_014423 | 6 |
| BNO874 | ALDH9A1 | aldehyde dehydrogenase 9 family, member A1 | Hs.2533 | NM_000696 | 24 |
| BNO875 | CDC42EP3 | CDC42 effector protein (Rho GTPase binding) 3 | Hs.260024 | NM_006449 | 0.5, 24 |
| BNO877 | MIS12 | homolog of yeast Mis12 | Hs.267194 | NM_024039 | 6 |
| BNO879 | ATP6V1D | ATPase, H+ transporting, lysosomal 34kDa, V1 subunit D | Hs.272630 | NM_015994 | 6 |
| BNO880 | VCIP135 | valosin-containing protein (p97)/p47 complex-interacting protein p135 | Hs.287727 | NM_025054 | 6 |
| BNO882 | D10S170 | DNA segment on chromosome 10 (unique) 170 | Hs.288862 | NM_005436 | 6 |
| BNO884 | ARPC3 | actin related protein 2/3 complex, subunit 3, 21kDa | Hs.293750 | NM_005719 | 24 |
| BNO885 | RPS19 | ribosomal protein S19 | Hs.298262 | NM_001022 | 6 |
| BNO888 | NEUGRIN | mesenchymal stem cell protein DSC92 | Hs.323467 | NM_016645 | 6 |
| BNO889 | CALD1 | caldesmon 1 | Hs.325474 | NM_033138 | 0.5 |
| BNO891 | NFIB | nuclear factor I/B | Hs.33287 | NM_005596 | 0.5 |
| BNO893 | HSPCA | heat shock 90kDa protein 1, alpha | Hs.356531 | NM_005348 | 6 |
| BNO896 | NSAP1 | NS1-associated protein 1 | Hs.373499 | NM_006372 | 6 |
| BNO897 | SYT11 | synaptotagmin XI | Hs.380439 | NM_152280 | 6 |
| BNO899 | HNRPC | heterogeneous nuclear ribonucleoprotein C (C1/C2) | Hs.406125 | NM_006321 | 24 |
| BNO900 | STMN1 | stathmin 1/oncoprotein 18 | Hs.406269 | NM_005563 | 6 |
| BNO901 | ATP5B | ATP synthase, H+ transporting, mitochondrial F1 complex, beta | Hs.406510 | NM_001686 | 0.5, 24 |
| BNO902 | PSMB1 | proteasome (prosome, macropain) subunit, beta type, 1 | Hs.407981 | NM_002793 | 0.5, 24 |
| BNO903 | DDX10 | DEAD/H (Asp-Glu-Ala-Asp/His) box polypeptide 10 (RNA helicase) | Hs.41706 | NM_004398 | 6 |
| BNO904 | RPL36AL | ribosomal protein L36a-like | Hs.419465 | NM_001001 | 24 |
| BNO907 | NDUFV2 | NADH dehydrogenase (ubiquinone) flavoprotein 2, 24kDa | Hs.51299 | NM_021074 | 0.5,24 |
| BNO909 | DCK | deoxycytidine kinase | Hs.709 | NM_000788 | 24 |
| BNO911 | MDH1 | malate dehydrogenase 1, NAD (soluble) | Hs.75375 | NM_005917 | 24 |
| BNO912 | SERP1 | stress-associated endoplasmic reticulum protein 1 | Hs.76698 | NM_014445 | 0.5 |
| BNO913 | RPS3A | ribosomal protein S3A | Hs.77039 | NM_001006 | 0.5 |
| BNO914 | ARHA | ras homolog gene family, member A | Hs.77273 | NM_001664 | 0.5 |
| BNO915 | LAMA4 | laminin, alpha 4 | Hs.78672 | NM_002290 | 6 |
| BNO916 | SNX9 | sorting nexin 9 | Hs.7905 | NM_016224 | 6 |
| BNO918 | RAD21 | RAD21 homolog (S. pombe) | Hs.81848 | NM_006265 | 0.5, 24 |
| BNO920 | PHLDA1 | pleckstrin homology-like domain, family A, member 1 | Hs.82101 | NM_007350 | 6 |
| BNO921 | ARHGDIB | Rho GDP dissociation inhibitor (GDI) beta | Hs.83656 | NM_001175 | 24 |
| BNO922 | ELP2 | elongator protein 2 | Hs.8739 | NM_018255 | 6 |
| BNO924 | ATP6V1G1 | ATPase, H+ transporting, lysosomal 13kDa, V1 subunit G isoform 1 | Hs.90336 | NM_004888 | 24 |
| BNO925 | DNAJA1 | DnaJ (Hsp40) homolog, subfamily A, member 1 | Hs.94 | NM_001539 | 3 |
| BNO927 | CYB561 | cytochrome b-561 | None | NM_001915 | 24 |
| BNO947 | HNRPDL | Heterogeneous nuclear ribonucleoprotein D-like | Hs.372673 | NM_005463 | 3 |
| BNO952 | ARHB | Ras homolog gene family, member B | Hs.406064 | NM_004040 | 3 |
| BNO955 | CYB561 | Cytochrome b-561 | Hs.355264 | AK095244 | 24 |
| BNO958 | ATP6 | ATP synthase F0 subunit 6 - mitochondrial gene | None | NC_001807 | 24 |
| BNO969 | ND4L | NADH dehydrogenase subunit 4L - mitochondrial gene | None | NC_001807 | 6 |
| BNO960 | COX2 | cytochrome C oxidase subunit II - mitochondrial gene | None | NC_001807 | 0.5, 24 |
| BNO1014 | SET | SET translocation (myeloid leukemia-associated) | Hs.145279 | NM_003011 | 6 |
| BNO1015 | JUNB | jun B proto-oncogene | Hs.400124 | NM_002229 | 0.5 |
| BNO1016 | HMGB1 | high-mobility group box 1 | Hs:6727 | NM_002128 | 6 |
| BNO1017 | PAFAH1B2 | Platelet-activating factor acetylhydrolase, isoform Ib, beta subunit | Hs.93354 | NM_002572 | 24 |

**TABLE 3**

| Genes Previously Associated with Angiogenesis | | | | | |
|---|---|---|---|---|---|
| BNO Number | Symbol | Gene Description - Homology | UniGene Number | GenBank Number | Peak Expression (h) |
| BNO435 | ICAM1 | intercellular adhesion molecule 1 (CD54), human rhinovirus receptor | Hs.168383 | NM_000201 | 3 |
| BNO437 | IL8 | interleukin 8 | Hs.624 | NM_000584 | 3 |
| BNO439 | VCAM1 | vascular cell adhesion molecule 1 | Hs.109225 | NM_001078 | 3 |
| BNO440 | ANGPT2 | angiopoietin 2 | Hs.115181 | NM_001147 | 6 |
| BNO444 | CTNNB1 | catenin (cadherin-associated protein), beta 1, 88kDa | Hs.171271 | NM_001904 | 3 |
| BNO445 | F3 | coagulation factor III (thromboplastin, tissue factor) | Hs.62192 | NM_001993 | 3 |
| BNO450 | STC1 | stanniocalcin 1 | Hs.25590 | NM_003155 | 24 |
| BNO458 | ADAMTS4 | a disintegrin-like and metalloprotease (thrombospondin type 1 motif, 4) | Hs.211604 | NM_005099 | 6 |
| BNO471 | ESM1 | endothelial cell-specific molecule 1 | Hs.41716 | NM_007036 | 3, 24 |
| BNO482 | CMG2 | capillary morphogenesis protein 2 | Hs.5897 | NM_058172 | 6 |
| BNO486 | EFNB2 | ephrin-B2 | Hs.30942 | NM_004093 | 3 |
| BNO493 | PTGS1 | prostaglandin-endoperoxide synthase 1 | Hs.88474 | NM_000962 | 6 |
| BNO494 | KDR | kinase insert domain receptor (a type III receptor tyrosine kinase) | Hs.12337 | NM_002253 | |
| BNO522 | F2R | coagulation factor II (thrombin) receptor | Hs.128087 | NM_001992 | 3 |
| BNO529 | CTSB | cathepsin B | Hs.297939 | NM_001908 | 24 |
| BNO530 | LIF | leukemia inhibitory factor (cholinergic differentiation factor) | Hs.2250 | NM_002309 | 3 |
| BNO547 | EDN1 | endothelin 1 | Hs.2271 | NM_001955 | 0.5 |
| BNO550 | JAK1 | Janus kinase 1 (a protein tyrosine kinase) | Hs.50651 | NM_002227 | 24 |
| BNO563 | THBD | thrombomodulin | Hs.2030 | NM_000361 | 24 |
| BNO592 | PSEN1 | presenilin 1 (Alzheimer disease 3) | Hs.3260 | NM_000021 | 0.5 |
| BNO593 | STAT3 | signal transducer and activator of transcription 3 | Hs.321677 | NM_139276 | 6 |
| BNO601 | GJA1 | gap junction protein, alpha 1, 43kDa (connexin 43) | Hs.74471 | NM_000165 | 3 |
| BNO608 | HEY1 | hairy/enhancer-of-split related with YRPW motif 1 | Hs.234434 | NM_012258 | 0.5 |
| BNO846 | CXCR4 | chemokine (C-X-C motif) receptor 4 | Hs.89414 | NM_003467 | 24 |
| BNO869 | ENTPD1 | ectonucleoside triphosphate diphosphohydrolase 1 | Hs.205353 | NM_001776 | 0.5 |
| BNO919 | SERPINE1 | serine (or cysteine) proteinase Inhibitor, clade E, member 1 | Hs.82085 | NM_000602 | 3 |
| BNO923 | THBS1 | thrombospondin 1 | Hs.87409 | NM_003246 | 0.5 |

### References

References cited herein are listed on the following pages, and are incorporated herein by this reference.
Altschul, SF. et al. (1997). Nucleic Acids Res. 25: 3389-3402.
Breaker, RR. and Joyce, GF. (1995). Chem. Biol. 2: 655-600.
Cole, SP. et al. (1984). Mol. Cell Biol. 62: 109-120.
Cote, RJ. et al. (1983). Proc. Natl. Acad. Sci. USA 80: 2026-2030.
Culver, K. (1996). Gene Therapy : A Primer for Physicians. Second Edition. (Mary Ann Liebert).
Folkman, J. and Haudenschild, C. (1980). Nature (Lond.) 288: 551-556.
Friedman, T. (1991). In Therapy for Genetic Diseases. (T Friedman (Ed) Oxford University, Press. pp 105-121.
Gamble, JR. et al. (1993). J. Cell Biol. 121: 931-943.
Gamble, JR. et al. (1999). Endothelium 7: 23-34.
Gecz, J. et al. (1997). Genomics 44: 201-213.
Goldman, CK. et al. (1997). Nature Biotechnology 15: 462-466.
Green, LL. et al. (1994). Nature Genet. 7: 13-21.
Grishok, A. et al. (2001). Science 287: 2494-2497.
Haseloff, J. and Gerlach, WL. (1988). Nature 334: 585-591.
Heller, RA. et al. (1997). Proc. Natl. Acad. Sci. USA 94: 2150-2155.
Huse, WD. et al. (1989). Science 246: 1275-1281.
Kohler, G. and Milstein, C. (1975). Nature 256: 495-497.
Kozbor, D. et al. (1985). J. Immunol. Methods 81:31-42.
Lonberg, N. et al. (1994). Nature 368: 856-859.
Meyer, GT. et al. (1997). The Anatomical Record 249: 327-340.
Orlandi, R. et al. (1989). Proc. Natal. Acad. Sci. USA 86: 3833-3837.
Rickert, RC. et al. (1997). Nucleic Acids Res. 25: 1317-1318.
Sambrook, J. et al. (1989). Molecular cloning: a laboratory manual. Second Edition. (Cold Spring Harbour Laboratory Press, New York).
Scharf, D. et al. (1994). Results Probl. Cell Differ. 20: 125-162.
Schena, M. et al. (1996). Proc. Natl. Acad. Sci. USA 93 : 10614-10619.
Schwenk, F. et al. (1995). Nucleic Acids Res. 23: 5080-5081.
Sharp, PA. and Zamore, PD. (2000). Science 287: 2431-2432.
Taylor, LD. et al. (1994). Int. Immunol. 6: 579-591.
Winter, G. et al. (1991). Nature 349: 293-299.

### Preferred Embodiments of the invention include

(A) A method for the identification of a nucleic acid molecule differentially expressed in an in vitro model of a biological system, comprising the steps of:
   (1) harvesting cells from the model system at predetermined time points;
   (2) obtaining total RNA from the cells harvested at each time point;
   (3) preparing cDNA from the total RNA from each time point to provide a plurality of pools of cDNA;
   (4) performing a suppression subtractive hybridization (SSH) on the cDNA pools from each time point sequentially so as to progressively amplify cDNAs derived from nucleic acid molecules differentially expressed from one time period to the next.

The model system may be an in vitro model for angiogenesis.

A nucleic acid molecule differentially expressed during angiogenesis when identified by the method described above.

The nucleic acid molecule may be selected from the group consisting of those laid out in Tables 1 and 2.
(B) A method for the identification of a nucleic acid molecule up-regulated in an in vitro model of a biological system, comprising the steps of:
   (1) harvesting cells from the model system at predetermined time points;
   (2) obtaining total RNA from the cells harvested at each time point;
   (3) preparing cDNA from the total RNA from each time point to provide a plurality of pools of cDNA;
   (4) performing a suppression subtractive hybridization (SSH) on the cDNA pools from each time point sequentially so as to progressively amplify cDNAs derived from nucleic acid molecules differentially expressed from one time period to the next.
   (5) cloning the amplified cDNAs;
   (6) locating DNA from each clone on a microarray;
   (7) generating antisense RNA by reverse transcription of total RNA from cells harvested from the in vitro model at said predetermined time intervals and labelling the antisense RNA; and
   (8) probing the microarray with labelled antisense RNA from 0 hours and each of the other time points separately to identify clones containing cDNA derived from nucleic acid molecules which are up-regulated at said time points in the in vitro model.

   The in vitro model may be an in vitro model for angiogenesis.
   A nucleic acid molecule when identified by the method described above.
   The nucleic acid molecule may be selected from the group consisting of those set forth in Tables 1 and 2.
(C) A polypeptide encoded by a nucleic acid molecule as defined above.
(D) An isolated nucleic acid molecule comprising the sequence set forth in one of SEQ ID Numbers: 1 to 44.

An isolated nucleic acid molecule comprising the sequence set forth in one of SEQ ID Numbers: 1 to 44 or as laid out in Tables 1 and 2, or a fragment thereof, and which encodes a polypeptide that plays a role in an angiogenic process.

An isolated nucleic acid molecule that is at least 70% identical to a nucleic acid molecule comprising the sequence set forth in one of SEQ ID Numbers: 1 to 44 or as laid out in Tables 1 and 2, and which encodes a polypeptide that plays a role in an angiogenic process.

The isolated nucleic acid molecule may be at least 85% identical or at least 95% identical.

An isolated nucleic acid molecule that encodes a polypeptide that plays a role in an angiogenic process, and which hybridizes under stringent conditions with a nucleic acid molecule comprising the nucleotide sequence set forth in one of SEQ ID Numbers: 1 to 44 or as laid out in Tables 1 and 2.

An isolated nucleic acid molecule as defined above which encodes a polypeptide that plays a role in diseases associated with angiogenesis including but not restricted to cancer, rheumatoid arthritis, diabetic retinopathy, psoriasis, cardiovascular diseases such as atherosclerosis, ischaemic limb disease and coronary artery disease.

An isolated nucleic acid molecule consisting of any one of the nucleotide sequences set forth in SEQ ID Numbers: 1 to 44.
(E) Use of a nucleic acid molecule selected from the group consisting of DNA molecules having the sequence set forth in SEQ ID Numbers: 1 to 15, 17 to 37, and 39 to 44 to identify and/or obtain full-length human genes involved in an angiogenic process.

The use as defined above wherein additional sequence is obtained using hybridization with one or more of said nucleic acid molecules, inverse PCR, restriction site PCR, PCR walking techniques or RACE.
(F) A gene when identified by the use of a nucleic acid molecule selected from any one of SEQ ID Numbers: 1 to 15, 17 to 37, and 39 to 44.
(G) An isolated polypeptide comprising the sequence set forth in one of SEQ ID Numbers: 51 to 58.

An isolated polypeptide comprising the sequence set forth in one of SEQ ID Numbers: 51 to 58 or as laid out in Tables 1 and 2, or a fragment thereof, that plays a role in an angiogenic process.

An isolated polypeptide that plays a role in an angiogenic process, and having at least 70% identity with the amino acid sequence set forth in SEQ ID Numbers: 51 to 58 or a gene as laid out in Tables 1 and 2. The isolated polypeptide may have at least 85% sequence identity or at least 95% sequence identity.

An isolated polypeptide as defined above that plays a role in diseases associated with an angiogenic process including but not restricted to cancer, rheumatoid arthritis, diabetic retinopathy, psoriasis, cardiovascular diseases such as atherosclerosis, ischaemic limb disease and coronary artery disease.

An isolated polypeptide consisting of any one of the amino acid sequences set forth in SEQ ID Numbers: 51 to 58.
(H) An expression vector comprising a nucleic acid molecule as defined above.
(I) A cell comprising the expression vector defined above. The cell may be an eukaryotic cell.
(J) A method of preparing a polypeptide comprising the steps of:
   (1) culturing cells as defined above under conditions effective for polypeptide production; and
   (2) harvesting the polypeptide.

A polypeptide prepared by the method described above.

A method of modulating angiogenesis comprising modulating the expression or activity of a polypeptide in a cell, wherein the polypeptide is encoded by a nucleic acid molecule as defined above.

The method described above wherein the nucleic acid molecule is selected from the group consisting of SEQ ID Numbers: 1 to 44.

The method defined above wherein the polypeptide is that as described above or an active fragment thereof. The polypeptide may comprise an amino acid sequence selected from the group consisting of SEQ ID Numbers: 51 to 58.

The method defined above wherein the expression or activity of the polypeptide is modulated by introducing into the cell an antagonist or agonist of a nucleic acid molecule or a polypeptide as defined above.

The method defined above wherein the expression or activity of the polypeptide is modulated by introducing into the cell an antisense to the nucleic acid molecule.

The method defined above wherein the expression or activity of the polypeptide is modulated by introducing into the cell a nucleic acid molecule which is the complement of at least a portion of a nucleic acid molecule as defined above and is capable of modulating expression or levels of the nucleic acid molecule. The nucleic acid molecule may be an RNA molecule that hybridizes with the mRNA encoded by the nucleic acid molecule. Alternatively the nucleic acid molecule may be a short interfering oligonucleotide that hybridizes with the mRNA encoded by the nucleic acid molecule. Alternatively, the nucleic acid molecule is a catalytic nucleic acid molecule that is targeted to the nucleic acid molecule. The catalytic nucleic acid molecule described above may be a DNAzyme or ribozyme.

The polypeptide expression or activity may be modulated by an antibody capable of binding the polypeptide. The antibody may be a fully human antibody and may be selected from the group consisting of a monoclonal antibody, a humanised antibody, a chimaeric antibody or an antibody fragment including a Fab fragment, (Fab')₂ fragment, Fv fragment, single chain antibodies and single domain antibodies.

The polypeptide expression or activity may be modulated by introducing into the cell a nucleic acid molecule as defined above, or an active fragment or variant thereof. The nucleic acid molecule may be introduced by way of an expression vector as defined above.

The polypeptide expression or activity may be modulated by introducing into the cell a polypeptide as defined above.

The angiogenes may be inappropriately arrested or decreased.
(K) A method for the treatment of an angiogenesis-related disorder, comprising modulating the expression or activity of a polypeptide encoded by a nucleic acid molecule as defined above.

The nucleic acid molecule may be selected from the group consisting of SEQ ID Numbers: 1 to 44.

The polypeptide may be as defined above or an active fragment thereof.

The polypeptide may comprise an amino acid sequence selected from the group consisting of SEQ ID Numbers: 51 to 58.

The expression or activity of the polypeptide may be modulated by introducing into the cell an antagonist or agonist of a nucleic acid molecule as defined above or an antagonist or agonist of a polypeptide as defined above.

The expression or activity of the polypeptide may be modulated by introducing into the cell an antisense to a nucleic acid molecule as defined above.

The expression or activity of the polypeptide may be modulated by introducing into the cell a nucleic acid molecule which is the complement of at least a portion of a nucleic acid molecule as defined above and is capable of modulating expression or levels of the nucleic acid molecule. The nucleic acid molecule may be an RNA molecule that hybridizes with the mRNA encoded by a nucleic acid molecule as defined above. Alternatively the nucleic acid molecule is a short interfering oligonucleotide that hybridizes with the mRNA encoded by a nucleic acid molecule as defined above. Alternatively the nucleic acid molecule is a catalytic nucleic acid molecule that is targeted to a nucleic acid molecule as defined above.

The catalytic nucleic acid molecule may be a DNAzyme or ribozyme.

The polypeptide expression or activity may be modulated by an antibody capable of binding the polypeptide. The antibody may be a full human antibody and may be selected from the group consisting of a monoclonal antibody, a humanised antibody, a chimaeric antibody or an antibody fragment including a Fab fragment, (Fab')₂ fragment, Fv fragment, single chain antibodies and single domain antibodies.

The polypeptide expression or activity may be modulated by introducing into the cell a nucleic acid molecule as defined above or an active fragment or variant thereof.

The nucleic acid molecule may be introduced by way of an expression vector as defined above.

The polypeptide expression or activity may be modulated by introducing into the cell a polypeptide as defined above.

The angiogenesis-related disorder may involve uncontrolled or enhanced angiogenesisis, or may be a disorder in which a decreased vasculature is of benefit. The disorder may be selected from the group consisting of cancer, rheumatoid arthritis, diabetic retinopathy, psoriasis and cardiovascular diseases such as atherosclerosis.

The angiogenesis-related disorder may involve inappropriately arrested or decreased angiogenesisis, or be a disorder in which an expanding vasculature is of benefit. The disorder may be selected from the group consisting of ischemic limb disease or coronary artery disease.
(L) Use of a modulator of expression or activity of a polypeptide encoded by a nucleic acid molecule as defined above in the manufacture of a medicament for the treatment of an angiogenesis-related disorder.

The nucleic acid sequence may be selected from the group consisting of SEQ ID Numbers: 1 to 44.

The polypeptide may be as defined above or an active fragment thereof.

The polypeptide may comprise an amino acid sequence selected from the group consisting of SEQ ID Numbers: 51 to 58.

The use defined above wherein the expression or activity of the polypeptide is modulated by introducing into the cell an antagonist or agonist of a nucleic acid molecule as defined above or an antagonist or agonist of a polypeptide as defined above.

The expression or activity of the polypeptide may be modulated by introducing into the cell an antisense to a nucleic acid molecule as defined above.

The use defined above wherein the expression or activity of the polypeptide is modulated by introducing into the cell a nucleic acid molecule which is the complement of at least a portion of a nucleic acid molecule as defined above and is capable of modulating expression or levels of the nucleic acid molecule. The nucleic acid molecule may be an RNA molecule that hybridizes with the mRNA encoded by the nucleic acid molecule. Alternatively the nucleic acid molecule is a short interfering oligonucleotide that hybrizes with the mRNA encoded by the nucleic acid molecule. Alternatively the nucleic acid molecule is a catalytic nucleic acid molecule that is targeted to the nucleic acid molecule.

The catalytic nucleic acid molecule may be a DNAzyme or ribozyme.

The use defined above wherein the polypeptide expression or activity is modulated by an antibody capable of binding the polypeptide. The antibody may be a full human antibody and may be selected from the group consisting of a monoclonal antibody, a humanised antibody, a chimaeric antibody or an antibody fragment including a Fab fragment, (Fab')₂ fragment, Fv fragment, single chain antibodies and single domain antibodies.

The use defined above wherein the polypeptide expression or activity is modulated by introducing into the cell a nucleic acid molecule as defined above, or an active fragment or variant thereof. The nucleic acid molecule may be introduced by way of an expression vector as defined above.

The use as defined above wherein the polypeptide expression or activity is modulated by introducing into the cell a polypeptide as defined above.

The angiogenesis-related disorder may involve uncontrolled or enhanced angiogenesis, or be a disorder in which a decreased vasculature is of benefit. The disorder may be selected from the group consisting of cancer, rheumatoid arthritis, diabetic retinopathy, psoriasis and cardiovascular diseases such as atherosclerosis.

The use as defined above wherein the angiogenesis-related disorder involves inappropriately arrested or decreased angiogenesis, or is a disorder in which an expanding vasculature is of benefit. The disorder may be selected from the group consisting of ischaemic limb disease or coronary artery disease.
(M) The use of a nucleic acid molecule as defined above for the screening of candidate pharmaceutical compounds useful in the treatment of angiogenesis-related disorders.

A compound useful in the treatment of angiogenesis-related disorders when identified by the use of a nucleic acid molecule as defined above.

The use of a polypeptide as defined above for the screening of candidate pharmaceutical compounds useful in the treatment of angiogenesis-related disorders.

A compound useful in the treatment of angiogenesis-related disorders when identified by the use of a polypeptide as defined above.

The use of a cell as defined above for the screening of candidate pharmaceutical compounds useful in the treatment of angiogenesis-related disorders.

A compound useful in the treatment of angiogenesis-related disorders when identified by the use of a cell as defined above.
(N) A method of screening for a candidate pharmaceutical compound useful in the treatment of angiogenesis-related disorders comprising the steps of:
   (1) providing a polypeptide as defined above;
   (2) adding a candidate pharmaceutical compound to said polypeptide; and
   (3) determining the binding of said candidate compound to said polypeptide;
   wherein a compound that binds to the polypeptide is a candidate pharmaceutical compound.

A method of screening for candidate pharmaceutical compound useful in the treatment of angiogenesis-related disorders comprising the steps of:
(1) providing a cell, as defined above;
(2) adding a candidate pharmaceutical compound to said cell; and
(3) determining the effect of said candidate pharmaceutical compound on the functional properties of said cell;
wherein a compound that alters the functional properties of said cell is a candidate pharmaceutical compound.

A method of screening for a candidate pharmaceutical compound useful in the treatment of angiogenesis-related disorders comprising the steps of:
(1) providing a cell, as defined above;
(2) adding a candidate pharmaceutical compound to said cell; and
(3) determining the effect of said candidate pharmaceutical compound on the expression of the nucleic acid molecule that is part of the expression vector in said cell;
wherein a compound that alters the expression of the nucleic acid molecule that is part of the expression vector in said cell is a candidate pharmaceutical compound.

A method of screening for a candidate pharmaceutical compound useful in the treatment of angiogenesis-related disorders comprising the steps of:
(1) providing a cell, as defined above
(2) adding a candidate pharmaceutical compound to said cell; and
(3) determining the effect of said candidate pharmaceutical compound on the expression or activity of the polypeptide encoded by the nucleic acid molecule that is part of the expression vector in said cell;
wherein a compound that alters the expression or activity of polypeptide encoded by the nucleic acid molecule that is part of the expression vector in said cell is a candidate pharmaceutical compound.

A compound when identified by the screening methods.
(O) A pharmaceutical composition comprising a compound as defined in M or N and a pharmaceutically acceptable carrier.
(P) An antibody which is immunologically reactive with an isolated polypeptide as defined above.

The antibody may be a fully human antibody and may be selected from the group consisting of a monoclonal antibody, a humanised antibody, a chimaeric antibody or an antibody fragment including a Fab fragment, (Fab')₂ fragment, Fv fragment, single chain antibodies and single domain antibodies.
(Q) A short interfering oligonucleotide targeted to the mRNA encoded by a nucleic acid molecule as defined above.
(R) A catalytic nucleic acid molecule targeted to a nucleic acid molecule as defined above.

The catalytic nucleic acid molecule may be a DNAzyme or a ribozyme.
(S) Use of a nucleic acid molecule as defined above in the diagnosis or prognosis of an angiogenesis-related disorder.

Use of a polypeptide as defined above in the diagnosis or prognosis of an angiogenesis-related disorder.

Use of an antibody as defined above or an antibody to a polypeptide as defined above in the diagnosis or prognosis of an angiogenesis-related disorder.

A method for the diagnosis or prognosis of an angiogenesis-related disorder comprising the steps of:
(1) establishing a profile for normal expression and/or activity of a nucleic acid molecule as defined above in unaffected subjects;
(2) measuring the level of expression and/or activity of said nucleic acid molecule in a person suspected of abnormal expression and/or activity of the gene; and
(3) comparing the measured level of expression and/or activity of said nucleic acid molecule with the profile for normal expression and/or activity;
wherein an altered level of expression and/or activity of said nucleic acid molecule in said subject is an indication of an angiogenesis-related disorder, or a predisposition thereto.

Reverse transcriptase PCR may be employed to measure levels of expression.

A hybridization assay using a probe derived from the gene, or a fragment thereof, may be employed to measure levels of expression.

A method for the diagnosis or prognosis of an angiogenesis-related comprising the steps of:
(1) obtaining DNA from a subject corresponding to a nucleic acid molecule as defined above; and
(2) comparing the DNA from said subject to the DNA of the corresponding wild-type nucleic acid molecule;
wherein altered DNA properties in said subject is an indication of an angiogenesis-related disorder, or a predisposition thereto.

The DNA of the nucleic acid molecule may be sequenced and the sequences compared.

The DNA of the nucleic acid molecule may be subjected to SSCP analysis.

A method for the diagnosis or prognosis of an angiogenesis-related disorder comprising the steps of:
(1) establishing a physical property of a wild-type polypeptide as defined above;
(2) obtaining the polypeptide from a person suspected of an abnormality of that polypeptide; and;
(3) measuring the property for the polypeptide expressed by said person and comparing it to the established property for the wild-type polypeptide;
wherein altered polypeptide properties in said subject is an indication of an angiogenesis-related disorder, or a predisposition thereto.

The property may be the electrophoretic mobility or the proteolytic cleavage pattern.
(T) A genetically modified non-human animal comprising a isolated a nucleic acid molecule as defined above.

A genetically modified non-human animal comprising a disruption of the nucleic acid molecule.

The animal may be selected from the group consisting of rats, mice, hamsters, guinea pigs, rabbits, dogs, cats, goats, sheep, pigs and non-human primates such as monkeys and chimpanzees.

The animal is preferably a mouse.

Use of a genetically modified non-human animal as defined above in screening for candidate pharmaceutical compounds useful for the treatment of angiogenesis-related disorders.
(U) The use of any one of the screening methods defined above wherein the angiogenesis-related disorder involves uncontrolled or enhanced angiogeneis, or is a disorder in which a decreased vasculature is of benefit.

The disorder may be selected from the group consisting of cancer, rheumatoid arthritis, diabetic retinopathy, psoriasis and cardiovascular diseases such as atherosclerosis.

The angiogenesis-related disorder may involve inappropriately arrested or decreased angiogenesis, or be a disorder in which an expanding vasculature is of benefit. The disorder may be selected from the group consisting of ischaemic limb disease or coronary artery disease.

## Claims

1. A modulator of expression of a polypeptide encoded by a nucleic acid molecule identified herein as SEQ ID NO: 59 (BNO802) wherein the modulator is an antisense nucleic acid molecule, for use in the treatment of an angiogenesis-related desorder.

2. A modulator of expression of a polypeptide encoded by a nucleic acid molecule identified herein as SEQ ID NO: 59 (BNO802) wherein the modulator is a nucleic acid which is a complement of at least a portion of said the nucleic acid molecule and is capable of modulating expression or levels of the nucleic acid molecule, for use in the treatment of an angiogenesis-related desorder.

3. The modulator of claim 2, wherein the nucleic acid is an RNA molecule that hybridizes with the mRNA encoded by said nucleic acid molecule.

4. The modulator of claim 2, wherein the nucleic acid is a short interfering oligonucleotide that hybridizes with the mRNA encoded by said nucleic acid molecule.

5. A modulator of expression of a polypeptide encoded by a nucleic acid molecule identified herein as SEQ ID NO: 59 (BNO802) wherein the modulator is an antibody capable of binding the polypeptide, for use in the treatment of an angiogenesis-related desorder.

6. The modulator of claim 5, wherein the antibody is a full human antibody.

7. The modulator of claim 5, wherein the antibody is selected from the group consisting of a monoclonal antibody, a humanised antibody, a chimaeric antibody or an antibody fragment including a Fab fragment, (Fab')₂ fragment, Fv fragment, single chain antibodies and single domain antibodies.

8. Use of a nucleic acid molecule identified herein as SEQ ID NO: 59 (BNO802) or a fragment thereof which encodes a polypeptide that plays a role in angiogenesis process, for the screening of candidate pharmaceutical compounds useful in the treatment of an angiogenesis-related disorder.

9. Use of a polypeptide identified herein as SEQ ID NO: 59 (BNO802), or a fragment thereof that plays a role in an angiogenesis process, for the screening of candidate pharmaceutical compounds useful in the treatment of an angiogenesis-related disorder.

10. Use of cell comprising an expression vector comprising a nucleic acid molecule identified herein as SEQ ID NO: 59 (BNO802), or a fragment thereof which encodes a polypeptide that plays a role in an angiogenesis process, for the screening of candidate pharmaceutical compounds useful in the treatment of an angiogenesis-related disorder.

11. Use according to any one of claims 8-10 wherein the angiogenesis-related disorder involves uncontrolled or enhanced angiogenesis, or is a disorder in which to decreased vasculature is of benefit.

12. Use according to claim 11 wherein the disorder is selected from the group consisting of cancer, rheumatoid arthritis, diabetic retinopathy, psoriasis and cardiovascular diseases such as atherosclerosis.

13. Use according to any one of claims 8-10 wherein the angiogenesis related disorder involves inappropriately arrested or decreased angiogenesis, or is a disorder in which an expanding vasculature is of benefit.

14. Use according to claim 13 wherein the disorder is selected from the group consisting of ischaemic limb diseases or coronary artery disease.

15. A method of screening for a candidate pharmaceutical compound useful in the treatment of an angiogenesis-related disorder, comprising the steps of:
(1) providing a polypeptide identified herein as SEQ ID NO: 59 (BNO802), or a fragment thereof that plays a role in an angiogenesis process;
(2) adding a candidate pharmaceutical compound to said polypeptide, and
(3) determining the binding of said candidate compound to said polypeptide;
wherein a compound that binds to the polypeptide is a candidate pharmaceutical compound.

16. A method of screening for a candidate pharmaceutical compound useful in the treatment of an angiogenesis-related disorder, comprising the steps of:
(1) providing a cell comprising an expression vector comprising a nucleic acid molecule identified herein as SEQ ID NO: 59 (BNO802) or a fragment thereof which encodes a polypeptide that plays a role in angiogenesis process;
(2) adding a candidate pharmaceutical compound to said cell; and
(3) determining the effect of said candidate pharmaceutical compound on the functional properties of said cell;
wherein a compound that modulates angiogenesis in a system comprising said cell is a candidate pharmaceutical compound.

17. A method of screening for a candidate pharmaceutical compound useful in the treatment of an angiogenesis-related disorder, comprising the steps of:
(1) providing a cell comprising an expression vector comprising a nucleic acid molecule identified herein as SEQ ID NO: 59 (BNO802), or a fragment thereof which encodes a polypeptide that plays a role in angiogenesis process;
(2) adding a candidate pharmaceutical compound to said cell; and
(3) determining the effect of said candidate pharmaceutical compound on the expression of the nucleic acid molecule that is part of the expression vector in said cell;
wherein a compound that alters the expression of the nucleic acid molecule that is part of the expression vector in said cell is a candidate pharmaceutical compound.

18. A method of screening for a candidate pharmaceutical compound useful in the treatment of an angiogenesis-related disorder, comprising the steps of:
(1) providing a cell comprising an expression vector comprising a nucleic acid molecule identified herein as SEQ ID NO: 59 (BNO802), or a fragment thereof which encodes a polypeptide that plays a role in angiogenesis process;
(2) adding a candidate pharmaceutical compound to said cell; and
(3) determining the effect of said candidate pharmaceutical compound on the expression or activity of the polypeptide encoded by the nucleic acid molecule that is part of the expression vector in said cell;
wherein a compound that alters the expression or activity of the polypeptide encoded by the nucleic acid molecule that is part of the expression vector in said cell is a candidate pharmaceutical compound.

19. A method according to any one of claims 8-11, wherein the angiogenesis-related disorder involves uncontrolled or enhanced angiogenesis, or is a disorder in which a decreased vasculature is of benefit.

20. A method according to any one of claims 15-18, wherein the disorder is selected from the group consisting of cancer, rheumatoid arthritis, diabetic retinopathy, psoriasis and cardiovascular diseases such as atherolsclerosis.

21. A method according to any one of claims 15-18, wherein the angiogenesis-related disorder involves inappropriately arrested or decreased angiogenesis, or is a disorder in which an expanding vasculature is of benefit.

22. A method according to any one of claims 15-18, wherein the disorder is selected form the group consisting of ischaemic limb disease or coronary artery disease.

## Patentansprüche

1. Expressionsmodulator eines Polypeptids, das durch ein hierin als SEQ ID Nr.: 59 (BNO802) **gekennzeichnetes** Nucleinsäuremolekül codiert wird, wobei der Modulator ein Antisense-Nucleinsäuremolekül ist, zur Verwendung bei der Behandlung einer mit Angiogenese verbundenen Erkrankung.

2. Expressionsmodulator eines Polypeptids, das durch ein hierin als SEQ ID Nr.: 59 (BNO802) **gekennzeichnetes** Nucleinsäuremolekül codiert wird, wobei der Modulator ein Nucleinsäuremolekül ist, das ein Komplement zumindest eines Teils des Nucleinsäuremoleküls ist und die Expression oder Konzentrationen des Nucleinsäuremoleküls modulieren kann, zur Verwendung bei der Behandlung einer mit Angiogenese verbundenen Erkrankung.

3. Modulator nach Anspruch 2, wobei die Nucleinsäure ein RNA-Molekül ist, das mit der durch das Nucleinsäuremolekül codierten mRNA hybridisiert.

4. Modulator nach Anspruch 2, wobei die Nucleinsäure ein kurzes interferierendes Oligonucleotid ist, das mit der durch das Nucleinsäuremolekül codierten mRNA hybridisiert.

5. Expressionsmodulator eines Polypeptids, das durch ein hierin als SEQ ID Nr.: 59 (BNO802) **gekennzeichnetes** Nucleinsäuremolekül codiert wird, wobei der Modulator ein Antikörper ist, der das Polypeptid binden kann, zur Verwendung bei der Behandlung einer mit Angiogenese verbundenen Erkrankung.

6. Modulator nach Anspruch 5, wobei der Antikörper ein voll-humaner Antikörper ist.

7. Modulator nach Anspruch 5, wobei der Antikörper aus der Gruppe ausgewählt ist, die aus einem monoklonalen Antikörper, einem humanisierten Antikörper, einem chimären Antikörper oder einem Antikörperfragment besteht, wozu ein Fab-Fragment, (Fab')₂-Fragment, Fv-Fragment, Einzelkettenantikörper und Einzeldomänenantikörper gehören.

8. Verwendung eines hierin als SEQ ID Nr.: 59 (BNO802) **gekennzeichneten** Nucleinsäuremoleküls oder eines Fragments davon, das für ein Polypeptid codiert, das eine Rolle in einem Angiogeneseprozess spielt, zum Durchmustern von pharmazeutischen Kandidatenverbindungen, die bei der Behandlung einer mit Angiogenese verbundenen Erkrankung verwendbar sind.

9. Verwendung eines hierin als SEQ ID Nr.: 59 (BNO802) **gekennzeichneten** Polypeptids oder eines Fragments davon, das eine Rolle in einem Angiogeneseprozess spielt, zum Durchmustern von pharmazeutischen Kandidatenverbindungen, die bei der Behandlung einer mit Angiogenese verbundenen Erkrankung verwendbar sind.

10. Verwendung einer Zelle, die einen Expressionsvektor mit einem hierin als SEQ ID Nr.: 59 (BNO802) **gekennzeichneten** Nucleinsäuremolekül oder einem Fragment davon aufweist, das für ein Polypeptid codiert, das eine Rolle in einem Angiogeneseprozess spielt, zum Durchmustern von pharmazeutischen Kandidatenverbindungen, die bei der Behandlung einer mit Angiogenese verbundenen Erkrankung verwendbar sind.

11. Verwendung nach einem der Ansprüche 8-10, wobei die mit Angiogenese verbundene Erkrankung mit unkontrollierter oder verstärkter Angiogenese verbunden ist oder eine Erkrankung ist, bei der verkleinerte Gefäße von Vorteil sind.

12. Verwendung nach Anspruch 11, wobei die Erkrankung aus der Gruppe ausgewählt ist, die aus Krebs, Rheumatoidarthritis, diabetischer Retinopathie, Psoriasis und Herz-Kreislauf-Erkrankungen, wie z.B. Atherosklerose, besteht.

13. Verwendung nach einem der Ansprüche 8-10, wobei die mit Angiogenese verbundene Erkrankung mit unzweckmäßig zum Stillstand gebrachter oder verminderter Angiogenese verbunden ist oder eine Erkrankung ist, bei der sich erweiternde Gefäße von Vorteil sind.

14. Verwendung nach Anspruch 13, wobei die Erkrankung aus der Gruppe ausgewählt ist, die aus ischämischen Gliedmaßenerkrankungen oder Koronararterienerkrankung besteht.

15. Durchmusterungsverfahren für eine pharmazeutische Kandidatenverbindung, die bei der Behandlung einer mit Angiogenese verbundenen Erkrankung verwendbar ist, wobei das Verfahren die folgenden Schritte aufweist:
(1) Bereitstellen eines hierin als SEQ ID Nr.: 59 (BNO802) **gekennzeichneten** Polypeptids oder eines Fragments davon, das eine Rolle in einem Angiogeneseprozess spielt;
(2) Zugabe einer pharmazeutischen Kandidatenverbindung zu dem Polypeptid; und
(3) Ermitteln der Bindung der Kandidatenverbindung an das Polypeptid; wobei eine Verbindung, die an das Polypeptid bindet, eine pharmazeutische Kandidatenverbindung ist.

16. Durchmusterungsverfahren für eine pharmazeutische Kandidatenverbindung, die bei der Behandlung einer mit Angiogenese verbundenen Erkrankung verwendbar ist, wobei das Verfahren die folgenden Schritte aufweist:
(1) Bereitstellen einer Zelle, die einen Expressionsvektor mit einem hierin als SEQ ID Nr.: 59 (BNO802) **gekennzeichneten** Nucleinsäuremolekül oder einem Fragment davon aufweist, das für ein Polypeptid codiert, das eine Rolle im Angiogeneseprozess spielt;
(2) Zugabe einer pharmazeutischen Kandidatenverbindung zu der Zelle; und
(3) Ermitteln der Wirkung der pharmazeutischen Kandidatenverbindung auf die funktionellen Eigenschaften der Zelle;
wobei eine Verbindung, die Angiogenese in einem die Zelle aufweisenden System moduliert, eine pharmazeutische Kandidatenverbindung ist.

17. Durchmusterungsverfahren für eine pharmazeutische Kandidatenverbindung, die bei der Behandlung einer mit Angiogenese verbundenen Erkrankung verwendbar ist, wobei das Verfahren die folgenden Schritte aufweist:
(1) Bereitstellen einer Zelle, die einen Expressionsvektor mit einem hierin als SEQ ID Nr.: 59 (BNO802) **gekennzeichneten** Nucleinsäuremolekül oder einem Fragment davon aufweist, das für ein Polypeptid codiert, das eine Rolle im Angiogeneseprozess spielt;
(2) Zugabe einer pharmazeutischen Kandidatenverbindung zu der Zelle; und
(3) Ermitteln der Wirkung der pharmazeutischen Kandidatenverbindung auf die Expression des Nucleinsäuremoleküls, das Teil des Expressionsvektors in der Zelle ist;
wobei eine Verbindung, welche die Expression des Nucleinsäuremoleküls verändert, das Teil des Expressionsvektors in der Zelle ist, eine pharmazeutische Kandidatenverbindung ist.

18. Durchmusterungsverfahren für eine pharmazeutische Kandidatenverbindung, die bei der Behandlung einer mit Angiogenese verbundenen Erkrankung verwendbar ist, wobei das Verfahren die folgenden Schritte aufweist:
(1) Bereitstellen einer Zelle, die einen Expressionsvektor mit einem hierin als SEQ ID Nr.: 59 (BNO802) **gekennzeichneten** Nucleinsäuremolekül oder einem Fragment davon aufweist, das für ein Polypeptid codiert, das eine Rolle im Angiogeneseprozess spielt;
(2) Zugabe einer pharmazeutischen Kandidatenverbindung zu der Zelle; und
(3) Ermitteln der Wirkung der pharmazeutischen Kandidatenverbindung auf die Expression oder Aktivität des Polypeptids, das durch das Nucleinsäuremolekül codiert wird, das Teil des Expressionsvektors in der Zelle ist;
wobei eine Verbindung, welche die Expression oder Aktivität des Polypeptids verändert, das durch das Nucleinsäuremolekül codiert wird, das Teil des Expressionsvektors in der Zelle ist, eine pharmazeutische Kandidatenverbindung ist.

19. Verfahren nach einem der Ansprüche 8-11, wobei die mit Angiogenese verbundene Erkrankung mit unkontrollierter oder verstärkter Angiogenese verbunden ist oder eine Erkrankung ist, bei der verkleinerte Gefäße von Vorteil sind.

20. Verfahren nach einem der Ansprüche 15-18, wobei die Erkrankung aus der Gruppe ausgewählt ist, die aus Krebs, Rheumatoidarthritis, diabetischer Retinopathie, Psoriasis und Herz-Kreislauf-Erkrankungen, wie z. B. Atherosklerose, besteht.

21. Verfahren nach einem der Ansprüche 15-18, wobei die mit Angiogenese verbundene Erkrankung mit unzweckmäßig zum Stillstand gebrachter oder verminderter Angiogenese verbunden ist oder eine Erkrankung ist, bei der sich erweiternde Gefäße von Vorteil sind.

22. Verfahren nach einem der Ansprüche 15-18, wobei die Erkrankung aus der Gruppe ausgewählt ist, die aus ischämischer Gliedmaßenerkrankung oder Koronararterienerkrankung besteht.

## Revendications

1. Modulateur d'expression d'un polypeptide codé par une molécule d'acide nucléique identifiée ici comme la SEQ ID NO:59 (BNO802), où le modulateur est une molécule d'acide nucléique anti-sens, pour une utilisation dans le traitement d'un trouble lié à l' angiogenèse.

2. Modulateur d'expression d'un polypeptide codé par une molécule d'acide nucléique identifiée ici comme la SEQ ID NO:59 (BNO802), où le modulateur est un acide nucléique qui est un complément d'au moins une portion de ladite molécule d'acide nucléique et il est capable de moduler l'expression ou des niveaux de la molécule d'acide nucléique, pour une utilisation dans le traitement d'un trouble lié à l'angiogenèse.

3. Modulateur selon la revendication 2, dans lequel l'acide nucléique est une molécule d'ARN qui s'hybride avec l'ARNm codé par ladite molécule d'acide nucléique.

4. Modulateur selon la revendication 2, dans lequel l'acide nucléique est un oligonucléotide à interférence courte qui s'hybride avec l'ARNm codé par ladite molécule d'acide nucléique.

5. Modulateur d'expression d'un polypeptide codé par une molécule d'acide nucléique identifiée ici comme la SEQ ID NO:59 (BNO802), où le modulateur est un anticorps capable de se lier au polypeptide, pour une utilisation dans le traitement d'un trouble lié à l'angiogenèse.

6. Modulateur selon la revendication 5, dans lequel l'anticorps est un anticorps total humain.

7. Modulateur selon la revendication 5, dans lequel l'anticorps est choisi dans le groupe constitué d'un anticorps monoclonal, d'un anticorps humanisé, d'un anticorps chimérique ou d'un fragment d'anticorps incluant un fragment Fab, un fragment (Fab')₂, un fragment Fv, d'anticorps à une seule chaîne et d'anticorps à un seul domaine.

8. Utilisation d'une molécule d'acide nucléique identifiée ici comme la SEQ ID NO:59 (BNO802), ou d'un fragment de celle-ci qui code pour un polypeptide qui joue un rôle dans un processus d'angiogenèse, pour le criblage de composés pharmaceutiques candidats utiles dans le traitement d'un trouble lié à l'angiogenèse.

9. Utilisation d'un polypeptide identifié ici comme la SEQ ID NO:59 (BNO802), ou d'un fragment de celui-ci qui joue un rôle dans un processus d'angiogenèse, pour le criblage de composés pharmaceutiques candidats utiles dans le traitement d'un trouble lié à l'angiogenèse.

10. Utilisation d'une cellule comprenant un vecteur d'expression comprenant une molécule d'acide nucléique identifiée ici comme la SEQ ID NO:59 (BNO802), ou un fragment de celle-ci qui code pour un polypeptide qui joue un rôle dans un processus d'angiogenèse, pour le criblage de composés pharmaceutiques candidats utiles dans le traitement d'un trouble lié à l'angiogenèse.

11. Utilisation selon l'une quelconque des revendications 8-10, dans laquelle le trouble lié à l'angiogenèse implique une angiogenèse incontrôlée ou accrue ou c'est un trouble dans lequel une diminution des vaisseaux est avantageuse.

12. Utilisation selon la revendication 11, dans laquelle le trouble est choisi dans le groupe constitué d'un cancer, d'une polyarthrite rhumatoïde, d'une rétinopathie diabétique, d'un psoriasis et de maladies cardiovasculaires telles qu'une athérosclérose.

13. Utilisation selon l'une quelconque des revendications 8-10, dans laquelle le trouble lié à l'angiogenèse implique une angiogenèse inopportunément arrêtée ou diminuée ou c'est un trouble dans lequel une expansion des vaisseaux est avantageuse.

14. Utilisation selon la revendication 13, dans laquelle le trouble est choisi dans le groupe constitué de maladies ischémiques des membres ou d'une maladie des artères coronaires.

15. Procédé pour le criblage d'un composé pharmaceutique candidat utile dans le traitement d'un trouble lié à l'angiogenèse, comprenant les étapes:
(1) de fourniture d'un polypeptide identifié ici comme la SEQ ID NO:59 (BNO802), ou d'un fragment de celui-ci qui joue un rôle dans un processus d'angiogenèse;
(2) d'ajout d'un composé pharmaceutique candidat audit polypeptide; et
(3) de détermination de la liaison dudit composé candidat avec ledit polypeptide;
dans lequel un composé qui se lie avec le polypeptide est un composé pharmaceutique candidat.

16. Procédé pour le criblage d'un composé pharmaceutique candidat utile dans le traitement d'un trouble lié à l'angiogenèse, comprenant les étapes:
(1) de fourniture d'une cellule comprenant un vecteur d'expression comprenant une molécule d'acide nucléique identifiée ici comme la SEQ ID NO:59 (BNO802), ou un fragment de celle-ci qui code pour un polypeptide qui joue un rôle dans un processus d'angiogenèse;
(2) d'ajout d'un composé pharmaceutique candidat à ladite cellule; et
(3) de détermination de l'effet dudit composé pharmaceutique candidat sur les propriétés fonctionnelles de ladite cellule;
dans lequel un composé qui module l'angiogenèse dans un système comprenant ladite cellule est un composé pharmaceutique candidat.

17. Procédé pour le criblage d'un composé pharmaceutique candidat utile dans le traitement d'un trouble lié à l'angiogenèse, comprenant les étapes:
(1) de fourniture d'une cellule comprenant un vecteur d'expression comprenant une molécule d'acide nucléique identifiée ici comme la SEQ ID NO:59 (BNO802), ou un fragment de celle-ci qui code pour un polypeptide qui joue un rôle dans un processus d'angiogenèse;
(2) d'ajout d'un composé pharmaceutique candidat à ladite cellule; et
(3) de détermination de l'effet dudit composé pharmaceutique candidat sur l'expression de la molécule d'acide nucléique qui est une partie du vecteur d'expression dans ladite cellule;
dans lequel un composé qui modifie l'expression de la molécule d'acide nucléique qui est une partie du vecteur d'expression dans ladite cellule est un composé pharmaceutique candidat.

18. Procédé pour le criblage d'un composé pharmaceutique candidat utile dans le traitement d'un trouble lié à l'angiogenèse, comprenant les étapes:
(1) de fourniture d'une cellule comprenant un vecteur d'expression comprenant une molécule d'acide nucléique identifiée ici comme la SEQ ID NO:59 (BNO802), ou un fragment de celle-ci qui code pour un polypeptide qui joue un rôle dans un processus d'angiogenèse;
(2) d'ajout d'un composé pharmaceutique candidat à ladite cellule; et
(3) de détermination de l'effet dudit composé pharmaceutique candidat sur l'expression ou l'activité du polypeptide codé par la molécule d'acide nucléique qui est une partie du vecteur d'expression dans ladite cellule;
dans lequel un composé qui modifie l'expression ou l'activité du polypeptide codé par la molécule d'acide nucléique qui est une partie du vecteur d'expression dans ladite cellule est un composé pharmaceutique candidat.

19. Procédé selon l'une quelconque des revendications 8-11, dans lequel le trouble lié à l'angiogenèse implique une angiogenèse incontrôlée ou accrue ou c'est un trouble dans lequel une diminution des vaisseaux est avantageuse.

20. Procédé selon l'une quelconque des revendications 15-18, dans lequel le trouble est choisi dans le groupe constitué d'un cancer, d'une polyarthrite rhumatoïde, d'une rétinopathie diabétique, d'un psoriasis et de maladies cardiovasculaires telles qu'une athérosclérose.

21. Procédé selon l'une quelconque des revendications 15-18, dans lequel le trouble lié à l'angiogenèse implique une angiogenèse inopportunément arrêtée ou diminuée ou c'est un trouble dans lequel une expansion des vaisseaux est avantageuse.

22. Procédé selon l'une quelconque des revendications 15-18, dans lequel le trouble est choisi dans le groupe constitué d'une maladie ischémique des membres ou d'une maladie des artères coronaires.
